(19) Europäisches Patentamt
European Patent Office
Office européen des brevets

(11) EP 3 878 440 A1

(12) EUROPEAN PATENT APPLICATION
published in accordance with Art. 153(4) EPC

(43) Date of publication:
**15.09.2021 Bulletin 2021/37**

(21) Application number: **19813375.3**

(22) Date of filing: **08.11.2019**

(51) Int Cl.:
***A61K 31/01*** *(2006.01)* ***A61K 31/015*** *(2006.01)*
***A61K 31/045*** *(2006.01)* ***A61K 31/05*** *(2006.01)*
***A61K 31/192*** *(2006.01)* ***A61K 31/352*** *(2006.01)*
***A61P 9/00*** *(2006.01)* ***A61P 11/14*** *(2006.01)*
***A61P 13/10*** *(2006.01)* ***A61P 25/02*** *(2006.01)*

(86) International application number:
**PCT/ES2019/070765**

(87) International publication number:
**WO 2020/094908 (14.05.2020 Gazette 2020/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2018 US 201862757660 P**
**06.09.2019 US 201962897235 P**

(71) Applicants:
- **GBS Global Biopharma, Inc.**
**Ottawa, Ontario K2H 8K7 (CA)**
- **University of Seville**
**41004 Seville (ES)**

(72) Inventors:
- **SMALL-HOWARD, Andrea**
**Ottawa, Ontario K2H 8K7 (CA)**
- **MARTÍN BANDERAS, Lucía**
**41004 Sevilla (ES)**
- **EL-HAMMADI, Mazen**
**41004 Sevilla (ES)**
- **FERNÁNDEZ ARÉVALO, Mercedes**
**41004 Sevilla (ES)**

(74) Representative: **Mewburn Ellis LLP**
**Aurora Building**
**Counterslip**
**Bristol BS1 6BX (GB)**

# (54) THERAPEUTIC NANOPARTICLES WHICH ENCAPSULATE TERPENOIDS AND/OR CANNABINOIDS

(57) Provided herein are terpenoid- and cannabinoid-encapsulating PLGA nanoparticles and pharmaceutical compositions comprising the nanoparticles. Further provided are methods of making and using the terpenoid- and cannabinoid-encapsulating PLGA nanoparticles for therapeutic purposes.

EP 3 878 440 A1

Printed by Jouve, 75001 PARIS (FR)

## Description

### CROSS-REFERENCE TO RELATED APPLICATIONS

This application claims the benefit of U.S. Provisional Application No. 62/757,660 filed November 8, 2018, and No. 62/897,235 filed September 6, 2019. The contents of these applications are each incorporated herein by reference.

### BACKGROUND OF THE INVENTION

Channels of the Transient Receptor Superfamily (TRP), such as TRPV1, TRPM8 and TRPA1, are non-selective cation channels that conduct calcium and sodium into a range of cell types in mammals. They are present on sensory neurons, and they were initially identified as having a role in nociception because of their responsiveness at the molecular level to plant secondary metabolites that are nociomimetic (e.g., capsaicin) and to compounds that are otherwise pungent and mimic burning or cooling sensations (e.g., allicin, cinnamaldehyde, menthol).

Because of their role in nociception, TRP channels have been identified as targets for treating pain disorders. Both antagonism and agonism of the TRP channel have been exploited for pain management. For example, TRPV1 antagonists have utility in acute analgesia. For chronic pain management, TRPV1 agonists are typically used. This latter strategy exploits the fact that continued TRPV1 receptor agonism causes desensitization at the cell surface (receptor internalization, degradation and recycling). Prolonged agonism of TRPV1 also leads to calcium and sodium cationic overload of the TRPV1-containing sensory neuron, leading to cell death.

In practice, the use of TRPV1 agonists to effect desensitization involves topical application of high levels of a well-known TRPV1 agonist, capsaicin, repeatedly over time to the affected area. This therapeutic approach has the benefit of efficacy and low cost. However, it also has weaknesses.

First, high affinity and high specificity TRPV1 agonists target only TRPV1-containing nociceptors, leaving other sensory neurons and TRP channels involved in pain untouched. Second, use of high affinity and high specificity TRPV1 agonists such as capsaicin causes high levels of discomfort during initial treatment, in the period prior to desensitization. It is for this reason that post-herpetic pain is currently not addressable using TRPV1-mediated desensitization due to the highly irritant nature of the therapy on sensitive areas such as the gastric mucosa and reproductive tract mucosa. Third, capsaicin-mediated desensitization treatments are limited to topical use; visceral pain, headache and certain musculoskeletal pain disorders are not addressed by this therapy.

There is, therefore, a need for therapeutic TRPV1 ligands, such as TRPV1 agonists, that are lower affinity than capsaicin and which can be administered by mouth for systemic relief. Such lower affinity ligands should cause reduced pain during desensitization, thereby allowing topical treatment of sensitive body areas. There is a need for TRPV1 ligands with broader target specificity, able to target multiple types of TRP-bearing nociceptors, thereby improving the degree of tissue desensitization. Furthermore, there is also a need for pharmaceutical compositions comprising such TRPV1 ligands suitable for systemic administration in addition to topical application and long-term release of such TRPV1 ligands to induce chronic downregulation of TRPV1. Oral administration is highly desirable for pain medications for systemic usage.

Such new oral medications would also be useful for the treatment of various diseases associated with TRPV1 other than pain. While TRP channels were first shown to be involved in pain and nociception, they are now known to have various other physiological roles, suggesting that they can be a target for treatment of other diseases. For example, TRP channels have been identified as a target for treatment of cardiovascular disease; targeted pharmacological inhibition of TRPV1 has been shown to significantly diminish cardiac hypertrophy in a mouse model. See U.S. Pat. No. 9,084,786. Chronic downregulation of TRPV1 levels by receptor desensitization with a TRPV1 agonist would therefore be expected to similarly protect, and potentially rescue, cardiac hypertrophy and its associated symptoms and outcomes (cardiac remodeling, cardiac fibrosis, apoptosis, hypertension, or heart failure).

However, there is currently no oral or other pharmacological composition of TRP agonist suitable for systemic administration and chronic downregulation of TRP in a visceral organ. There is therefore a need to develop such approaches in an analogous manner to the chronic pain approaches described above.

Creating nanoparticles is an effective way to alter the surface properties of active molecules to make them more bioavailable and to alter the kinetics of their release systemically. Oral nanoparticles can be created which provide efficient, time-released formulations of various TRP channel modulators, such as terpenoids and cannabinoids. Such oral formulations of TRP ligands and pharmacological compositions would provide novel and more effective ways of treating various diseases associated with the TRP channels, including pain disorders and cardiovascular diseases.

### SUMMARY OF THE INVENTION

Applicant recently demonstrated that myrcene is a new TRPV1 agonist that causes TRPV1 desensitization

after prolonged exposure. Applicant further showed that other terpenoids and cannabinoids, including those that do not demonstrate significant TRPV1 agonist activity on their own, act in combination to increase the efficacy of myrcene. Furthermore, multiple TRP channels additional to TRPV1 were identified as a target for myrcene, suggesting that efficacy of myrcene will likely extend beyond TRPV1 to other nociceptive neurons in which the primary pain conduction channel is a distinct TRP receptor. These findings are described in the PCT application, PCT/US2018/033956, which is incorporated by reference in its entirety herein.

The new findings suggest that myrcene, as well as other terpenoids and cannabinoids, can be an alternative therapeutic TRP receptor ligand that can affect multiple TRP channels additional to TRPV1, induce less discomfort during initial treatment than capsaicin, and is potentially suitable for systemic administration for chronic effects. See PCT/US2018/033956. Further development of a pharmaceutical composition comprising myrcene and other terpenoids and cannabinoids, in particular those allowing systemic administration and chronic and long-term release of myrcene, other terpenoid or cannabinoid, is required for therapeutic use of the new TRPV1 agonist and agonist mixtures. This is particularly important because terpenoids and cannabinoids are highly lipophilic and volatile with a low boiling point, and thus administration of terpenoids and cannabinoids have been limited to fast release medications or in natural product applications.

The present invention answers to the need to stabilize terpenoids and cannabinoids and make them more bioavailable with sustained release, by providing novel therapeutic compositions for systemic administration *(e.g.,* oral administration) and long-term release of terpenoids and cannabinoids, and methods of making and using the therapeutic compositions. In particular, the present invention uses a PLGA nanoparticle encapsulating terpenoids and cannabinoids for delivering said terpenoids and cannabinoids to mammalian subjects. Additionally, the present disclosure provides therapeutic compositions containing more than one terpenoid or cannabinoid. The therapeutic compositions can contain more than one populations of nanoparticles, each population encapsulating an individual terpenoid or cannabinoid, or a population of nanoparticles, each nanoparticle encapsulating more than one terpenoid or cannabinoid. Terpenoids or cannabinoids encapsulated in nanoparticles can be synthesized compounds or compounds isolated from chemical or natural mixtures. In some embodiments, the compounds are isolated from *Cannabis sativa* extracts. In some embodiments, delta-9 tetrahydrocannabinol is additionally or separately encapsulated in nanoparticles.

Accordingly, in a first aspect, provided herein are terpenoid-encapsulating PLGA nanoparticles comprising a PLGA nanoparticle and a first terpenoid encapsulated in the PLGA nanoparticle.

In some embodiments, wherein the PLGA nanoparticle comprises PLGA copolymer and the weight ratio between the first terpenoid and PLGA copolymer is between 1:50 and 1:10. In one embodiment, the weight ratio between the first terpenoid and PLGA copolymer is about 1:14.

In some embodiments, the first terpenoid is selected from the group consisting of myrcene, β-caryophyllene, and nerolidol. In some embodiments, wherein the first terpenoid is myrcene, and the weight ratio between the first terpenoid and the PLGA copolymer is about 1:22. In some embodiments, wherein the first terpenoid is β-caryophyllene, and the weight ratio between the first terpenoid and the PLGA copolymer is between 1:5 and 1:7. In some embodiments, the first terpenoid is nerolidol, and the weight ratio between the first terpenoid and the PLGA copolymer is between 1:5 and 1:7.

In some embodiments, the PLGA nanoparticle has surface modified with PEG. Molecular weight of PEG can be in the range of 2,000-20,000 Da.

In some embodiments, the nanoparticle further encapsulates at least a second compound, wherein the second encapsulated compound is (i) a cannabinoid or (ii) a second terpenoid other than the first terpenoid. In some embodiments, the second encapsulated compound is cannabigerolic acid (CBGA). In some embodiments, the second encapsulated compound is cannabidiol (CBD). In some embodiments, the second encapsulated compound is cannabinol (CBN). In some embodiments, the second encapsulated compound is cannabidivarin (CBDV). In some embodiments, the second encapsulated compound is cannabichromene (CBC). In some embodiments, the second encapsulated compound is cannabidiolic acid (CBDA). In some embodiments, the second encapsulated compound is cannabigerol (CBG). In some embodiments, the second encapsulated compound is myrcene, β-caryophyllene, or nerolidol. In some embodiments, the second encapsulated compound is limonene. In some embodiments, the second encapsulated compound is phytol. In some embodiments, the second encapsulated compound is pinene. In some embodiments, the second encapsulated compound is linalool.

In some embodiments, the PLGA nanoparticles have an average diameter between 200-350 nm.

In some embodiments, the PLGA nanoparticles comprise PLGA copolymer having a ratio of lactic acid to glycolic acid between about 10-90% lactic acid and about 90-10% glycolic acid. In some embodiments, the ratio of lactic acid to glycolic acid is about 10% lactic acid to about 90% glycolic acid. In some embodiments, the ratio of lactic acid to glycolic acid is about 25% lactic acid to about 75% glycolic acid. In some embodiments, the ratio of lactic acid to glycolic acid is about 50% lactic acid to about 50% glycolic acid. In some embodiments, the ratio of lactic acid to glycolic acid is about 75% lactic acid to about 25% glycolic acid. In some embodiments, the ratio of lactic acid to glycolic acid is about 90% lactic acid to about 10% glycolic acid.

In another aspect, the present disclosure provides a pharmaceutical composition comprising a first population of terpenoid-encapsulating PLGA nanoparticles described herein, and a pharmaceutically acceptable carrier or diluent. In some embodiments, the pharmaceutical composition further comprises trehalose. In some embodiments, the composition is lyophilized. In some embodiments, the composition further comprises a second population of PLGA nanoparticles, wherein the second population of nanoparticles encapsulates a third compound, wherein the third compound is a cannabinoid or terpenoid different from the cannabinoids and terpenoids encapsulated in the first population of nanoparticles.

In some embodiments, the third encapsulated compound is cannabigerolic acid (CBGA). In some embodiments, the third encapsulated compound is cannabidiol (CBD). In some embodiments, the third encapsulated compound is cannabinol (CBN). In some embodiments, the third encapsulated compound is cannabidivarin (CBDV). In some embodiments, the third encapsulated compound is cannabichromene (CBC). In some embodiments, the third encapsulated compound is cannabidiolic acid (CBDA). In some embodiments, the third encapsulated compound is cannabigerol (CBG). In some embodiments, the third encapsulated compound is myrcene, β-caryophyllene, or nerolidol. In some embodiments, the third encapsulated compound is limonene. In some embodiments, the third encapsulated compound is phytol. In some embodiments, the third encapsulated compound is pinene. In some embodiments, the third encapsulated compound is linalool.

In some embodiments, the composition further comprises a third population of PLGA nanoparticles, wherein the third population of nanoparticles encapsulates a fourth compound, wherein the fourth compound is a cannabinoid or terpenoid different from the cannabinoids and terpenoids encapsulated in the first population of nanoparticles or the second population of nanoparticles. In some embodiments, the composition further comprises a fourth population of PLGA nanoparticles, wherein the fourth population of nanoparticles encapsulates a fifth compound, wherein the fifth compound is a cannabinoid or terpenoid different from the cannabinoids and terpenoids encapsulated in the first population of nanoparticles, the second population of nanoparticles or the third population of nanoparticles.

In yet another aspect, the present disclosure provides a method for obtaining terpenoid-encapsulating PLGA nanoparticles, wherein the method comprises the steps of: (a) providing an organic solution comprising terpenoid, a PLGA copolymer, and a solvent, and an aqueous solution comprising a surfactant; (b) emulsifying the two solutions to form a suspension of the terpenoid-encapsulating PLGA nanoparticles; (c) evaporating the solvent from theemulsion; and (d)obtaining the terpenoid-encapsulating PLGA nanoparticles.

In some embodiments, the weight ratio of the first terpenoid to PLGA copolymer in the solution of step (a) is from about 1:5 to about 1:1. In some embodiments, the weight ratio of the first terpenoid to PLGA copolymer in the solution of step (a) is about 1:5. In some embodiments, the weight ratio of the first terpenoid to PLGA copolymer in the solution of step (a) is about 1:4. In some embodiments, the weight ratio of the first terpenoid to PLGA copolymer in the solution of step (a) is about 1:3. In some embodiments, the weight ratio of the first terpenoid to PLGA copolymer in the solution of step (a) is about 1:2. In some embodiments, the weight ratio of the first terpenoid to PLGA copolymer in the solution of step (a) is about 1:1.

In some embodiments, the entrapment efficiency of the first terpenoid in step (b) is between about 4% to about 10%. In some embodiments, the entrapment efficiency is at least 4%. In some embodiments, the entrapment efficiency is at least 5%. In some embodiments, the entrapment efficiency is at least 6%. In some embodiments, the entrapment efficiency is at least 7%. In some embodiments, the entrapment efficiency is at least 8%. In some embodiments, the entrapment efficiency is at least 9%. In some embodiments, the entrapment efficiency is at least 10%.

In some embodiments, the average weight ratio between encapsulated terpenoid and PLGA copolymer in the terpenoid-encapsulating PLGA nanoparticles of step (d) is between about 1:50 and about 1:10. In some embodiments, the average weight ratio between encapsulated terpenoid and PLGA copolymer in the terpenoid-encapsulating PLGA nanoparticles is at least about 1:50. In some embodiments, the average weight ratio between encapsulated terpenoid and PLGA copolymer in the terpenoid-encapsulating PLGA nanoparticles is at least about 1:40. In some embodiments, the average weight ratio between encapsulated terpenoid and PLGA copolymer in the terpenoid-encapsulating PLGA nanoparticles is at least about 1:30. In some embodiments, the average weight ratio between encapsulated terpenoid and PLGA copolymer in the terpenoid-encapsulating PLGA nanoparticles is at least about 1:20. In some embodiments, the average weight ratio between encapsulated terpenoid and PLGA copolymer in the terpenoid-encapsulating PLGA nanoparticles is at least about 1:10.

In some embodiments, the PLGA copolymer has a ratio of lactic acid to glycolic acid between about 10-90% lactic acid and about 90-10% glycolic acid. In some embodiments, the ratio of lactic acid to glycolic acid is about 10% lactic acid to about 90% glycolic acid. In some embodiments, the ratio of lactic acid to glycolic acid is about 25% lactic acid to about 75% glycolic acid. In some embodiments, the ratio of lactic acid to glycolic acid is about 50% lactic acid to about 50% glycolic acid. In some embodiments, the ratio of lactic acid to glycolic acid is about 75% lactic acid to about 25% glycolic acid. In some embodiments, the ratio of lactic acid to glycolic acid is about 90% lactic acid to about 10% glycolic acid.

In some embodiments, the solution of step (a) further comprises at least one cannabinoid or terpenoid other

than the first terpenoid. In some embodiments, the at least one cannabinoid or terpenoid is selected from the group consisting of: cannabidiol (CBD), cannabinol (CBN), cannabidivarin (CBDV), cannabichromene (CBC), cannabidiolic acid (CBDA), and cannabigerol (CBG). In some embodiments, the at least one cannabinoid or terpenoid is selected from the group consisting of: myrcene, β-caryophyllene, nerolidol, phytol, limonene, linalool, and pinene.

In some embodiments, the solvent is acetone, dichloromethane, or ethyl acetate. In some embodiments, the surfactant is polyethylene glycol, a poloxamer, or polyvinyl alcohol (PVA). In some embodiments, the surfactant is polyvinyl alcohol (PVA).

In some embodiments, the step of emulsifying comprises homogenization or sonication. In some embodiments, the step of emulsifying is homogenization. In some embodiments, the homogenization is performed at 20,000 to 30,000 rpm. In some embodiments, the homogenization is performed at 24,000 rpm. In some embodiments, the solution is homogenized for 30s to 10 min. In some embodiments, the solution is homogenized for 1 min.

In some embodiments, the step of evaporating the solvent comprises at least one of stirring the solvent, applying gas streams, applying heat, maintaining cold temperature of 10 °C, or creating a vacuum. In some embodiments, the step of evaporating the solvent comprises stirring the suspension at room temperature. In some embodiments, the suspension is stirred for 5 min to 120 min to evaporate the solvent. In some embodiments, the suspension is stirred for 60 min.

In some embodiments, the step of obtaining the terpenoid-encapsulating PLGA nanoparticles comprises centrifugation, filtration, or centrifugation and filtration. In some embodiments, the step of obtaining comprises centrifugation. In some embodiments, the centrifugation is performed between 2,000 x g and 15,000 x g. In some embodiments, the centrifugation is at 4,000 x g.

In some embodiments, the method further comprises the subsequent step of adding a cryoprotectant to the terpenoid-encapsulating PLGA nanoparticles. In some embodiments, the cryoprotectant is trehalose. In some embodiments, the cryoprotectant is added in an amount of 1-10% (w/v) of the terpenoid-encapsulating PLGA nanoparticles. In some embodiments, the cryoprotectant is added in an amount of 5% (w/v) of the terpenoid-encapsulating PLGA nanoparticles.

In some embodiments, the method further comprises lyophilizing the terpenoid-encapsulating PLGA nanoparticles.

In one aspect, the present disclosure provides a terpenoid-encapsulating PLGA nanoparticle, obtained by the method of described herein.

In another aspect, the present disclosure provides a method of effecting TRPV1 desensitization in cells of a mammalian subject comprising: administering to the mammalian subject the pharmaceutical composition described herein in an amount, by a route of administration, and for a time sufficient to cause TRPV1 desensitization in cells within the mammalian subject. In some embodiments, the cells are nociceptors. In some embodiments, the nociceptors are peripheral nociceptors. In some embodiments, the nociceptors are visceral nociceptors.

In some embodiments, the pharmaceutical composition is administered orally. In some embodiments, the pharmaceutical composition is administered topically. In some embodiments, the pharmaceutical composition is administered systemically. In some embodiments, the pharmaceutical composition is administered intravenously. In some embodiments, the pharmaceutical composition is administered subcutaneously. In some embodiments, the pharmaceutical composition is administered by inhalation.

In one aspect, the present disclosure provides a method of treating pain in a mammalian subject, the method comprising the step of: administering to the subject the pharmaceutical composition described herein in an amount, by a route of administration, and for a time sufficient to cause TRPV1 desensitization in nociceptors within the subject.

In some embodiments, the nociceptors are peripheral nociceptors, and the pharmaceutical composition is administered topically. In some embodiments, the nociceptors are visceral nociceptors, and the pharmaceutical composition is administered systemically.

In some embodiments, the pain is neuropathic pain. In some embodiments, the neuropathic pain is diabetic peripheral neuropathic pain. In some embodiments, the pain is post-herpetic neuralgia.

In some embodiments, the pharmaceutical composition is administered at least once a day for at least 3 days. In some embodiments, the pharmaceutical composition is administered at least once a day for at least 5 days. In some embodiments, the pharmaceutical composition is administered at least once a day for at least 7 days.

In some embodiments, the pharmaceutical composition is administered at a dose, by a route of administration, and on a schedule sufficient to maintain levels of the first terpenoid at the nociceptors for at least 3 days that are effective to desensitize TRPV1 in nociceptors. In some embodiments, the pharmaceutical composition is administered at a dose, by a route of administration, and on a schedule sufficient to maintain levels of the first terpenoid at the nociceptors for at least 5 days that are effective to desensitize TRPV1 in nociceptors. In some embodiments, the pharmaceutical composition is administered at a dose, by a route of administration, and on a schedule sufficient to maintain levels of the first terpenoid at the nociceptors for at least 7 days that are effective to desensitize TRPV1 in nociceptors.

In one aspect, the present disclosure provides a method of treating cardiac hypertrophy in a mammalian subject,

comprising the step of: administering to the subject an anti-hypertrophic effective amount of the pharmaceutical composition described herein. In some embodiments, the pharmaceutical composition is administered orally. In some embodiments, the pharmaceutical composition is administered systemically. In some embodiments, the pharmaceutical composition is administered intravenously. In some embodiments, the pharmaceutical composition is administered subcutaneously. In some embodiments, the pharmaceutical composition is administered by inhalation. In some embodiments, the pharmaceutical composition is administered orally.

In one aspect, the present disclosure provides a method of prophylactic treatment of cardiac hypertrophy in a mammalian subject, comprising the step of: administering to a subject at risk of cardiac hypertrophy an anti-hypertrophic effective amount of the pharmaceutical composition described herein.

In one aspect, the present disclosure provides a method of treating overactive bladder in a mammalian subject, comprising the step of: administering to the subject a therapeutically effective amount of the pharmaceutical composition provided herein. In some embodiments, the pharmaceutical composition is administered systemically. In some embodiments, the pharmaceutical composition is administered by bladder irrigation.

In one aspect, the present disclosure provides a method of treating refractory chronic cough in a mammalian subject, comprising the step of: administering to the subject a therapeutically effective amount of the pharmaceutical composition described herein. In some embodiments, the pharmaceutical composition is administered systemically. In some embodiments, the pharmaceutical composition is administered by inhalation.

In another aspect, the present disclosure provides a cannabinoid-encapsulating PLGA nanoparticle, comprising: a PLGA nanoparticle and a first cannabinoid encapsulated in the PLGA nanoparticle.

In some embodiments, the PLGA nanoparticle comprises PLGA copolymer and the weight ratio between the first cannabinoid and the PLGA copolymer is between 1:50 and 1:4. In some embodiments, the weight ratio between the first cannabinoid and the PLGA copolymer is between 1:25 and 1:5. In some embodiments, the first cannabinoid is selected from the group consisting of cannabidiol, cannabidivarin, cannabinol, cannabigerol, and cannabichromene.

In some embodiments, the first cannabinoid is cannabidiol, and the weight ratio between the first cannabinoid and the PLGA copolymer is about 1:14. In some embodiments, the first cannabinoid is cannabidiol, and the weight ratio between the first cannabinoid and the PLGA copolymer is between 1:5 and 1:7. In some embodiments, the first cannabinoid is cannabidiol, and the weight ratio between the first cannabinoid and the PLGA copolymer is between 1:5 and 1:7.

In some embodiments, the nanoparticle further encapsulates at least a second compound, wherein the second encapsulated compound is (i) a terpenoid or (ii) a second cannabinoid other than the first cannabinoid. In some embodiments, the second encapsulated compound is cannabigerolic acid (CBGA). In some embodiments, the second encapsulated compound is cannabidivarin (CBV). In some embodiments, the second encapsulated compound is cannabinol (CBN). In some embodiments, the second encapsulated compound is cannabidivarin (CBDV). In some embodiments, the second encapsulated compound is cannabichromene (CBC). In some embodiments, the second encapsulated compound is cannabidiolic acid (CBDA). In some embodiments, the second encapsulated compound is cannabigerol (CBG). In some embodiments, the second encapsulated compound is myrcene, β-caryophyllene, or nerolidol. In some embodiments, the second encapsulated compound is limonene. In some embodiments, the second encapsulated compound is phytol. In some embodiments, the second encapsulated compound is pinene. In some embodiments, the second encapsulated compound is linalool. In some embodiments, the second encapsulated compound is myrcene.

In some embodiments, the PLGA nanoparticles have an average diameter between 200-350 nm. In some embodiments, the PLGA nanoparticles comprise PLGA copolymer having a ratio of lactic acid to glycolic acid between about 10-90% lactic acid and about 90-10% glycolic acid. In some embodiments, the ratio of lactic acid to glycolic acid is about 10% lactic acid to about 90% glycolic acid. In some embodiments, the ratio of lactic acid to glycolic acid is about 25% lactic acid to about 75% glycolic acid. In some embodiments, the ratio of lactic acid to glycolic acid is about 50% lactic acid to about 50% glycolic acid. In some embodiments, the ratio of lactic acid to glycolic acid is about 75% lactic acid to about 25% glycolic acid. In some embodiments, the ratio of lactic acid to glycolic acid is about 90% lactic acid to about 10% glycolic acid.

One aspect of the present disclosure provides a pharmaceutical composition comprising a first population of cannabinoid-encapsulating PLGA nanoparticles provided herein, and a pharmaceutically acceptable carrier or diluent. In some embodiments, the pharmaceutical composition further comprises trehalose.

In some embodiments, the composition is lyophilized. In some embodiments, the composition further comprises a second population of PLGA nanoparticles, wherein the second population of nanoparticles encapsulates a third compound, wherein the third compound is a terpenoid or a cannabinoid different from the terpenoids and cannabinoids encapsulated in the first population of nanoparticles. In some embodiments, the third encapsulated compound is cannabigerolic acid (CBGA). In some embodiments, the third encapsulated compound is cannabidiol (CBD). In some embodiments, the third encapsulated compound is cannabinol (CBN). In some embodiments, the third encapsulated compound is cannabidivarin (CBDV). In some embodiments, the third encapsulated compound is cannabichromene (CBC). In some embodiments, the third encapsulated compound is cannabidiolic acid (CBDA). In some embodiments, the third

encapsulated compound is cannabigerol (CBG). In some embodiments, the third encapsulated compound is myrcene, β-caryophyllene, or nerolidol. In some embodiments, the third encapsulated compound is limonene. In some embodiments, the third encapsulated compound is phytol. In some embodiments, the third encapsulated compound is pinene. In some embodiments, the third encapsulated compound is linalool. In some embodiments, the third encapsulated compound is myrcene.

In some embodiments, the composition further comprises a third population of PLGA nanoparticles, wherein the third population of nanoparticles encapsulates a fourth compound, wherein the fourth compound is a cannabinoid or a terpenoid different from the cannabinoids and terpenoids encapsulated in the first population of nanoparticles or the second population of nanoparticles. In some embodiments, the composition further comprises a fourth population of PLGA nanoparticles, wherein the fourth population of nanoparticles encapsulates a fifth compound, wherein the fifth compound is a cannabinoid or a terpenoid different from the cannabinoids and terpenoids encapsulated in the first population of nanoparticles, the second population of nanoparticles or the third population of nanoparticles.

Another aspect of the present disclosure provides a method for obtaining cannabinoid-encapsulating PLGA nanoparticles, wherein the method comprises the steps of: (a) providing an organic solution comprising terpenoid, a PLGA copolymer, and a solvent, and an aqueous solution comprising a surfactant; (b)emulsifying the two solutions to form a suspension of the cannabinoid-encapsulating PLGA nanoparticles; (c) evaporating the solvent from theemulsion; and (d) obtaining the cannabinoid-encapsulating PLGA nanoparticles.

In some embodiments, the weight ratio of the first cannabinoid to PLGA copolymer in the solution of step (a) is from about 1:5 to about 1:1. In some embodiments, the weight ratio of the first cannabinoid to PLGA copolymer in the solution of step (a) is about 1:5. In some embodiments, the weight ratio of the first cannabinoid to PLGA copolymer in the solution of step (a) is about 1:4. In some embodiments, the weight ratio of the first cannabinoid to PLGA copolymer in the solution of step (a) is about 1:3. In some embodiments, the weight ratio of the first cannabinoid to PLGA copolymer in the solution of step (a) is about 1:2. In some embodiments, the weight ratio of the first cannabinoid to PLGA copolymer in the solution of step (a) is about 1:1.

In some embodiments, the entrapment efficiency of the first cannabinoid in step (b) is between about 4% to about 10%. In some embodiments, the entrapment efficiency is at least 4%. In some embodiments, the entrapment efficiency is at least 5%. In some embodiments, the entrapment efficiency is at least 6%. In some embodiments, the entrapment efficiency is at least 7%. In some embodiments, the entrapment efficiency is at least 8%. In some embodiments, the entrapment efficiency is at least 9%. In some embodiments, the entrapment efficiency is at least 10%.

In some embodiments, the average weight ratio between encapsulated cannabinoid and PLGA copolymer in the cannabinoid-encapsulating PLGA nanoparticles of step (d) is between about 1:50 and about 1:10. In some embodiments, the average weight ratio between encapsulated cannabinoid and PLGA copolymer in the cannabinoid-encapsulating PLGA nanoparticles is at least about 1:50. In some embodiments, the average weight ratio between encapsulated cannabinoid and PLGA copolymer in the cannabinoid-encapsulating PLGA nanoparticles is at least about 1:40. In some embodiments, the average weight ratio between encapsulated cannabinoid and PLGA copolymer in the cannabinoid-encapsulating PLGA nanoparticles is at least about 1:30. In some embodiments, the average weight ratio between encapsulated cannabinoid and PLGA copolymer in the cannabinoid-encapsulating PLGA nanoparticles is at least about 1:20. In some embodiments, the average weight ratio between encapsulated cannabinoid and PLGA copolymer in the cannabinoid-encapsulating PLGA nanoparticles is at least about 1:10.

In some embodiments, the PLGA copolymer has a ratio of lactic acid to glycolic acid between about 10-90% lactic acid and about 90-10% glycolic acid. In some embodiments, the ratio of lactic acid to glycolic acid is about 10% lactic acid to about 90% glycolic acid. In some embodiments, the ratio of lactic acid to glycolic acid is about 25% lactic acid to about 75% glycolic acid. In some embodiments, the ratio of lactic acid to glycolic acid is about 50% lactic acid to about 50% glycolic acid. In some embodiments, the ratio of lactic acid to glycolic acid is about 75% lactic acid to about 25% glycolic acid. In some embodiments, the ratio of lactic acid to glycolic acid is about 90% lactic acid to about 10% glycolic acid.

In some embodiments, the solution of step (a) further comprises at least one cannabinoid or terpenoid other than the first cannabinoid. In some embodiments, the at least one cannabinoid or terpenoid is selected from the group consisting of: cannabinol (CBN), cannabidivarin (CBDV), cannabichromene (CBC), cannabidiolic acid (CBDA), and cannabigerol (CBG). In some embodiments, the at least one cannabinoid or terpenoid is selected from the group consisting of: myrcene, β-caryophyllene, nerolidol, phytol, limonene, linalool, and pinene.

In some embodiments, the solvent is acetone, dichloromethane, or ethyl acetate. In some embodiments, the surfactant is polyethylene glycol, a poloxamer, or polyvinyl alcohol (PVA). In some embodiments, the surfactant is polyvinyl alcohol (PVA).

In some embodiments, the step of emulsifying comprises homogenization or sonication. In some embodiments, the step of emulsifying is homogenization. In some embodiments, the homogenization is performed at 20,000 to 30,000 rpm. In some embodiments, the homogenization is performed at 24,000 rpm. In some embodiments, the solution is homogenized for 30 seconds to 10 min. In some embodiments, the solution is homogenized for 1 min.

In some embodiments, the step of evaporating the solvent comprises at least one of stirring the solvent, applying gas streams, applying heat, maintaining cold temperature of 10°C, or creating a vacuum. In some embodiments, the step of evaporating the solvent comprises stirring the suspension at room temperature. In some embodiments, the suspension is stirred for 5 min to 120 min to evaporate the solvent. In some embodiments, the suspension is stirred for 60 min.

In some embodiments, the step of obtaining the cannabinoid-encapsulating PLGA nanoparticles comprises centrifugation, filtration, or centrifugation and filtration. In some embodiments, the step of obtaining comprises centrifugation. In some embodiments, the centrifugation is performed between 2,000 x g and 15,000 x g. In some embodiments, the centrifugation is at 4,000 x g.

In some embodiments, the method further comprises the subsequent step of adding a cryoprotectant to the cannabinoid-encapsulating PLGA nanoparticles. In some embodiments, the cryoprotectant is trehalose. In some embodiments, the cryoprotectant is added in an amount of 1-10% (w/v) of the cannabinoid-encapsulating PLGA nanoparticles. In some embodiments, the cryoprotectant is added in an amount of 5% (w/v) of the cannabinoid-encapsulating PLGA nanoparticles.

In some embodiments, the method further comprises lyophilizing the cannabinoid-encapsulating PLGA nanoparticles.

In one aspect, the present disclosure provides a cannabinoid-encapsulating PLGA nanoparticle, obtained by the method described herein.

In another aspect, the present disclosure provides a method of effecting TRPV1 desensitization in cells of a mammalian subject comprising: administering to the mammalian subject the pharmaceutical composition provided herein in an amount, by a route of administration, and for a time sufficient to cause TRPV1 desensitization in cells within the mammalian subject. In some embodiments, the cells are nociceptors. In some embodiments, the nociceptors are peripheral nociceptors. In some embodiments, the nociceptors are visceral nociceptors.

In some embodiments, the pharmaceutical composition is administered orally. In some embodiments, the pharmaceutical composition is administered topically. In some embodiments, the pharmaceutical composition is administered systemically. In some embodiments, the pharmaceutical composition is administered intravenously. In some embodiments, the pharmaceutical composition is administered subcutaneously. In some embodiments, the pharmaceutical composition is administered by inhalation.

In yet another aspect, the present disclosure provides a method of treating pain in a mammalian subject, the method comprising the step of: administering to the subject the pharmaceutical composition provided herein in an amount, by a route of administration, and for a time sufficient to cause TRPV1 desensitization in nociceptors within the subject.

In some embodiments, the nociceptors are peripheral nociceptors, and the pharmaceutical composition is administered topically. In some embodiments, the nociceptors are visceral nociceptors, and the pharmaceutical composition is administered systemically.

In some embodiments, the pain is neuropathic pain. In some embodiments, the neuropathic pain is diabetic peripheral neuropathic pain. In some embodiments, the pain is post-herpetic neuralgia.

In some embodiments, the pharmaceutical composition is administered at least once a day for at least 3 days. In some embodiments, the pharmaceutical composition is administered at least once a day for at least 5 days. In some embodiments, the pharmaceutical composition is administered at least once a day for at least 7 days. In some embodiments, the pharmaceutical composition is administered at least once a day for more than 7 days. In some embodiments, the pharmaceutical composition is administered at a dose, by a route of administration, and on a schedule sufficient to maintain levels of cannabinoid at the nociceptors for at least 3 days that are effective to desensitize TRPV1 in nociceptors. In some embodiments, the pharmaceutical composition is administered at a dose, by a route of administration, and on a schedule sufficient to maintain levels of cannabinoid at the nociceptors for at least 5 days that are effective to desensitize TRPV1 in nociceptors. cannabinoid the pharmaceutical composition is administered at a dose, by a route of administration, and on a schedule sufficient to maintain levels of cannabinoid at the nociceptors for at least 7 days that are effective to desensitize TRPV1 in nociceptors.

One aspect of the present disclosure provides a method of treating cardiac hypertrophy in a mammalian subject, comprising the step of: administering to the subject an anti-hypertrophic effective amount of the pharmaceutical composition provided herein.

In some embodiments, the pharmaceutical composition is administered orally. In some embodiments, the pharmaceutical composition is administered systemically. In some embodiments, the pharmaceutical composition is administered intravenously. In some embodiments, the pharmaceutical composition is administered subcutaneously. In some embodiments, the pharmaceutical composition is administered by inhalation. In some embodiments, the pharmaceutical composition is administered orally.

Another aspect of the present disclosure provides a method of prophylactic treatment of cardiac hypertrophy in a mammalian subject, comprising the step of: administering to a subject at risk of cardiac hypertrophy an anti-hypertrophic effective amount of the pharmaceutical composition provided herein.

In yet another aspect, the present disclosure provides a method of treating overactive bladder in a mammalian subject, comprising the step of: administering to the subject a therapeutically effective amount of the pharmaceutical composition provided herein.

In some embodiments, the pharmaceutical composition is administered systemically. In some embodiments, the pharmaceutical composition is administered by bladder irrigation.

In one aspect, the present disclosure provides a method of treating refractory chronic cough in a mammalian subject, comprising the step of: administering to the subject a therapeutically effective amount of the pharmaceutical composition provided herein.

In some embodiments, the pharmaceutical composition is administered systemically. In some embodiments, the pharmaceutical composition is administered by inhalation.

**BRIEF DESCRIPTION OF THE DRAWINGS**

These and other features, aspects, and advantages of the present invention will become better understood with regard to the following description, and accompanying drawings, where:

FIG. 1 provides a scheme of the nanoprecipitation method.
FIG. 2 provides a scheme of PLGA nanocapsules obtained using a microemulsion system.
FIG. 3 provides a scheme of the emulsification (high speed homogenizer) method for solvent evaporation.
FIG. 4A shows the gas chromatogram of myrcene, FIG. 4B mass spectrum of myrcene, and FIG. 4C shows the calibration curve of myrcene with concentrations ranging between 1-60 ppm as measured and analyzed by the GC-MS instrument of Thermo Scientific Instrument Delta V.
FIG. 5A shows the gas chromatogram of β-caryophyllene, FIG. 5B mass spectrum of β-caryophyllene, and FIG. 5C shows the calibration curve of β-caryophyllene with concentrations ranging between 1-60 ppm as measured and analyzed by the GC-MS instrument of Thermo Scientific Instrument Delta V.
FIG. 6 shows the gas chromatogram of both cis- and trans-nerolidol as measured and analyzed by the GC-MS instrument Thermo Scientific Instrument Delta V.
FIG. 7A shows the mas spectrum of cis-nerolidol, FIG. 7B shows the calibration curve of cis-nerolidol with concentrations between 1-60 ppm as measured and analyzed by the GC-MS instrument of Thermo Scientific Instrument Delta V. Similarly, FIG. 7C and FIG. 7D show the mass spectrum and calibration curve of trans-nerolidol, respectively, as measured and analyzed under the same conditions as the cis isomer.
FIGs. 8A-8F shows the mean diameter (FIGs 8A, 8C, and 8E) and zeta potential (FIGs. 8B, 8D, and 8F) measurements of nanoparticles (NPs) prepared using nanoprecipitation.
FIGs. 9A-9B shows the mean diameter (FIG. 9A) and zeta potential (FIG. 9B) measurements of NPs prepared using microemulsification.
FIGs. 10A-10B shows the mean diameter (FIG. 10A) and zeta potential (FIG. 10B) measurements of NPs prepared using emulsification with a homogenizer.
FIGs. 11A-11D shows the mean diameter (FIG. 11A and 11C) and zeta potential (FIG. 11B and 11D) measurements of NPs prepared by emulsification (FIG. 11A and 11B) and nanoprecipitation (FIG. 11C and 11D).
FIGs. 12A-12B shows the mean diameter (FIG. 12A) and zeta potential (FIG. 12B) measurements of an emulsion prepared by addition of myrcene dissolved in acetone to a solution of PVA 0.5% (w/w).
FIG. 13A and 13B show the scanning electron microscope (SEM) images of myrcene-containing nanoparticles (NPs).
FIG. 14A and 14B show the scanning electron microscope (SEM) images of β-caryophyllene-containing nanoparticles (NPs).
FIG. 15A and 15B show the scanning electron microscope (SEM) images of nerolidol-containing nanoparticles (NPs).
FIG. 16 shows a schematic representation of a nanocapsule.
FIG. 17 shows fluorescence changes measured using calcium signaling assay after treatment of HEK TRPV1 cells with both free and encapsulated terpenoids, including myrcene, nerolidol, and β-caryophyllene, and ionomycin as represented in a single graph.
FIGs. 18A-18C show individual representations of fluorescence changes measured using calcium signaling assay after treatment of HEK TRPV1 cells with non-encapsulated and encapsulated Myrcene (FIG. 18A), Nerolidol (FIG. 18B), and β-caryophyllene (FIG. 18C).
FIGs. 19A-19D show fluorescence changes measured using calcium signaling assay after treatment of HEK TRPV1 cells of individual non-encapsulated terpenoids in comparison to their corresponding combinations, namely FIG. 19A shows non-encapsulated myrcene, nerolidol and their combination; FIG. 19B shows non-encapsulated myrcene, β-caryophyllene and their combination; FIG. 19C shows non-encapsulated β-caryophyllene, nerolidol and their combination; and FIG. 19D shows non-encapsulated myrcene, nerolidol, β-caryophyllene, and their combination.
FIGs. 20A-20D show fluorescence changes measured using calcium signaling assay after treatment of HEK TRPV1

cells of individual nanoparticles (NPs) encapsulated terpenoids in comparison with their corresponding combinations, namely FIG. 20A shows results from myrcene NPs, nerolidol NPs, and their combination; FIG. 20B shows results from myrcene NPs, β-caryophyllene NPs, and their combination; FIG. 20C shows results from nerolidol NPs, β-caryophyllene NPs, and their combination; and FIG. 20D shows results from NPs containing myrcene, nerolidol, and β-caryophyllene, NPs containing myrcene and nerolidol, NPs containing myrcene and β-caryophyllene, and NPs containing nerolidol and β-caryophyllene.

FIGs. 21A-21D show fluorescence changes measured using calcium signaling assay after treatment of HEK TRPV1 cells of individually encapsulated terpenoid nanoparticles in comparison with their corresponding non-encapsulated terpenoid combinations. FIG. 21A shows the results from the combinations of non-encapsulated and encapsulated myrcene and nerolidol; FIG. 21B shows the results from the combinations of non-encapsulated and encapsulated myrcene and β-caryophyllene; FIG. 21C shows the results from the combinations of non-encapsulated and encapsulated nerolidol and β-caryophyllene; and FIG. 21D shows the results from the combinations of non-encapsulated and encapsulated myrcene, nerolidol, and β-caryophyllene.

## DETAILED DESCRIPTION OF THE INVENTION

### Definitions

Terms used in the claims and specification are defined as set forth below unless otherwise specified.

The term, **"myrcene"** or "**β-myrcene**" as used herein refers to 7-methyl-3-methylideneocta-1,6-diene and is illustrated by the structural formula

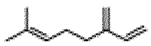.

The term "**α-ocimene**" as used herein refers *cis*-3,7-dimethyl-1,3,7-octatriene and is illustrated by the structural formula

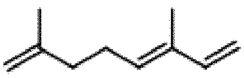.

The term "***cis*-β-ocimene**" as used herein refers to (Z)-3,7-dimethyl-1,3,6-octatriene and is illustrated by the structural formula

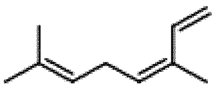.

The term "***trans*-β-ocimene**" as used herein refers to (E)-3,7-dimethyl-1,3,6-octatriene and is illustrated by the structural formula

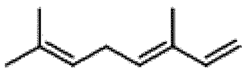.

The term **"linalool"** as used herein refers to 3,7-dimethyl-1,6-octadien-3-ol and is illustrated by the structural formula

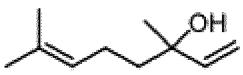.

The term **"nerolidol"** as used herein refers to 3,7,11-Trimethyl-1,6,10-dodecatrien-3-ol and is illustrated by the structural formula

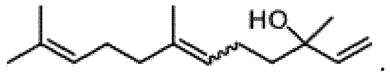

The term **"*cis*-nerolidol"** as used herein refers to the cis-isomer of nerolidol and is illustrated by the structural formula

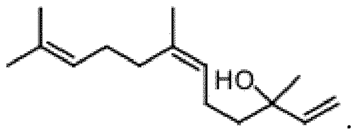

The term "***trans*-nerolidol**" as used herein refers to the trans-isomer of nerolidol and is illustrated by the structural formula

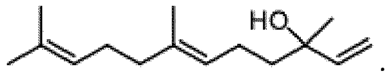

The term **"bisabolol"** as used herein refers to 6-methyl-2-(4-methylcyclohex-3-en-1-yl)hept-5-en-2-ol and is illustrated by the structural formula

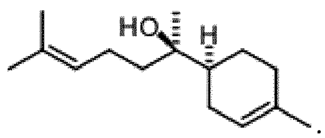

The term "**β-caryophyllene**" as used herein refers to (1*R*,4*E*,9*S*)-4,11,11-Trimethyl-8-methylidenebicyclo[7.2.0]undec-4-ene and is illustrated by the structural formula

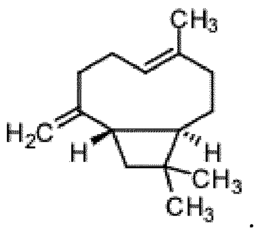

The term **"dimethylallyl"** group refers to an unsaturated $C_5H_9$ alkyl substituent as illustrated by the formula

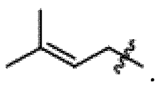

The term, **"terpenoids"** as used herein, refers to a class of organic compounds produced by plants, comprising alpha-bisabolol (a-bisabolol), alpha-humulene (a-humulene), alpha-pinene (a-pinene), β-caryophyllene (β-caryophyllene), myrcene, (+)-beta-pinene (β-pinene), camphene, limonene, linalool, phytol, and nerolidol.

The term, **"cannabinoid"** as used herein refers to a class of chemical compounds comprising cannabigerolic acid (CBGA), cannabidiol (CBD), cannabidivarin (CBDV), cannabichromene (CBC), cannabidiolic acid (CBDA), and cannabigerol (CBG).

**"Pharmaceutically active ingredient"** (synonymously, active pharmaceutical ingredient) means any substance or mixture of substances intended to be used in the manufacture of a drug product and that, when used in the production of a drug, becomes an active ingredient in the drug product. Such substances are intended to furnish pharmacological activity or other direct effect in the diagnosis, cure, mitigation, treatment or prevention of disease or to affect the structure and function of the body. Such substances or mixture of substances are preferably generated in compliance

with the Current Good Manufacturing Practice (CGMP) regulations pursuant to Section 501(a)(2)(B) of the Federal Food, Drug, and Cosmetic Act.

A pharmaceutically active ingredient is **"substantially free of THC"** if the ingredient contains less than 0.3% (w/w) of delta-9 tetrahydrocannabinol. A pharmaceutical composition is **"substantially free of THC"** if the pharmaceutical composition contains less than 0.3% (w/v) of delta-9 tetrahydrocannabinol.

A ***"Cannabis sativa* extract"** is a composition obtained from *Cannabis sativa* plant materials by fluid and/or gas extraction, for example by supercritical fluid extraction (SFE) with $CO_2$. The ***Cannabis sativa* extract** typically contains myrcene, cannabinoids, and terpenoids, and also can contain phytocannabinoids and other secondary metabolites.

The terms **"treatment," "treating,"** and the like are used herein to generally mean obtaining a desired pharmacologic and/or physiologic effect. The effect may be prophylactic, in terms of completely or partially preventing a disease, condition, or symptoms thereof, and/or may be therapeutic in terms of a partial or complete cure for a disease or condition and/or adverse effect, such as a symptom, attributable to the disease or condition. "Treatment" as used herein covers any treatment of a disease or condition of a mammal, particularly a human, and includes: (a) preventing the disease or condition from occurring in a subject which may be predisposed to the disease or condition but has not yet been diagnosed as having it; (b) inhibiting the disease or condition (*e.g.,* arresting its development); or (c) relieving the disease or condition (*e.g.,* causing regression of the disease or condition, providing improvement in one or more symptoms). Improvements in any conditions can be readily assessed according to standard methods and techniques known in the art. The population of subjects treated by the method includes subjects suffering from the undesirable condition or disease, as well as subjects at risk for development of the condition or disease.

By the term **"therapeutically effective dose"** or **"therapeutically effective amount"** is meant a dose or amount that produces the desired effect for which it is administered. The exact dose or amount will depend on the purpose of the treatment, and will be ascertainable by one skilled in the art using known techniques (*see, e.g.,* Lloyd (2012) The Art, Science and Technology of Pharmaceutical Compounding, Fourth Edition). A therapeutically effective amount can be a "prophylactically effective amount" as prophylaxis can be considered therapy.

The term **"sufficient amount"** means an amount sufficient to produce a desired effect.

The term **"ameliorating"** refers to any therapeutically beneficial result in the treatment of a disease state, *e.g.,* an immune disorder, including prophylaxis, lessening in the severity or progression, remission, or cure thereof.

The term **"mammal"** as used herein includes both humans and non-humans and include but is not limited to humans, non-human primates, canines, felines, murines, bovines, equines, and porcines.

The term **"PLGA nanoparticle"** as used herein refers to a nanoparticle comprising PLGA copolymers and encompasses both a PLGA nanocapsule and PLGA nanosphere. A PLGA nanocapsule is characterized by an outer PLGA polymeric membrane (shell) that surrounds an inner core of a substance, such as a drug compound like myrcene, other terpenoids, or cannabinoids. A PLGA nanosphere is characterized by a PLGA polymeric spherical matrix in which a substance or drug compound is dispersed or embedded.

The term **"myrcene-encapsulating PLGA nanoparticle"** as used herein refers to a nanoparticle made of PLGA copolymers that encapsulates or contains myrcene. The nanoparticle can be a PLGA nanosphere or a PLGA nanocapsule. The myrcene can be encapsulated as a liquid inner core inside a PLGA outer membrane (shell), forming a nanocapsule; or the myrcene can be dispersed or embedded in a PLGA matrix, forming a nanosphere.

The term **"cannabinoid-encapsulating PLGA nanoparticle"** as used herein refers to a nanoparticle made of PLGA copolymers that encapsulates or contains a cannnabinoid. The nanoparticle can be a PLGA nanosphere or a PLGA nanocapsule. The cannabinoid can be encapsulated as a liquid inner core inside a PLGA outer membrane (shell), forming a nanocapsule; or the cannabinoid can be dispersed or embedded in a PLGA matrix, forming a nanosphere.

The term **"terpenoid-encapsulating PLGA nanoparticle"** as used herein refers to a nanoparticle made of PLGA copolymers that encapsulates or contains a terpenoid. The nanoparticle can be a PLGA nanosphere or a PLGA nanocapsule. The terpenoid can be encapsulated as a liquid inner core inside a PLGA outer membrane (shell), forming a nanocapsule; or the terpenoid can be dispersed or embedded in a PLGA matrix, forming a nanosphere.

The term "**terpenoid/cannabinoid-encapsulating PLGA nanoparticle**" as used herein refers to a terpene-encapsulating PLGA nanoparticle or a cannabinoid-encapsulating PLGA nanoparticle.

The terms **"free terpenoids"** and **"non-encapsulated terpenoids"** as used herein refer to terpenoids that are not encapsulated inside a nanoparticle and are in bulk solution. These terms are used interchangeably through the specification.

The term **"PLGA polymer"** as used herein refers to poly(lactic-co-glycolic acid).

The term **"lyophilization"** as used herein refers to a low temperature dehydration process, in which water is removed from a material by freezing the material under low pressure or in a vacuum. The water freezes and then is removed from the material via sublimation. Alternative terms for the process include freeze drying.

The term **"entrapment efficiency"** or **"EE%"** as used herein refers to the percentage of the mass of the compound in the nanoparticles as compared to the starting mass of the compound. The EE percentage is calculated

using the following equation. "CPD" is an abbreviation of "compound" and indicates the drug compound encapsulated in the nanoparticles, such as a myrcene or other terpenoids or cannabinoids.

The term **"drug loading"** or **"DL%"** as used herein refers to the mass of compound incorporated into the nanoparticles as compared to the total mass of the nanoparticles including the compound. The DL percentage is calculated using the following equation. "CPD" indicates the drug compound encapsulated in the nanoparticles, such as a myrcene or other terpenoids or cannabinoids.

$$DL\ (\%) = \frac{mass\ of\ CPDs\ \ incorporated\ \ (mg)}{mass\ of\ nanoparticles\ \ (mg)} \times 100$$

It must be noted that, as used in the specification and the appended claims, the singular forms "a," "an" and "the" include plural referents unless the context clearly dictates otherwise.

Ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

Unless otherwise indicated, reference to a compound that has one or more stereo centers intends each stereoisomer, and all combinations of stereoisomers, thereof.

**Terpenoid/cannabinoid-encapsulating PLGA nanoparticles**

*Terpenoid-encaysulating PLGA nanoparticles*

In one aspect, the present invention provides a terpenoid-encapsulating PLGA nanoparticle comprising a PLGA nanoparticle and a first terpenoid encapsulated in the PLGA nanoparticle. The terpenoid-encapsulating PLGA nanoparticle can encapsulate compounds other than the first terpenoid, such as other terpenoids and/or cannabinoids.

The first terpenoid encapsulated in the PLGA nanoparticle can be a terpenoid extractable from *Cannabis sativa.*

The first terpenoid can be myrcene, β-caryophyllene, or nerolidol.

In various such embodiments, the first terpenoid, optional cannabinoids, and optional terpenoids other than the first terpenoid collectively constitute at least 75% by weight, but less than 100 wt%, of the total compounds encapsulated in the PLGA nanoparticles. In specific embodiments, the first terpenoid, optional cannabinoids, and optional terpenoids other than the first terpenoid collectively constitute at least 80%, at least at least 85%, at least 90%, at least 91%, at least 92%, at least 93%, at least 94%, or at least 95% by weight, but less than 100 wt%, of the total compounds encapsulated in the PLGA nanoparticles. In particular embodiments, the first terpenoid, optional cannabinoids, and optional terpenoids other than the first terpenoid collectively constitute at least 96%, at least 97%, at least 98%, or at least 99% by weight, but less than 100 wt%, of the total compounds encapsulated in the PLGA nanoparticles.

In embodiments in which the first terpenoid, optional cannabinoids, and optional terpenoids other than the first terpenoid collectively constitute less than 100% by weight (wt%) of the total compounds encapsulated in the PLGA nanoparticles, the PLGA nanoparticles can further encapsulate compounds other than the first terpenoid, optional cannabinoids, or optional terpenoids. In such embodiments, the additional compound can be extractable from *Cannabis sativa.* In specific embodiments, all or some compounds encapsulated in the PLGA nanoparticle are present in an extract made from *Cannabis sativa.*

The amount of the first terpenoid encapsulated in the PLGA nanoparticle can be quantified as a weight ratio between the weight of PLGA copolymer in the nanoparticle and the amount of the first terpenoid encapsulated within the nanoparticle. The weight ratio between the first terpenoid and PLGA copolymer can be between 1:50 and 1:1. The weight ratio between the first terpenoid and PLGA copolymer can be between 1:100 and 1:5, 1:75 and 1:10, 1:50 and 1:10, 1:40 and 1:10, 1:30 and 1:10, 1:20 and 1:10, 1:50 and 1:40, 1:40 and 1:30, 1:30 and 1:20, 1:20 and 1:10, 1:10 and 1:5 and 1:5 and 1:1. The weight ratio between the first terpenoid and PLGA copolymer can be 1:50, 1:45, 1:40, 1:35, 1:30, 1:25, 1:20, 1:19, 1:17, 1:18, 1:16, 1:15, 1:14, 1:13, 1:12, 1:11 1:10, 1:9, 1:8, 1:7, 1:6, 1:5, 1:4, 1:3, 1:2 or 1:1. In a particular embodiment, the ratio is about 1:14. In a particular embodiment, the ratio is about 1:22.

In some embodiments, the weight ratio between the first terpenoid and PLGA copolymer is between 1:50 and 1:10. In one embodiment, the weight ratio between the first terpenoid and PLGA copolymer is about 1:14. In one embodiment, the weight ratio between the first terpenoid and PLGA copolymer is about 1:22.

In one embodiment, terpenoid encapsulated in the nanoparticle is myrcene. The amount of myrcene encapsulated in the PLGA nanoparticle can be quantified as a weight ratio between the weight of PLGA copolymer in the PLGA nanoparticle and the amount of the myrcene encapsulated within the nanoparticle. The weight ratio between the myrcene and PLGA copolymer can be between 1:50 and 1:1. The weight ratio between the myrcene and PLGA copolymer

can be between 1:100 and 1:5, 1:75 and 1:10, 1:50 and 1:10, 1:40 and 1:10, 1:30 and 1:10, 1:20 and 1:10, 1:50 and 1:40, 1:40 and 1:30, 1:30 and 1:20, 1:20 and 1:10, 1:10 and 1:5 and 1:5 and 1:1. The weight ratio between the myrcene and PLGA copolymer can be 1:50, 1:45, 1:40, 1:35, 1:30, 1:25, 1:20, 1:19, 1:17, 1:18, 1:16, 1:15, 1:14, 1:13, 1:12, 1:11 1:10, 1:9, 1:8, 1:7, 1:6, 1:5, 1:4, 1:3, 1:2 or 1:1. In a particular embodiment, the ratio is about 1:22.

In some embodiments, the weight ratio between myrcene and PLGA copolymer is between 1:50 and 1:10. In one embodiment, the weight ratio between myrcene and PLGA copolymer is about 1:14 or about 1:22.

Compounds other than the first terpenoid ("second encapsulated compound") can also be encapsulated in the PLGA nanoparticles. In various embodiments, the second encapsulated compounds are cannabinoids and/or terpenoids other than the first terpenoid. In one embodiment, the second encapsulated compound is cannabigerolic acid (CBGA). In one embodiment, the second encapsulated compound is cannabidiol (CBD). In one embodiment, the second encapsulated compound is cannabinol (CBN). In one embodiment, the second encapsulated compound is cannabidivarin (CBDV). In one embodiment, the second encapsulated compound is cannabichromene (CBC). In one embodiment, the second encapsulated compound is cannabidiolic acid (CBDA). In one embodiment, the second encapsulated compound is cannabigerol (CBG). In one embodiment, the second encapsulated compound is myrcene. In one embodiment, the second encapsulated compound is caryophyllene. In one embodiment, the second encapsulated compound is nerolidol. In one embodiment, the second encapsulated compound is limonene. In one embodiment, the second encapsulated compound is phytol. In one embodiment, the second encapsulated compound is pinene. In one embodiment, the second encapsulated compound is linalool.

*Cannabinoid-encapsulating PLGA nanoparticles*

In another aspect, the present invention provides a cannabinoid-encapsulating PLGA nanoparticle comprising a PLGA nanoparticle and a first cannabinoid encapsulated in the PLGA nanoparticle. The cannabinoid-encapsulating PLGA nanoparticle can encapsulate compounds other than the first cannabinoid, such as other terpenoids and/or cannabinoids.

The first terpenoid encapsulated in the PLGA nanoparticle can be a cannabinoid extractable from *Cannabis sativa.*

The first cannabinoid can be cannabidiol, cannabidivarin, cannabinol, cannabigerol, or cannabichromene.

In various such embodiments, the first cannabinoid, optional cannabinoids, and optional terpenoids other than the first cannabinoid collectively constitute at least 75% by weight, but less than 100 wt%, of the total compounds encapsulated in the PLGA nanoparticles. In specific embodiments, the first cannabinoid, optional cannabinoids, and optional terpenoids other than the first cannabinoid collectively constitute at least 80%, at least at least 85%, at least 90%, at least 91%, at least 92%, at least 93%, at least 94%, or at least 95% by weight, but less than 100 wt%, of the total compounds encapsulated in the PLGA nanoparticles. In particular embodiments, the first cannabinoid, optional cannabinoids, and optional terpenoids other than the first cannabinoid collectively constitute at least 96%, at least 97%, at least 98%, or at least 99% by weight, but less than 100 wt%, of the total compounds encapsulated in the PLGA nanoparticles.

In embodiments in which the first cannabinoid, optional cannabinoids, and optional terpenoids other than the first cannabinoid collectively constitute less than 100% by weight (wt%) of the total compounds encapsulated in the PLGA nanoparticles, the PLGA nanoparticles can further encapsulate compounds other than the first cannabinoid, optional cannabinoids, or optional terpenoids. In such embodiments, the additional compound can be extractable from *Cannabis sativa.* In specific embodiments, all or some compounds encapsulated in the PLGA nanoparticle are present in an extract made from *Cannabis sativa.*

The amount of the first cannabinoid encapsulated in the PLGA nanoparticle can be quantified as a weight ratio between the weight of PLGA copolymer in the PLGA nanoparticle shell and the amount of the first cannabinoid encapsulated within the nanoparticle. The weight ratio between the first cannabinoid and PLGA copolymer can be between 1:50 and 1:1. The weight ratio between the first cannabinoid and PLGA copolymer can be between 1:100 and 1:5, 1:75 and 1:10, 1:50 and 1:10, 1:40 and 1:10, 1:30 and 1:10, 1:20 and 1:10, 1:50 and 1:40, 1:40 and 1:30, 1:30 and 1:20, 1:20 and 1:10, 1:10 and 1:5 and 1:5 and 1:1. The weight ratio between the first cannabinoid and PLGA copolymer can be 1:50, 1:45, 1:40, 1:35, 1:30, 1:25, 1:20, 1:19, 1:17, 1:18, 1:16, 1:15, 1:14, 1:13, 1:12, 1:11 1:10, 1:9, 1:8, 1:7, 1:6, 1:5, 1:4, 1:3, 1:2 or 1:1. In a particular embodiment, the ratio is about 1:14. In a particular embodiment, the ratio is about 1:22.

In some embodiments, the weight ratio between the first cannabinoid and PLGA copolymer is between 1:50 and 1:10. In one embodiment, the weight ratio between the first cannabinoid and PLGA copolymer is about 1:14.

Compounds other than the first cannabinoid ("second encapsulated compound") can also be encapsulated in the PLGA nanoparticles. In various embodiments, the second encapsulated compounds are cannabinoids and/or terpenoids other than the first cannabinoid. In one embodiment, the second encapsulated compound is cannabigerolic acid (CBGA). In one embodiment, the second encapsulated compound is cannabidiol (CBD). In one embodiment, the second encapsulated compound is cannabinol (CBN). In one embodiment, the second encapsulated compound is cannabidivarin

(CBDV). In one embodiment, the second encapsulated compound is cannabichromene (CBC). In one embodiment, the second encapsulated compound is cannabidiolic acid (CBDA). In one embodiment, the second encapsulated compound is cannabigerol (CBG). In one embodiment, the second encapsulated compound is myrcene. In one embodiment, the second encapsulated compound is caryophyllene. In one embodiment, the second encapsulated compound is nerolidol. In one embodiment, the second encapsulated compound is limonene. In one embodiment, the second encapsulated compound is phytol. In one embodiment, the second encapsulated compound is pinene. In one embodiment, the second encapsulated compound is linalool.

*Delta-9 Tetrahydrocannabinol (THC) content*

In typical embodiments, the terpenoid/cannabinoid-encapsulating PLGA nanoparticles are either completely or substantially free of delta-9 tetrahydrocannabinol (THC), and thus lack psychoactive effects, which offer certain regulatory and other physiological advantages.

In certain embodiments, the terpenoid/cannabinoid-encapsulating PLGA nanoparticle is not substantially free of delta-9 THC. In certain of these embodiments, the terpenoid/cannabinoid-encapsulating PLGA nanoparticles comprises 1-10 percent by weight (wt%) THC. In specific embodiments, the terpenoid/cannabinoid-encapsulating PLGA nanoparticle comprises 2 - 9 wt% THC, 3 - 8 wt% THC, 4 - 7 wt% THC. In certain embodiments, the terpenoid/cannabinoid-encapsulating PLGA nanoparticle comprises 1, 2, 3, 4, 5, 6, 7, 8, 9 or 10 wt% THC.

*PLGA nanoparticles*

The nanoparticles comprise the copolymer poly(lactic-co-glycolic acid) (PLGA), which is known to have high biocompatibility, low toxicity, and high control of drug delivery. PLGA copolymer suitable for use in the nanoparticles described herein is available commercially, for example, those available from commercial sources including Sigma-Aldrich under the Resomer brand.

The ratios of the lactic acid and glycolic acid in the PLGA copolymer can be adjusted, resulting in varying amounts of each component in the PLGA polymer and in the PLGA nanoparticle composition. The weight percentage (w/w) of lactic acid in the PLGA polymer can be between about 10%-90%, 10%-20%, 20%-30%, 30%-40%, 40%-50%, 50%-60%, 60%-70%, 70%-80%, or 80%-90%. The weight percentage (w/w) of lactic acid in the PLGA polymer can be about 10%, 15%, 20%, 30%, 35%, 40%, 45%, 50%, 55%, 60%, 65%, 70%, 75%, 80%, 85%, or 90%. The weight percentage (w/w) of glycolic acid in the PLGA polymer can be between about 10%-90%, 10%-20%, 20%-30%, 30%-40%, 40%-50%, 50%-60%, 60%-70%, 70%-80%, or 80%-90%. The weight percentage (w/w) of glycolic acid in the PLGA polymer can be about 10%, 15%, 20%, 30%, 35%, 40%, 45%, 50%, 55%, 60%, 65%, 70%, 75%, 80%, 85%, or 90%. The weight ratio (w/w) of lactic acid to glycolic acid in the polymer can be between about 10% lactic acid and about 90% glycolic acid to about 90% lactic acid and about 10% glycolic acid. The weight ratio (w/w) of lactic acid to glycolic acid in the polymer can be between about 10%, 15%, 20%, 30%, 35%, 40%, 45%, 50%, 55%, 60%, 65%, 70%, 75%, 80%, 85% or 90% lactic acid and about 90%, 85%, 80%, 75%, 70%, 65%, 60%, 55%, 50%, 45%, 40%, 35%, 30%, 25%, 20%, 15%, or 10% glycolic acid

In various embodiments, the weight ratio (w/w) of lactic acid to glycolic acid is between about 10-90% lactic acid and about 90-10% glycolic acid. In some embodiments, the weight ratio (w/w) of lactic acid to glycolic acid is about 10% lactic acid to about 90% glycolic acid. In some embodiments, the weight ratio (w/w) of lactic acid to glycolic acid is about 25% lactic acid to about 75% glycolic acid. In some embodiments, the weight ratio (w/w) of lactic acid to glycolic acid is about 50% lactic acid to about 50% glycolic acid. In some embodiments, the weight ratio (w/w) of lactic acid to glycolic acid is about 75% lactic acid to about 25% glycolic acid. In some embodiments, the weight ratio (w/w) of lactic acid to glycolic acid is about 90% lactic acid to about 10% glycolic acid.

In addition, the PLGA nanoparticles can be surface modified or functionalized with additional polymers, such as polyethylene glycol (PEG) or copolymers such as poloxamers. Various PEG and poloxamer reagents commercially available from a variety of sources including Sigma-Aldrich and Thermo Fisher Scientific are suitable for this use.

The size and diameter of the PLGA nanoparticles can depend on the method used to manufacture the nanoparticles, such as nanoprecipitation or emulsion. In all cases, the size and surface charge of the nanoparticles can be quantified via Dynamic Light Scattering (DLS) and zeta potential (ζ potential) measurements, respectively. A common instrument used to make these measurements is the Malvern Zetasizer Nano ZS; unless otherwise specified, all measurements recited herein are those obtained using this device. These measurements can be used to determine the average diameter of the particles, the surface charge of the particles (ζ potential), and polydispersity of the particles in a population (polydispersity index, PdI).

In various embodiments, the myrcene-encapsulating PLGA nanoparticles have an average diameter of about 150-500 nm, 150-200 nm, 200-250 nm, 200-350 nm, 250-300 nm, 300-350 nm, 350-400 nm, 400-450 nm, or 450-500 nm. In some embodiments, the nanoparticles have an average diameter of about 150 nm, 175 nm, 200 nm, 225 nm,

250 nm, 275 nm, 300 nm, 325 nm, 350 nm, 375 nm, 400 nm, 450 nm, 475 nm, or 500 nm. In some embodiments, the nanoparticles have an average diameter of no more than about 150-500 nm, 150-200 nm, 200-250 nm, 250-300 nm, 300-350 nm, 350-400 nm, 400-450 nm, or 450-500 nm. In some embodiments, the nanoparticles have an average diameter of no more than about 150 nm, 175 nm, 200 nm, 225 nm, 250 nm, 275 nm, 300 nm, 325 nm, 350 nm, 375 nm, 400 nm, 450 nm, 475 nm, or 500 nm.

The diameter of the nanoparticles in a population can also be described using particle size distribution (D values). D values reflect the mass of the nanoparticles in a population as a percentage when the particles are arranged on an ascending mass basis. For instance, the D10 value is the diameter at which 10% of the nanoparticle population mass is comprised of particles less than the indicated diameter value. In such a case, the population of nanoparticles is comprised mainly of particles larger than the indicated diameter value. The D50 value is the diameter at which 50% of the nanoparticle population mass is comprised of particles less than the indicated diameter value and 50% of the nanoparticle population mass is comprised of particles larger than the indicated value. In such a case, the population of nanoparticles is comprised equally of particles larger than the indicated diameter value and smaller than the indicated diameter. The D90 value is the diameter at which 90% of the nanoparticle population mass is comprised of particles less than the indicated diameter value. In this case, the population of nanoparticles is comprised mainly of particles smaller than the indicated diameter value.

In various embodiments, the D10 diameter value of the terpenenoid/cannabinoid-encapsulating PLGA nanoparticles is about 150 nm, 175 nm, 200 nm, 225 nm, 250 nm, 275 nm, 300 nm, 325 nm, 350 nm, 375 nm, 400 nm, 450 nm, 475 nm, or 500 nm. The D50 diameter value of the terpenenoid/cannabinoid-encapsulating PLGA nanoparticles can be about 150 nm, 175 nm, 200 nm, 225 nm, 250 nm, 275 nm, 300 nm, 325 nm, 350 nm, 375 nm, 400 nm, 450 nm, 475 nm, or 500 nm. The D90 diameter value of the terpenenoid/cannabinoid-encapsulating PLGA nanoparticles can be about 150 nm, 175 nm, 200 nm, 225 nm, 250 nm, 275 nm, 300 nm, 325 nm, 350 nm, 375 nm, 400 nm, 450 nm, 475 nm, or 500 nm.

In certain embodiments, the nanoparticles comprise additional polymers known in the art. Such polymers include poly(lactic acid), poly(glycolic acid), gelatins, dextrans, chitosans, lipids, phospholipids, polycyanoacrylates, polyetsers, and poly(ε-caprolactone). The polymers can be chosen based on their degradation characteristics, resulting in formulations of pharmaceutical compositions for prolonged drug delivery. Polymers can be selected for their ability to affect persistence in a subject for an extended period of time, allowing for a timed release of drug during treatment.

## Pharmaceutical compositions

In another aspect, provided herein are pharmaceutical compositions comprising a population of terpenoid/cannabinoid-encapsulating PLGA nanoparticles as described herein, and a pharmaceutically acceptable carrier or diluent. In some embodiments, the pharmaceutical composition is lyophilized. In some embodiments, the pharmaceutical composition is lyophilized in the presence of trehalose.

In some embodiments, the pharmaceutical composition further comprises another population of PLGA nanoparticles (i.e., "the second population of PLGA nanoparticles"), wherein the second population of nanoparticles encapsulates at least one compound (i.e., "the third encapsulated compound"). The compound encapsulated in the second population of nanoparticles can be a cannabinoid or terpenoid. In some embodiments, the cannabinoid or terpenoid in the second population of nanoparticles is different from the cannabinoids and terpenoids encapsulated in the first population of terpenoid/cannabinoid-encapsulating PLGA nanoparticles.

In one embodiment, the third encapsulated compound is cannabigerolic acid (CBGA). In one embodiment, the third encapsulated compound is cannabidiol (CBD). In one embodiment, the third encapsulated compound is cannabinol (CBN). In one embodiment, the third encapsulated compound is cannabidivarin (CBDV). In one embodiment, the third encapsulated compound is cannabichromene (CBC). In one embodiment, the third encapsulated compound is cannabidiolic acid (CBDA). In one embodiment, the third encapsulated compound is cannabigerol (CBG). In one embodiment, the third encapsulated compound is myrcene. In one embodiment, the third encapsulated compound is β-caryophyllene. In one embodiment, the third encapsulated compound is nerolidol. In one embodiment, the third encapsulated compound is limonene. In one embodiment, the third encapsulated compound is phytol. In one embodiment, the third encapsulated compound is pinene. In one embodiment, the third encapsulated compound is linalool. In some embodiments, the second population of nanoparticles encapsulates a compound other than cannabinoids, or terpenoids.

In some embodiments, the pharmaceutical composition further comprises an additional population of PLGA nanoparticles (i.e., "the third population of PLGA nanoparticles"), wherein the third population of nanoparticles encapsulates at least one compound (i.e., "the fourth encapsulated compound"). The compound encapsulated in the third population of nanoparticles can be a cannabinoid or terpenoid. In some embodiments, the cannabinoid or terpenoid in the third population of nanoparticles is different from the cannabinoids and terpenoids encapsulated in the first or the second population of terpenoid/cannabinoid-encapsulating PLGA nanoparticles.

In some embodiments, the pharmaceutical composition further comprises one or more additional population

of PLGA nanoparticles (i.e., "the fourth population of PLGA nanoparticles", "the fifth population of PLGA nanoparticles", etc.), wherein the one or more additional population of nanoparticles encapsulate at least one compound. The compound encapsulated in the one or more additional population of nanoparticles can be a cannabinoid or terpenoid. In some embodiments, the cannabinoid or terpenoid in the one or more additional population of nanoparticles is different from the cannabinoids and terpenoids encapsulated in the other population of terpenoid/cannabinoid-encapsulating PLGA nanoparticles.

In some embodiments, the pharmaceutical composition comprises more than one population of nanoparticles, wherein each population of nanoparticles comprises a unique terpenoid or cannabinoid. In one embodiment, the pharmaceutical composition comprises a first population of nanoparticles encapsulating a terpenoid, and a second population of nanoparticles encapsulating a cannabinoid. In one embodiment, the pharmaceutical composition comprises a first population of nanoparticles encapsulating a first terpenoid and a second population of nanoparticles encapsulating a second terpenoid. The pharmaceutical composition can further comprise a third population of nanoparticles encapsulating a third terpenoid, a fourth population of nanoparticles encapsulating a fourth terpenoid, etc. In another embodiment, the pharmaceutical composition comprises a first population of nanoparticles encapsulating a first cannabinoid and a second population of nanoparticles encapsulating a second cannabinoid. The pharmaceutical composition can further comprise a third population of nanoparticles encapsulating a third cannabinoid, a fourth population of nanoparticles encapsulating a fourth cannabinoid, etc.

In one specific embodiment, the pharmaceutical composition comprises a nanoparticle encapsulating β-myrcene. In one embodiment, the pharmaceutical composition comprises a nanoparticle encapsulating β-caryophyllene. In one embodiment, the pharmaceutical composition comprises a nanoparticle encapsulating nerolidol. In one embodiment, the pharmaceutical composition comprises a first population of nanoparticles encapsulating β-myrcene, a second population of nanoparticles encapsulating β-caryophyllene, and/or a third population of nanoparticles encapsulating nerolidol.

In one embodiment, the pharmaceutical composition comprises a nanoparticle encapsulating cannabidiol. In one embodiment, the pharmaceutical composition comprises a first population of nanoparticles encapsulating cannabidiol, and a second population of nanoparticles encapsulating terpenoid, e.g., β-myrcene, β-caryophyllene, nerolidol, and/or other terpenoid(s). In one embodiment, each of the terpenoids is encapsulated in a separate population of nanoparticles.

## Dose ranges, generally

*In vivo* and/or *in vitro* assays may optionally be employed to help identify optimal dosage ranges for use. The precise dose to be employed in the formulation will also depend on the route of administration, and the seriousness of the condition, and should be decided according to the judgment of the practitioner and each subject's circumstances. Effective doses may be extrapolated from dose-response curves derived from *in vitro* or animal model test systems.

## Unit dosage forms

The pharmaceutical compositions comprising a terpenenoid/cannabinoid-containing PLGA nanoparticle can conveniently be presented in a unit dosage form.

The unit dosage form will typically be adapted to one or more specific routes of administration of the nanoparticle pharmaceutical composition.

In various embodiments, the unit dosage form is adapted for administration by inhalation. In certain of these embodiments, the unit dosage form is adapted for administration by a vaporizer. In certain of these embodiments, the unit dosage form is adapted for administration by a nebulizer. In certain of these embodiments, the unit dosage form is adapted for administration by an aerosolizer.

In various embodiments, the unit dosage form is adapted for oral administration, for buccal administration, or for sublingual administration.

In some embodiments, the unit dosage form is adapted for intravenous, intramuscular, or subcutaneous administration.

In some embodiments, the unit dosage form is adapted for intrathecal or intracerebroventricular administration.

In some embodiments, the unit dosage form is adapted for topical administration, including, for example, transdermal administration.

## Methods for obtaining terpenoid/cannabinoid-encapsulating PLGA nanoparticles

In another aspect, methods for obtaining terpenoid/cannabinoid-encapsulating PLGA nanoparticles are provided. In various embodiments, the method for obtaining terpenoid/cannabinoid-encapsulating PLGA nanoparticles comprises the steps of (a) providing an organic solution comprising a first terpenoid or a first cannabinoid, a PLGA

copolymer, and a solvent, and an aqueous solution comprising a surfactant, (b) emulsifying the two solutions to form a suspension of the terpenoid/cannabinoid-encapsulating PLGA nanoparticles, (c) evaporating the solvent from the emulsion, and (d) obtaining the terpenoid/cannabinoid-encapsulating PLGA nanoparticles.

*Solutions for obtaining teryenoid/cannabinoid-encapsulating PLGA nanoparticles*

Varying ratios of terpenoid/cannabinoid and PLGA copolymer may be used in the solution of step (a). These ratios can be equal amounts of the first terpenoid or cannabinoid and PLGA copolymer (e.g., a 1:1 weight ratio, or a 100% (w/w) the first terpenoid/cannabinoid to PLGA copolymer ratio), lower amounts of the first terpenoid/cannabinoid as compared to PLGA copolymer (e.g., a 1:2, 1:3, or 1:5 weight ratio, or a 50%, 33%, 20% (w/w) the first terpenoid/cannabinoid to PLGA copolymer ratio), or higher amounts of the first terpenoid/cannabinoid as compared to PLGA copolymer (e.g., a 2:1, 3:1, or 5:1 weight ratio, or a 200%, 300%, 500% (w/w) the first terpenoid/cannabinoid to PLGA copolymer ratio). The terpenenoid/cannabinoid to PLGA copolymer weight ratio can be between about 1:1 to 1:20, 1:1 to 1:10, 1:1 to 1:5, 1:5 to 1:10, 1:10 to 1:15, or 1:15 to 1:20. The first terpenoid/cannabinoid to PLGA copolymer weight ratio can be at least about 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20. In various embodiments, the weight ratio of the first terpenoid/cannabinoid and PLGA copolymer in the solution is from about 1:5 to about 1:1. In various embodiments, the weight ratio of the first terpenoid/cannabinoid and PLGA copolymer in the solution is about 1:5. In various embodiments, the weight ratio of the first terpenoid/cannabinoid and PLGA copolymer in the solution is about 1:4. In various embodiments, the weight ratio of the first terpenoid/cannabinoid and PLGA copolymer in the solution is about 1:3. In various embodiments, the weight ratio of the first terpenoid/cannabinoid and PLGA copolymer in the solution is about 1:2. In various embodiments, the weight ratio of the first terpenoid/cannabinoid and PLGA copolymer in the solution is about 1:1.

The solution may include additional terpenoids and/or cannabinoids other than the first terpenoid or cannabinoid. In one embodiment, the solution comprises at least one cannabinoid or terpenoid other than the first terpenoid or cannabinoid, and is substantially free of THC. In some embodiments, the at least one cannabinoid or terpenoid other than the first terpenoid or cannabinoid is selected from the group consisting of: cannabidiol (CBD), cannabinol (CBN), cannabidivarin (CBDV), cannabichromene (CBC), cannabidiolic acid (CBDA), and cannabigerol (CBG). In other embodiments, the at least one cannabinoid or terpenoid other than the first terpenoid or cannabinoid is selected from the group consisting of: myrcene, β-caryophyllene, nerolidol, phytol, limonene, linalool, and pinene.

The solution used in step (a) of the method to manufacture the terpenoid/cannabinoid -encapsulating PLGA nanoparticles also comprises a solvent and a surfactant.

Various solvents are known in the art. Any appropriate solvent may be used to manufacture the terpenoid/cannabinoid-encapsulating PLGA nanoparticles. Exemplary solvents include, but are not limited to, acetone, ethyl acetate, ethanol, methanol, diethyl ether, toluene, hexane, benzene, dichloromethane, tetrahydrofuran, acetonitrile, dimethylformamide, dimethyl sulfoxide, acetic acid, n-butanol, isopropanol, n-propanol, and formic acid, or any combination thereof. In one embodiment, the solvent is acetone, dichloromethane or ethyl acetate. In another embodiment, the solvent is ethyl acetate.

Various surfactants are known in the art and any appropriate surfactant may be used to manufacture the terpenoid/cannabinoid-encapsulating PLGA nanoparticles. Exemplary surfactants include, but are not limited to, anionic surfactants such as ammonium lauryl sulfate, sodium lauryl sulfate or sodium dodecyl sulfate, sodium lauryl ether sulfate, sodium myreth sulfate, sodium stearate and other carboxylate salts; cationic surfactants such as compounds with primary, secondary, tertiary, or quaternary amines such as octenidine dihydrochloride; cetrimonium bromide, benzalkonium chloride, and benzethonium chloride; or non-ionic surfactants such as ethoxylates including fatty acid exothylates, alkylphenol ethoxylates such as nonoxynols, poloxamers, fatty acid ester of glycerol such as glycerol monosterate and glycerol monolaurate, polyethylene glycol (PEG), and polyvinyl alcohol (PVA), or any combination thereof. Surfactants can be hydrophilic or lyophilic. In one embodiment, the surfactant is polyethylene glycol, a poloxamer, or polyvinyl alcohol (PVA). In one embodiment, the surfactant is polyvinyl alcohol (PVA).

*Emulsification*

The terpenoid/cannabinoid-encapsulating nanoparticles are formed in step (b) by mixing two solvent phases, which, depending on method chosen, produces either nanocapsules or nanospheres. Methods such as nanoprecipitation result in the formation of nanospheres, while microemulsion and emulsification with a high-speed homogenizer produce nanocapsules.

In various embodiments, the emulsification method is homogenization or sonication. In certain embodiments, the step of emulsifying is performed using homogenization.

The homogenization can be performed with a high-speed homogenizer, such as a Polytron Homogenizer, available from Thomas Scientific. The homogenization can be performed at about 10,000 to 50,000 rpm. In some

embodiments, the homogenization is performed at between about 10,000 to 50,000 rpm, 10,000 to 15,000 rpm, 15,000 to 20,000 rpm, 20,000 to 25,000 rpm, 25,000 to 30,000 rpm, 30,000 to 35,000 rpm, 35,000 to 40,000 rpm, 40,000 to 45,000 rpm, or 45,000 to 50,000 rpm. In some embodiments, the homogenization is performed at about 10,000 rpm, 15,000 rpm, 20,000 rpm, 21,000 rpm, 22,000 rpm, 23,000 rpm, 24,000 rpm, 25,000 rpm, 26,000 rpm, 27,000 rpm, 28,000 rpm, 29,000 rpm, 30,000 rpm, 35,000 rpm, 40,000 rpm, 45,000 rpm, or 50,000 rpm. In one embodiment, the homogenization is performed at 20,000 to 30,000 rpm. In another embodiment, the homogenization is performed at 24,000 rpm.

In some embodiments, the solution is homogenized for at least about 10 seconds to 10 minutes. In some embodiments, the solution is homogenized for at least about 10s, 15s, 20s, 30s, 40s, 45s, 50s, 55s, 1 min, 1.5 min, 2 min, 2.5 min, 3 min, 3.5 min, 4 min, 4.5 min, 5 min, 5.6 min, 6 min, 6.5 min, 7 min, 7.5 min, 8 min, 8.5 min, 9 min, 9.5 min, or 10 min. In one embodiment, the solution is homogenized for about 30s to 10 min. In one embodiment, the solution is homogenized for 1 min.

*Solvent Evaporation*

After the emulsification step, the solution comprises a suspension of terpenoid/cannabinoid-encapsulating PLGA nanoparticles. In step (c), the solvent in the nanoparticle suspension is then removed via evaporation. Evaporation techniques include, but are not limited to, stirring the suspension and solvent, applying gas streams, applying heat, maintaining the temperature at 10°C, and creating a vacuum. A rotary evaporator can be used to evaporate the solvent in the nanoparticle suspension at room temperature or at cooler temperatures. In such a case, the sample may be place on ice during evaporation, in a refrigerator or cold room, or the rotary evaporator may have an attached chiller unit. The solvent can also be evaporated by stirring the nanoparticle suspension. In some embodiments, evaporating the solvent comprises stirring the suspension at room temperature. The suspension can be stirred for about 5 min to 120 min. In some embodiments, the suspension is stirred for between about 5-10 min, 10-15 min, 15-30 min, 30-45 min, 45-60 min, 60-75 min, 75-90 min, 90-105 min, or 105-120 min. In some embodiments, the suspension is stirred for about 5 min, 10 min, 15 min, 20 min, 25 min, 30 min, 35 min, 40 min, 45 min, 50 min, 55 min, 60 min, 65 min, 70 min, 75 min, 80 min, 85 min, 90 min, 95 min, 100 min, 105 min, 110 min, 115 min, or 120 min. In one embodiment, the suspension is stirred for 5 min to 120 min to evaporate the solvent. In one embodiment, the suspension is stirred for 60 min.

*Centrifugation*

In some embodiments, evaporation of solvent in step (c) is sufficient to allow the PLGA nanoparticles to be obtained. In a particular embodiment, the centrifugation is performed at 10°C.

In some embodiments, after evaporating the solvent, the terpenoid/cannabinoid-encapsulating PLGA nanoparticles are obtained by further centrifugation, filtration, or centrifugation and filtration. In a particular embodiment, obtaining the nanoparticles comprises centrifugation. The nanoparticle suspension can be centrifuged at between 2,000 *x g* and 15,000 *x g*. In some embodiments, the nanoparticle suspension is centrifuged at between 2,000 and 4,000 *x g*, 2,000 and 10,000 *x g*, 2,000 and 15,000 *x g*, 4,000 and 10,000 *x g*, 4,000 and 15,000 *x g*, 7,000 and 10,000 *x g*, 7,000 and 15,000 *x g*, or 10,000 and 15,000 *x g*. The nanoparticle suspension can be centrifuged at between 2,000 rpm and 15,000 rpm. In some embodiments, the nanoparticle suspension is centrifuged at between 2,000 and 4,000 rpm, 2,000 and 10,000 rpm, 2,000 and 15,000 rpm, 4,000 and 10,000 rpm, 4,000 and 15,000 rpm, 7,000 and 10,000 rpm, 7,000 and 15,000 rpm, or 10,000 and 15,000 rpm. In one embodiment, the centrifugation is at 4,000 *x g*.

The suspension can be centrifuged for between about 5 min to 60 min. The suspension can be centrifuged for between about 5 and 10 min, 10 and 15 min, 15 and 20 min, 20 and 25 min, 25 and 30 min, 30 and 35 min, 35 and 40 min, 40 and 45 min, 45 and 50 min, 50 and 55 min, or 55 and 60 min. The suspension can be centrifuged for about 5 min, 10 min, 15 min, 20 min, 25 min, 30 min, 35 min, 40 min, 45 min, 50 min, 55 min, or 60 min. In some embodiments, the centrifugation is performed for 10 min, 15 min, 30 min, 45 min or 10-45 min. In one embodiment, the centrifugation is performed for 30 min. In some embodiments, the suspension can be centrifuged for longer. For example, the suspension can be centrifuged about 30 min to 1 hour, 30 min to 2 hours, or 3 min to 3 hours.

*Lyophilization*

After the terpenoid/cannabinoid-encapsulated nanoparticles are obtained, the nanoparticles can optionally be lyophilized. In this case, a cryoprotectant or cryopreservative can be added to the terpenoid/cannabinoid-encapsulating PLGA nanoparticles. Any appropriate cryoprotectant known in the art may be used, including but not limited to, trehalose, ethylene glycol, propylene glycol, glycerol, and dimethyl sulfoxide, and any combination thereof. In one embodiment, the cryoprotectant is trehalose. The cryoprotectant can be added at a concentration of between about 1-25% (w/v) of the terpenoid/cannabinoid-encapsulating PLGA nanoparticles. The cryoprotectant can be added at concentrations between about 1-2% (w/v), 2-5% (w/v), 5-10% (w/v), 10-15% (w/v), 15-20% (w/v), or 20-25 % (w/v) to the nanoparticles.

The cryoprotectant can be added at a concentration of at least about 1% (w/v), 2% (w/v), 3% (w/v), 4% (w/v), 5% (w/v), 6% (w/v), 7% (w/v), 8% (w/v), 9% (w/v), 10% (w/v), 12% (w/v), 15% (w/v), 17% (w/v), 20% (w/v), 22% (w/v), or 25% (w/v) to the nanoparticles. In some embodiments, the cryoprotectant is added in a concentration of 1-10% (w/v) of the terpenoid/cannabinoid-encapsulating PLGA nanoparticles. In one embodiment, the cryoprotectant is added at concentration of 5% (w/v) of the terpenoid/cannabinoid-encapsulating PLGA nanoparticles.

The nanoparticles can be lyophilized for between about 10 to 240 min, 10 to 30 min, 30 to 45 min, 45 to 60 min, 60 to 75 min, 75 to 90 min, 90 to 105 min, 105 to 120 min, 120 to 135 min, 135 to 150 min, 150 to 165 min, 165 to 180 min, 180 to 195 min, 195 to 210 min, 210 to 225 min, 225 to 240 min, or more than 240 min such as overnight. The nanoparticles can be lyophilized for about 10 min, 15 min, 30 min, 45 min, 60 min, 75 min, 90 min, 105 min, 120 min, 135 min, 150 min, 165 min, 180 min, 195 min, 210 min, 225 min, 240 min, or overnight. In some embodiments, the terpenoid/cannabinoid-encapsulating PLGA nanoparticles are lyophilized for 30 min, 60 min, 90 min, 120 min, 150 min, 180 min, or 30-180 min. In one embodiment, the terpenoid/cannabinoid-encapsulating PLGA nanoparticles are lyophilized for 120 min.

In some embodiments, the nanoparticles are lyophilized for longer. For example, the nanoparticles are lyophilized for about 3 hours, 4 hours, 5 hours, 6 hours, 7 hours, 8 hours, 9 hours, 10 hours, 11 hours, 12 hours, or longer (e.g., 24-72 hours) depending on the volume of water used.

*Drug loading and encapsulation efficiencies*

The amount of terpenoid/cannabinoid or other compounds encapsulated in the nanoparticles can be quantified. Drug compound content can be expressed as entrapment efficiency (EE, %) and drug loading (DL, %), as defined in Section 4.1 above.

In some embodiments, the entrapment efficiency (EE, %) of the method is between about 1% and 100%. In some embodiments, the entrapment efficiency is between about 1-5%, 5-10%, 4-7%, 4-10%, 10-15%, 15-20%, 20-25%, 25-30%, 30-35%, 35-40%, 40-45%, 45-50%, 50-55%, 60-65%, 65-70%, 70-75%, 75-80%, 80-85%, 85-90%, or 95-100%. In some embodiments, the entrapment efficiency is at least about 1%, 2%, 3%, 4%, 5%, 6%, 7%, 8%, 9%, 10%, 12%, 15%, 17%, 20%, 25%, 30%, 35%, 40%, 45%, 50%, 55%, 60%, 65%, 70%, 75%, 80%, 85%, 90%, 95%, 99%, or 100%. In some embodiments, the entrapment efficiency is about 1%, 2%, 3%, 4%, 5%, 6%, 7%, 8%, 9%, 10%, 12%, 15%, 17%, 20%, 25%, 30%, 35%, 40%, 45%, 50%, 55%, 60%, 65%, 70%, 75%, 80%, 85%, 90%, 95%, 99%, or 100%. In one embodiment, the entrapment efficiency is at least 4%. In one embodiment, the entrapment efficiency is at least 5%. In one embodiment, the entrapment efficiency is at least 6%. In one embodiment, the entrapment efficiency is at least 7%. In one embodiment, the entrapment efficiency is at least 8%. In one embodiment, the entrapment efficiency is at least 9%. In one embodiment, the entrapment efficiency is at least 10%.

In some embodiments, the drug loading (DL %) of the method is between about 1% and 40%. In some embodiments, the drug loading is between about 1-5%, 5-10%, 4-7%, 4-10%, 10-15%, 15-20%, 20-25%, or 25-30%. In some embodiments, the drug loading is at least about 1%, 2%, 3%, 4%, 5%, 6%, 7%, 8%, 9%, 10%, 12%, 15%, 17%, 20%, 25%, or 30%. In some embodiments, the drug loading is about 1%, 2%, 3%, 4%, 5%, 6%, 7%, 8%, 9%, 10%, 12%, 15%, 17%, 20%, 25%, or 30%.

In some embodiments, the drug loading is expressed as the average weight ratio of the encapsulated terpenoid/cannabinoid and the PLGA copolymer in the nanoparticles. In some embodiments, the average weight ratio between encapsulated terpenoid/cannabinoid and PLGA copolymer in the terpenoid/cannabinoid -encapsulating PLGA nanoparticles) is between about 1:50 and about 1:10. In some embodiments, the average weight ratio is between about 1:50 and 1:40, 1:40 and 1:30, 1:30 and 1:20, and 1:20 and 1:10. In some embodiments, the average weight ratio is at least about 1:50, 1:45, 1:40, 1:35, 1:30, 1:25, 1:20, 1:15, 1:14, 1:13, 1:12, 1:11, 1:10. In some embodiments, the average weight ratio is at least about 1:50. In some embodiments, the average weight ratio is at least about 1:40. In some embodiments, the average weight ratio is at least about 1:30. In some embodiments, the average weight ratio is at least about 1:20. In some embodiments, the average weight ratio is at least about 1:10.

**Methods of treatment using terpenoid/cannabinoid-encapsulating PLGA nanoparticles**

In yet another aspect, methods of treating various symptoms or diseases in mammalian subjects are provided. The methods comprise administering the terpenoid/cannabinoid-containing PLGA nanoparticle described herein. These methods are particularly aimed at therapeutic and prophylactic treatments of mammals, and more particularly, humans.

The actual amount administered, and rate and schedule of administration, will depend on the nature and severity of symptoms or disease being treated. Prescription of treatment, *e.g.* decisions on dosage *etc.,* is within the skill of general practitioners and other medical professionals, and typically takes account of the disorder to be treated, the condition of the individual patient, the route of administration, the site to be treated, and other factors known to practitioners.

*In vivo* and/or *in vitro* assays may optionally be employed to help identify optimal dosage ranges for use and

routes and times for administration. The precise dose to be employed in the formulation will also depend on the route of administration, and the seriousness of the condition, and should be decided according to the judgment of the practitioner and each subject's circumstances. Effective doses and methods of administration may be extrapolated from dose-response curves derived from *in vitro* or animal model test systems.

In some embodiments, the encapsulated terpenoid or cannabinoid is administered in an amount less than 1g, less than 500 mg, less than 100 mg, less than 10 mg per dose.

In the methods of treatment described herein, the pharmaceutical composition comprising terpenoid/cannabinoid-containing PLGA nanoparticle can be administered alone or in combination with other treatments administered either simultaneously or sequentially with the terpenoid/cannabinoid-containing composition.

## Methods of effecting TRPV1 desensitization in cells of a mammalian subject

Methods are presented for effecting TRPV1 desensitization in cells of a mammalian subject, the method comprising administering to the subject the terpenoid/cannabinoid-containing pharmaceutical compositions described herein in an amount, by a route of administration, and for a time sufficient to cause TRPV1 desensitization in cells within the subject. In some embodiments, the cells to be subjected to TRPV1 desensitization are nociceptors, such as peripheral nociceptors, and visceral nociceptors.

In various embodiments, the pharmaceutical composition is administered systemically. In some embodiments, the pharmaceutical composition is administered orally, by buccal administration, or sublingually.

In various embodiments, the pharmaceutical composition is administered topically. In particular embodiments, the pharmaceutical composition is administered topically to effect transdermal delivery.

In some embodiments, the pharmaceutical composition is administered parenterally. In certain embodiments, the pharmaceutical composition is administered intravenously. In some embodiments, the pharmaceutical composition is administered subcutaneously. In some embodiments, the pharmaceutical composition is administered by inhalation.

## Methods of treating pain

In some embodiments, the receptors to be subjected to TRPV1 desensitization are nociceptive, and the method comprises administering to the subject the terpenoid/cannabinoid-containing pharmaceutical compositions described herein in an amount, by a route of administration, and for a time sufficient to cause TRPV1 desensitization in nociceptors within the subject.

In some embodiments, the nociceptors are peripheral nociceptors. In certain of these embodiments, the pharmaceutical composition is administered topically. In some embodiments, the pain-sensing neurons are visceral. In certain of these embodiments, the pharmaceutical composition is administered systemically.

In a related aspect, methods are provided for treating pain in a mammalian subject. The method comprises administering to the subject the pharmaceutical compositions described herein in an amount, by a route of administration, and for a time sufficient to reduce pain.

In certain embodiments, the pain is neuropathic pain. In particular embodiments, the neuropathic pain is diabetic peripheral neuropathic pain. In particular embodiments, the pain is post-herpetic neuralgia. In particular embodiments, the pain is trigeminal neuralgia.

In some embodiments, the subject has pain related to or caused by strains, sprains, arthritis or other joint pain, bruising, backaches, fibromyalgia, endometriosis, surgery, migraine, cluster headaches, psoriasis, irritable bowel syndrome, chronic interstitial cystitis, vulvodynia, trauma, musculoskeletal disorders, shingles, sickle cell disease, heart disease, cancer, stroke, or mouth sores or ulceration due to chemotherapy or radiation.

In some embodiments, the pharmaceutical composition is administered at least once a day for at least 3 days. In some embodiments, the pharmaceutical composition is administered at least once a day for at least 5 days. In some embodiments, the pharmaceutical composition is administered at least once a day for at least 7 days. In some embodiments, the pharmaceutical composition is administered at least once a day for more than 7 days.

In various embodiments, the pharmaceutical composition is administered at a dose, by a route of administration, and on a schedule sufficient to maintain effective levels of the terpenoid or cannabinoid at the nociceptors for at least 3 days, at least 5 days, or at least 7 days.

## Methods of treating cardiac hypertrophy

In another aspect, methods of treating cardiac hypertrophy in a mammalian subject are provided. The methods comprise administering to the subject an anti-hypertrophic effective amount of the pharmaceutical compositions described herein.

In typical embodiments, the pharmaceutical composition is administered systemically. In some embodiments,

the pharmaceutical composition is administered orally. In some embodiments, the pharmaceutical composition is administered intravenously. In some embodiments, the pharmaceutical composition is administered subcutaneously. In some embodiments, the pharmaceutical composition is administered by inhalation.

## Methods of prophylactic treatment for cardiac hypertrophy

In another aspect, methods of prophylactic treatment for cardiac hypertrophy in a mammalian subject are provided. The methods comprise administering to a subject at risk of cardiac hypertrophy an anti-hypertrophic effective amount of the terpenoid/cannabinoid-containing pharmaceutical compositions described herein.

## Methods of treating overactive bladder

In another aspect, methods of treating overactive bladder in a mammalian subject, are provided. The methods comprise administering to the subject a therapeutically effective amount of the terpenoid/cannabinoid-containing pharmaceutical compositions described herein.
In typical embodiments, the pharmaceutical composition is administered systemically. In some embodiments, the pharmaceutical composition is administered by bladder irrigation.

## Methods of treating refractory chronic cough

In another aspect, methods of treating refractory chronic cough are provided, the methods comprising administering to the subject a therapeutically effective amount of the terpenoid/cannabinoid-containing pharmaceutical composition described herein.
In some embodiments, the pharmaceutical composition is administered systemically.
In some embodiments, the pharmaceutical composition is administered by inhalation.

## Methods of treating disorders with TRPV1 etiology

In another aspect, diseases or disorders that are treated with the terpenoid/cannabinoid-containing pharmaceutical compositions described herein include diseases related to abnormal function of TRPV1. The diseases can be related to abnormal activation, suppression, or dysregulation of TRPV1. In some embodiments, the diseases are related to abnormal expression or mutation of the gene encoding TRPV1.
In some embodiments, diseases treated with the terpenoid/cannabinoid-containing pharmaceutical compositions described herein are diseases related to abnormal synthesis of an endogenous TRPV1 agonist.

# EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.
The practice of the present invention will employ, unless otherwise indicated, conventional methods of protein chemistry, biochemistry, recombinant DNA techniques and pharmacology, within the skill of the art. Such techniques are explained fully in the literature.

## Abbreviations

| | |
|---|---|
| DCM | Dichloromethane |
| DL | Drug loading |
| EE | Entrapment efficiency |
| NPs | Nanoparticles |
| PEG | Polyethyleneglycol |

| | |
|---|---|
| PLGA | Copolymer of lactic and glycolic acid |
| PVA | Poly vinyl alcohol |
| ζ | Zeta potential |

**Methodology**

*Methods of characterizations of terpenoid-containing nanoparticles*

The mean diameter and size distributions of the synthesized and purified terpenoid-containing nanoparticles were measured, in triplicates, at 25.0 ± 0.5 °C by Dynamic Light Scattering (Nanosizer ZS, Malvern Instruments Ltd., UK

The zeta potential of the PLGA-PEG-based NPs (terpenoid-containing and terpenoid-free) was characterized by laser Doppler electrophoresis (Nanosizer ZS, Malvern Instruments Ltd., UK), and the ζ measurements, performed in triplicates, were done on NPs after final wash with MQ water at 25 °C.

The morphological characterization of the nanoparticles was performed by image analyses by scanning electron microscopy in a FEI TENEO microscope. The samples were prepared by diluting the terpenoid containing nanoparticle suspensions (free of trehalose) down to around 0.8 mg/ml and covered with a 8-9 nm Pd/Pt shell under vacuum (Leica EM SCD500).

The amounts of myrcene, β-caryophyllene, or nerolidol encapsulated in nanoparticles were measured by a GC-MS spectrometry method. Analyses were performed using a Trace 1300 GC gas chromatography system, fitted with a ZB-1MS capillary column (30 m × 0.25 mm × 0.25 μm, Thermo Scientific), in tandem with a mass spectrometer TSQ8000 (Thermo Scientific), and data acquisition and analyses were performed using Xcalibur.

For mass spectrometry detection, ionization was carried out by electronic impact (EI) with a voltage of 70 eV and full scan mode in the m/z range of 20-300 with an ion source temperature of 200 °C in positive ion mode. For quantification of myrcene, caryophyllene and nerolidol, characteristic ions for terpenoids at m/z 69, 93 and 133 respectively were monitored by selected ion monitoring (SIM) mode. For the purpose of generating a calibration curve, myrcene, caryophyllene, or nerolidol with a series of concentrations ranging between 1-60 ppm (1, 2, 5, 10, 20, 40 and 60 ppm) in dichloromethane (DCM) were prepared and measured in the GC-MS instrument.

The drug encapsulation capabilities of the PLGA-PEG nanoparticles can be expressed as the entrapment efficiency (EE, %) and the drug loading (DL, %) according to **Equation 1** and **2** respectively.

$$EE\ (\%) = \frac{mass\ of\ CBDs\ incorporated\ (mg)}{initial\ mass\ of\ CBDs\ (mg)} \times 100 \qquad \text{(Equation 1)}$$

$$DL\ (\%) = \frac{mass\ of\ CBDs\ incorporated\ (mg)}{mass\ of\ nanocarrier\ (mg)} \times 100 \qquad \text{(Equation 2)}$$

*Cells*

HEK TRPV1 cells were provided by the Helen Turner lab at Chaminade University of Honolulu and maintained in MEM essential medium supplemented with 10% FBS, 50 units/ml-50μg/ml Penicillin-Streptomycin, and 0.6 mg/ml geneticin.

*Calcium signaling assay procedure*

HEK TRPV1 cells were harvested with trypsin, deactivated with media, and counted. Cells were centrifuged at 1000rpm, for 5min, at room temperature. Cells were washed twice with 1mM Ca Assay Buffer (Na Ringers [140mM NaCl, 2.8mM KCl, 2mM $MgCl_2$, 11mM glucose, 10mM HEPES], 2mM Probenecid, 1mM $CaCl_2$; pH 7.4), and collected by centrifugation at 1000rpm, for 5min, at room temperature. Cells were resuspended in 1μM Fluo-4 (1 μl of 5mM Fluo-4, mixed with 1 μl of 20% Pluronic F-127 in DMSO, then 5 ml of 1mM Ca Assay Buffer was added and the mixture incubated at 37 °C for 10 min in the dark), and incubated for 30 min at 37 °C in the dark. Cells were then washed twice with 1mM Ca assay buffer, then resuspended in the assay buffer and pipetted to opaque-walled 96-well plates at a density of 150,000 cells/180μl/well. Fluorescence was measured using a plate reader (Synergy HTX, BioTek, USA), at

an excitation wavelength of 485 nm and an emission wavelength of 528 nm. Once a baseline was established (3 measurements), 20 μl of the stimulant (free terpenoids, terpenoids-loaded NPs, and controls) solution was added to each well and measurement was continued for 1 h with one read every 40 seconds.

*Preparation of stimulant solutions*

All solutions were prepared in 1mM Ca Assay Buffer. Free terpenoids were first dissolved in DMSO, then a specific volume was diluted with the assay buffer to produce the required concentration. Terpenoids loaded PLGA-PEG or poly(ethylene glycol) methyl ether-block-poly(lactide-co-glycolide) nanoparticles (NPs) were prepared by the emulsification method and were diluted directly using the assay buffer. Negative controls of assay buffer containing DMSO and blank NPs were also prepared. Ionomycin 4 μM was used as a positive control. Free and encapsulated terpenoids were prepared at a concentration of 400 μg /ml (to give a final concentration of 40 μg/ml after addition to the cells) either alone or in combinations, such as myrcene plus nerolidol, myrcene plus caryophyllene, nerolidol plus caryophyllene, and myrcene plus nerolidol plus β-caryophyllene).

Experiments were performed in triplicate, responses of negative controls were deducted, and averages were computed and used to plot the calcium responses profiles.

**Example 1 - Nanoprecipitation method for producing myrcene-encapsulated nanoparticles**

A nanoprecipitation method based on a solvent displacement (**FIG. 1**) was tested for generation of myrcene-containing PLGA nanoparticles. The particle formation by the nanoprecipitation method is ruled by the so-called Marangoni effect, which is subjected to interfacial turbulences that occur at the interface of the solvent and the non-solvent and results from complicated and cumulated phenomena like flow, diffusion, and surface tension variations. The presence of a stabilizer is very important to avoid aggregate formation and to impart stability to nanoparticles during the nanoprecipitation technique.

**Formulation**

PLGA based nanoparticles (NPs) were prepared using a nanoprecipitation method according to the formulas (F1-F11) in **Table 1.** A mixture of hydrophilic and lyophilic surfactants (Pluronic and Span) were used to ensure the system stability. Acetone was evaporated using different experimental conditions - at room temperature, or using rotary evaporator in the absence or in the presence of tocopheryl polyethylene glycol succinate (TPGS).

**Table 1.** Formulas of NPs prepared using the nanoprecipitation method.

| **Formula** | | **F1** | **F2** | **F3** | **F4** | **F5** | **F6** | **F7** | **F8** | **F9** | **F10** | **F11** |
|---|---|---|---|---|---|---|---|---|---|---|---|---|
| **Organic phase** | Myrcene* | - | 10%<br>2.25 mg | 20%<br>4.5 mg | 30%<br>6.75 mg | 40%<br>9 mg | 60%<br>13.5 mg | 40%<br>9 mg | 60%<br>13.5 mg | 40%<br>9 mg | 40%<br>9 mg | 40%<br>9 mg |
| | PLGA (Resomer 502) | 22.5 mg | 22.5 mg | 22.5 mg | 22.5 mg | 22.5 mg | 22.5 mg | 22.5 mg | 22.5 mg | 22.5 mg | 22.5 mg | 22.5 mg |
| | Span® 60 | 5 mg | 5 mg | 5 mg | 5 mg | 5 mg | 5 mg | 5 mg | 5 mg | 5 mg | 2.5 mg | - |
| | TPGS | - | - | - | - | - | - | - | - | 2.5 | 5 | 7.5 |
| | Acetone | 1.5 mL | 1.5 mL | 1.5 mL | 1.5 mL | 1.5 mL | 1.5 mL | 1.5 mL | 1.5 mL | 1.5 mL | 1.5 mL | 1.5 mL |
| **Aqueous phase** | Pluronic® F-68 0.5% w/v | 4.5 mL | 4.5 mL | 4.5 mL | 4.5 mL | 4.5 mL | 4.5 mL | 4.5 mL | 4.5 mL | 4.5 mL | 4.5 mL | 4.5 mL |
| **Acetone evaporation** | | r.t. | | | | | | rotary evaporator | | | | |

* % w/w based on PLGA weight

## Preparation method using evaporation of organic solvent at room temperature (F1-F6)

PLGA (22.5 mg) and Span® 60 (7.5 mg) were dissolved in 1.5 mL acetone. Myrcene (10 - 100% w/w) was added to the organic solution. To 4.5 mL Pluronic® F-68 aqueous solution (0.5% w/v), the organic phase was added drop-wise using a syringe-pump at a rate of 5 mL/h with continuous stirring. Stirring was continued for 2 h at room temperature to allow acetone to evaporate. To remove non-encapsulated compounds as well as the excess surfactant, the suspension was adjusted up to 45 mL with MQ water and centrifuged at 10,000 rpm at 4°C for 30 min. The pellet was re-suspended in 45 mL MQ water and centrifuged again using the same conditions. The NP pellet was resuspended in MQ water to make 1 mL of NP suspension. This suspension was then dried in the oven to measure the loaded myrcene.

To measure the weight and yield of produced NPs, NPs were prepared using the same method. After final centrifugation the NP pellet was resuspended in 2 mL MQ water and the preparation was freeze-dried. The lyophilized NPs were collected as white cotton-like material. The supernatant was also collected and lyophilized to measure the non-encapsulated myrcene.

Samples were prepared for GC-MS analysis. One hundred μL of freshly prepared NPs was completely dried in the oven (removal of water) for 30 - 40 min at 40°C. DCM (2 mL) was added to dissolve the dried residue. Next, 1.5 mL of the DCM solution was transferred into suitable vials and sealed. The lyophilized supernatants stored at -20°C were weighed and samples were prepared for GC-MS analysis as described for the NPs. Briefly, 5 mg of NP samples were dissolved in DCM and 1.5 mL of the solution was transferred into a suitable vial and sealed.

## Preparation method using evaporation of organic solvent using rotary evaporator in the absence of TPGS (F7-8)

PLGA (22.5 mg) and Span® 60 (7.5 mg) were dissolved in 1.5 mL acetone. Myrcene (40 or 60% w/w) was added to the organic solution. To 4.5 mL Pluronic® F-68 aqueous solution (0.5% w/v), the organic phase was added drop-wise using a syringe pump at a rate of 5 mL/h. During mixing, the mixture was kept in a cool water bath to reduce evaporation of the drug. Acetone was removed using a rotary evaporator without heating over a period of 20 min. To remove non-encapsulated compounds as well as the excess surfactant, the suspension was adjusted up to 45 ml with MQ water and centrifuged at 10,000 rpm at 4°C for 30 min. The pellet was re-suspended in 45 mL MQ water and centrifuged again using the same conditions. The NP pellet was resuspended in 2 mL MQ water, and the preparation was freeze-dried. The lyophilized NPs were collected as white cotton-like material. The supernatant was also collected and lyophilized to measure the non-encapsulated myrcene.

Samples were prepared for GC-MS analysis. Lyophilized NPs were dissolved in DCM. To facilitate dissolution, samples were sonicated in the bath sonicator for 5 min. Samples were filtered using 1 μm syringe filter (compatible with DCM). 1.5 mL of sample solutions were transferred into suitable vials and sealed. The lyophilized supernatants stored at -20°C were weighed and samples were prepared for GC-MS analysis as described for the NPs. Briefly, 5 mg of NP samples were dissolved in DCM and 1.5 mL of the solution was transferred into a suitable vial and sealed.

## Preparation method using evaporation of organic solvent using rotary evaporator in the presence of TPGS (F9-11)

Tocopheryl polyethylene glycol succinate (TPGS), vitamin E TPGS, is a watersoluble derivative of natural vitamin E. It was selected as an emulsifier in the formulations due to its several advantages, including its bulky structure and large surface area characteristics, which make it an excellent emulsifier. PLGA based NPs were prepared using a nanoprecipitation method in the presence of tocopheryl polyethylene glycol succinate (TPGS) according to the formulas, F9-F11 (Table 1).

PLGA (22.5 mg), Span® 60 (5, 2.5 or 0 mg) and TPGS (2.5, 5 or 7.5 mg) were dissolved in 1.5 mL acetone. Myrcene (9 mg; 40% w/w) was added to the organic solution. To 4.5 mL Pluronic® F-68 aqueous solution (0.5% w/v), the organic phase was added drop-wise using a syringe pump at a rate of 5 mL/h. During mixing, the mixture was kept in a cool water bath to reduce evaporation of the drug. Acetone was removed using a rotary evaporator without heating over a period of 20 min. To remove non-encapsulated compounds as well as the excess surfactant, the suspension was adjusted up to 45 mL with MQ water and centrifuged at 10,000 rpm at 4°C for 30 min. The pellet was re-suspended in 45 mL MQ water and spinning was repeated using the same conditions. The NPs pellet was resuspended in 2 mL MQ water, and the preparation was freeze-dried. The lyophilized NPs were collected as white cotton-like material. The supernatant was also collected and lyophilized to measure the non-encapsulated myrcene.

Samples were prepared for GC-MS analysis. NPs lyophilizates were dissolved in DCM. To facilitate dissolution, samples were sonicated in the bath sonicator for 5 min. Samples were filtered using 1 μm syringe filter (compatible with DCM). 1.5 mL of sample solutions were transferred into suitable vials and sealed. The lyophilized supernatants stored at -20°C were weighed and samples were prepared for GC-MS analysis as described for the NPs. Briefly, 5 mg of NP samples were dissolved in DCM and 1.5 mL of the solution was transferred into a suitable vial and sealed.

## Characterization of Nanoparticles Prepared by Nanoprecipitation

Table 2 presents the mean diameter, size distribution, zeta potential, weight, yield, amounts of loaded myrcene, EE% and DL% of NPs prepared by nanoprecipitation.

**Table 2.** Characteristics of NPs prepared by nanoprecipitation.

| Acetone evaporation | Formula: Myrcene* | Diameter (nm) | PdI | Zeta Potential (mV) | NPs weight (mg) | Yield (%) | Loaded myrcene (mg) | EE % | DL % |
|---|---|---|---|---|---|---|---|---|---|
| r.t. | F1: Blank | 195.4 | 0.165 | -29.8 | 16.49 | 73.3 | - | - | - |
| | F2: 10%, 2.25 mg | 202.3 | 0.184 | -28.9 | 16.30 | 65.86 | 0.031 | 1.38 | 0.18 |
| | F3: 20%, 4.5 mg | 207.4 | 0.181 | -30.3 | 16.70 | 61.85 | 0.033 | 0.74 | 0.20 |
| | F4: 30%, 6.75 mg | 218.7 | 0.213 | -30.1 | 16.23 | 55.49 | 0.033 | 0.48 | 0.20 |
| | F5: 40%, 9 mg | 222.1 | 0.195 | -33.5 | 16.75 | 53.71 | 0.036 | 0.40 | 0.22 |
| | F6: 60%, 13.5 mg | 231.4 | 0.208 | -32.2 | 17.12 | 47.56 | 0.042 | 0.31 | 0.25 |
| rotary evaporator | F7: 40%, 9 mg | 224.3 | 0.124 | -30.4 | 17.67 | 56.10 | 0.155 | 1.72 | 0.88 |
| | F8: 60%, 13.5 mg | 237.3 | 0.159 | -31.1 | 17.86 | 54.61 | 0.275 | 2.04 | 1.34 |
| rotary evaporator | F9: 40%, 9 mg | 198.1 | 0.143 | -26.1 | 15.42 | 68.53 | 0.14 | 1.56 | 0.91 |
| | F10: 40%, 9 mg | 231.7 | 0.156 | -23.4 | 16.63 | 73.91 | 0.099 | 1.1 | 0.60 |
| | F11: 40%, 9 mg | aggregated | - | - | - | - | - | - | - |

* % w/w based on PLGA

The particle size of the NPs produced by nanoprecipitation ranged between 200 - 240 nm with good polydispersity (PdI) of 0.2 or less and a negative zeta potential around -30 **(Table 2). FIG. 8A** and **8B** show the size distribution by intensity and the zeta potential distribution, respectively, for sample F6. **FIG. 8C** and **8D** show the size distribution by intensity and the zeta potential distribution, respectively, for sample F8. **FIG. 8E** and **8F** show the size distribution by intensity and the zeta potential distribution, respectively, for sample F9.

Stable NPs were successfully produced with concentrations of myrcene up to 60%. At myrcene concentrations above 60% (w/w), NPs were not physically stable and aggregations formed during the initial mixing step.

The evaporation of acetone using the rotary evaporator, without heating, has significantly increased the amount of loaded myrcene by 5 times or more, from 0.036 mg and 0.042 mg in F5 and F6, respectively, to 0.155 mg and 0.275 mg in F7 and F8, respectively **(Table 2).**

TPGS is a synthetic amphiphile of vitamin E, and is FDA approved as a water-soluble vitamin E nutritional supplement and drug delivery vehicle. It functions as a surfactant, a solubilizer, with a potential to improve drug loading in the NPs. In addition, α-tocopheryl derivative may enhance oral absorption of hydrophobic materials. The use of TPGS did not appear to improve the amount of loaded myrcene in the PLGA NPs and instead it was found that the loaded myrcene was reduced when TPGS percentage increased (F9 and F10; **Table 2**).

**Example 2 - Microemulsification method for producing myrcene-encapsulated nanoparticles**

The formation of microemulsions was also used to obtain PLGA nanoparticles. A microemulsion is a system of water, oil and an amphiphile which is a single optically isotropic and thermodynamically stable liquid solution. Its formation is spontaneous **(FIG. 2).** Soybean lecithin, which is a natural lipid containing mixture of phospholipids, has been previously used as an amphiphile for the preparation of various delivery nanosystems such nanoemulsions, liposomes, micelles and nanoparticles. This method allows the formation of spherical nanocapsules where the oily core composed of myrcene, is entrapped and retained in a thin dense wall formed by PLGA polymer and phosphatidylcholine.

**Formulation**

PLGA based NPs were prepared using a microemulsification method according to the formulas in Table 3.

**Table 3.** Formulas of NPs prepared using the microemulsification method.

| **Formula** | | **F12** | **F13** | **F14** | **F15** | **F16** | **F17** | **F18** |
|---|---|---|---|---|---|---|---|---|
| **Organic phase** | Myrcene | - | - | - | 24 mg | 24 mg | 48 mg | 48 mg |
| | PLGA (Resomer 502) | 11 mg | 11 mg | 11 mg | 11 mg | 11 mg | 11 mg | 11 mg |
| | Epikuron® 200 (Soybean lecithin) | 13 mg | 13 mg | 13 mg | 13 mg | 13 mg | 13 mg | 13 mg |
| | Acetone | 2.5 mL | 2.5 mL | 2.5 mL | 2.5 mL | 2.5 mL | 2.5 mL | 2.5 mL |
| **Aqueous phase** | PVA 0.5% w/v | 5 mL | - | - | - | - | - | - |
| | Pluronic® F-68 **0.5%** w/v | - | 5 mL | | 5 mL | - | 5 mL | - |
| | Pluronic® F-68 **5**% w/v | - | - | 5 mL | - | 5 mL | - | 5 mL |

Myrcene-loaded NPs were prepared according to a method previously described by Iannitelli et al (Int. J. Mol. Sci. 2011, 12, 5039-5051). Briefly, PLGA (11 mg) and Epikuron 200 (13 mg) were dissolved in 2.5 mL acetone. Myrcene (48 mg) was added to the organic solution. The organic phase containing the drug was added at once to the aqueous phase (5 mL of Pluronic® F-68 (0.5% w/v or 5% w/v) or poly vinyl alcohol (PVA) (0.5% w/v)) with continuous stirring. The beaker containing the mixture was covered with parafilm to prevent evaporation of myrcene and stirring was continued for 10 min. Acetone was removed using a rotary evaporator without heating over a period of 40 min. To remove non-encapsulated compounds as well as the excess surfactant, the suspension was adjusted up to 45 ml with MQ water and centrifuged at 10,000 rpm at 4°C for 30 min. The pellet was re-suspended in 45 mL MQ water and centrifuged again using the same condition. The NPs pellet was resuspended in 2 mL MQ water, and the preparation was freeze-dried. The lyophilized NPs were collected as white cotton-like material. The supernatant was also collected and lyophilized to measure the non-encapsulated myrcene.

Samples were prepared for GC-MS analysis. Lyophilized NPs were dissolved in DCM. To facilitate dissolution, samples were sonicated in the bath sonicator for 5 min. Samples were filtered using 1 μm syringe filter (compatible with DCM). 1.5 mL of sample solutions were transferred into suitable vials and sealed. The lyophilized supernatants stored

at -20°C were weighed and samples were prepared for GC-MS analysis as described for the NPs. Briefly, 5 mg of NP samples were dissolved in DCM and 1.5 mL of the solution was transferred into a suitable vial and sealed.

## Characterization of Nanoparticles Prepared by Microemulsification

The microemulsification method of NPs preparation involves the preparation of an intermediate microemulsion that forms spontaneously when a high concentration and a correct combination of surfactants is used (normally a surfactant and a co-surfactant; in this work Pluronic® F-68 and Epikuron 200. The solvent is then diffused from the droplets of the emulsion and evaporated off leaving the NPs behind.

Mean diameter, size distribution, zeta potential, weight, yield, amounts of loaded myrcene, EE% and DL% of NPs prepared by microemulsification are presented in **Table 4**. **FIG. 9A** shows the size distribution by intensity and **FIG. 9B** shows the zeta potential distribution of sample F17.

**Table 4.** Characteristics of NPs prepared by microemulsification

| Acetone evaporation | Formula: Myrcene amount | Diameter (nm) | PdI | Zeta Potential (mV) | NPs weight (mg) | Yield (%) | Loaded myrcene (mg) | EE% | DL% |
|---|---|---|---|---|---|---|---|---|---|
| rotary evaporat or | F12: Blank with PVA 0.5% | aggr | - | - | - | - | - | - | - |
| | F13: Blank with Pluronic® F-68 0.5% | 145.9 | 0.156 | -30.3 | 17.52 | 73.00 | - | - | - |
| | F14: Blank with Pluronic® F-68 5% | 158.5 | 0.160 | -33.4 | 17.16 | 71.50 | - | - | - |
| | F15: Pluronic® F-68 0.5%; 24 mg | 262.3 | 0.124 | -34.4 | 17.93 | 37.35 | 0.004 | 0.017 | 0.022 |
| | F16: Pluronic® F-68 5%; 24 mg | 242.4 | 0.182 | -30.9 | 18.41 | 38.35 | 0.004 | 0.017 | 0.022 |
| | F17: Pluronic® F-68 0.5%; 48 mg | 263.9 | 0.155 | -33.2 | 17.08 | 23.72 | 0.005 | 0.010 | 0.029 |
| | F18: Pluronic® F-68 5%; 48 mg | 243.1 | 0.175 | -31.2 | 18.54 | 25.75 | 0.006 | 0.013 | 0.032 |

NPs prepared using PVA 0.5% (as the aqueous phase) were not physically stable and aggregates formed during preparation (F12). Particle size of blank NPs was around 150 nm (F13, F14) whereas myrcene loaded NPs were approximately 100 nm larger with a diameter around 250 nm. All NPs showed good polydispersity of less than 0.2 and a negative zeta potential between -30 to -35.

In all cases particles in the nanometric range with a narrow size distribution (PdI < 0.2) and zeta potential around -32 mV were obtained. No differences between loaded and blank (-30mV) nanoparticles were observed. Thus, almost no drug is retained on the surface.

**Example 3** - **Emulsification (high speed homogenizer) method for producing myrcene-encapsulated nanoparticles**

A method based on the emulsification of polymer organic solution into a water phase, followed by organic solvent evaporation **(FIG. 3)** was used for preparation of myrcene-encapsulating nanoparticles. The organic phase is poured into the continuous phase (aqueous phase) in which a surfactant is dissolved to impart stability to the emulsion. Emulsification is carried out under high-shear force to reduce the size of the emulsion droplet. This process will largely determine the final particle size.

**Formulation**

PLGA based NPs were prepared using a single emulsion method according to the formulas in Table 5. The emulsification was performed with a Polytron homogenizer.

**Table 5.** Formulas of NPs prepared using the emulsification method.

| Formula | | F19 | F20 | F21 | F22 | F23 | F24 |
|---|---|---|---|---|---|---|---|
| **Organic phase** | Myrcene* | - | 10%<br>4 mg | 20%<br>8 mg | 40%<br>16 mg | 80%<br>32 mg | 100%<br>40 mg |
| | PLGA (Resomer 502) | 40 mg | 40 mg | 40 mg | 40 mg | 40 mg | 40 mg |
| | Ethyl acetate | 1 mL | 1 mL | 1 mL | 1 mL | 1 mL | 1 mL |
| **Aqueous phase** | PVA 0.5% w/v | 5 mL | 5 mL | 5 mL | 5 mL | 5 mL | 5 mL |
| * % w/w based on PLGA weight | | | | | | | |

PLGA (40 mg) was dissolved in 1 mL ethyl acetate. Myrcene (10 - 100% w/w based on PLGA) was added to the organic solution. Preparation of the NPs was done using a high-speed homogenizer). The probe of the homogenizer was immersed inside a falcon tube containing 5 mL PVA (0.5% w/v). The organic phase containing the drug was added drop-wise using a pipette with the homogenizer set at 24,000 rpm and homogenization was continued for 1 min. Homogenization was done on ice to reduce temperature generated during the process. The organic solvent was removed using a rotary evaporator without heating over a period of 1 h. To remove non-encapsulated compounds as well as the excess surfactant, the suspension was adjusted up to 45 ml with MQ water and centrifuged at 10,000 rpm at 4°C for 30 min. The pellet was re-suspended in 45 mL MQ water and centrifuged again using the same conditions. The NPs pellet was resuspended in 2 mL MQ water, and the preparation was freeze-dried. The lyophilized NPs were collected as white cotton-like material. The supernatant was also collected and lyophilized to measure the non-encapsulated myrcene.

Samples were prepared for GC-MS analysis. Lyophilized NPs were dissolved in DCM. To facilitate dissolution, samples were sonicated in the bath sonicator for 5 min. Samples were filtered using 1 μm syringe filter (compatible with DCM). 1.5 mL of sample solutions were transferred into suitable vials and sealed. The lyophilized supernatants stored at -20°C were weighed and samples were prepared for GC-MS analysis as described for the NPs. Briefly, 5 mg of NP samples were dissolved in DCM and 1.5 mL of the solution was transferred into a suitable vial and sealed.

**Characterization of Nanoparticles Produced by Emulsification**

Mean diameter, size distribution, zeta potential, weight, yield, amounts of loaded myrcene, EE% and DL% of NPs prepared by the emulsification method are presented in **Table 6. FIG. 10A** shows the size distribution by intensity and **FIG. 10B** shows the zeta potential distribution of sample F20.

| Table 6. Characteristics of NPs prepared by emulsification/homogenization | | | | | | | | | |
|---|---|---|---|---|---|---|---|---|---|
| **Ethyl acetate evap** | **Formula: Myrcene*** | **Diameter (nm)** | **PdI** | **Zeta Potential (mV)** | **NP weight (mg)** | **Yield (%)** | **Loaded myrcene (mg)** | **EE%** | **DL%** |
| Rotary evaporator | F19: Blank | 225.7 | 0.092 | -25.6 | 26.37 | 65.93 | - | - | - |
| | F20: 10%, 4 mg | 262.3 | 0.127 | -24.0 | 24.07 | 54.70 | 0.165 | 4.13 | 0.69 |
| | F21: 20%, 8 mg | 266.3 | 0.108 | -25.5 | 25.06 | 52.20 | 0.179 | 2.24 | 0.71 |
| | F22:40%, 16 mg | 267.5 | 0.211 | -25.7 | 18.59 | 33.20 | 0.230 | 1.44 | 1.23 |
| | F23: 80%, 32 mg | 351.0 | 0.132 | -24.2 | 22.40 | 31.11 | 0.288 | 0.90 | 1.28 |
| | F24: 100%, 40 mg | 405.0 | 0.166 | -24.7 | 22.57 | 28.21 | 0.265 | 0.66 | 0.25 |
| * % w/w based on PLGA weight | | | | | | | | | |

Particle size was 225 nm for of blank NPs, -265 nm for myrcene loaded NPs with drug concentration between 10-40%, and between 350 - 400 nm for myrcene loaded NPs with drug concentration between 80-100%. However, the NPs in F22, F23, and F24 appeared to be damaged during the centrifugation, resulting in turbid supernatant. The polydispersity of all NPs was good (0.2 or less) and a negative zeta potential slightly lower than other methods (around -25 mV). No differences between loaded and blank (-25mV) nanoparticles were observed. Thus, almost no drug on the surface is retained.

The emulsification method showed a higher drug loading efficiency in comparison with other methods used in this work; the highest DL% values were obtained for F23 (1.29%) and F 20 (1.20%). However, there have been signs of damaged or exploded NPs during centrifugation, as shown by turbid supernatant and a proportion of the polymer sticking on the falcon tube's wall near the top of the supernatant. The damage of the NPs can be reduced and may result in a higher drug loading efficiency by performing the centrifugation at a lower spinning speed or replacing centrifugation with another purification method such as dialysis.

**Example 4 - Solvent optimization**

Next, the volume of solvent used was optimized to reduce the time of the evaporation step to minimize the potential evaporation of myrcene during the solvent evaporation step. The effect of reducing the volume of solvent was explored using two preparation methods: nanoprecipitation (acetone) and emulsification (Ethyl acetate).

**Emulsification method**

The effect of varying the volume of the organic solvent using the single emulsion method was determined. Nanoparticles were prepared and lyophilized as previously described in Example 3.

| Table 7. Formulas of NPs prepared using the emulsification method. | | | | | | | |
|---|---|---|---|---|---|---|---|
| **Formula** | | **F25** | **F26** | **F27** | **F28** | **F29** | **F30** |
| **Organic phase** | Myrcene | 20%* 8 mg | 20%* 8 mg | 20%* 8 mg | 20%* 8 mg | 20%* 8 mg | 20% * |
| | PLGA (Resomer 502) | 40 mg | 40 mg | 40 mg | 40 mg | 40 mg | 40 |
| | Ethyl acetate | 0.125 mL | 0.25 mL | 0.5 mL | 0.75 mL | 1 mL | 1 mL |

(continued)

| Table 7. Formulas of NPs prepared using the emulsification method. | | | | | | | |
|---|---|---|---|---|---|---|---|
| Formula | | F25 | F26 | F27 | F28 | F29 | F30 |
| Aqueous phase | PVA (0.5% w/v) | 5 mL | 5 mL | 5 mL | 5 mL | 5 mL | 5 mL |
| | Evaporation time | 7 min | 10 | 12 | 15 | 25 min | 45 min |
| * % w/w based on PLGA weight | | | | | | | |

## Nanoprecipitation method

The effect of varying the volume of the organic solvent using the nanoprecipitation method was determined. Nanoparticles were prepared and lyophilized as previously described in Example 1.

| Table 8. Formulas of NPs prepared using the nanoprecipitation method. | | | | | | | |
|---|---|---|---|---|---|---|---|
| | Organic Phase: | F31 | F32 | F33 | F34 | F35 | F36 |
| Organic phase | Myrcene* | 40%<br>9 mg | 40%<br>9 mg | 40%<br>9 mg | 40%<br>9 mg | 40%<br>9 mg | 40%<br>9 mg |
| | PLGA (Resomer 502) | 22.5 mg | 22.5 mg | 22.5 mg | 22.5 mg | 22.5 mg | 22.5 mg |
| | Span® 60 | 7.5 mg | 7.5 mg | 7.5 mg | 7.5 mg | 7.5 mg | 7.5 mg |
| | Acetone | 0.75 mL | 0.75 mL | 1 mL | 1 mL | 1.5 mL | 1.5 mL |
| Aqueous phase | PVA 0.5% w/v | 4.5 mL | 4.5 mL | 4.5 mL | 4.5 mL | 4.5 mL | 4.5 mL |
| Conditions | Temperature during addition of organic phase | r.t. | cool bath | r.t. | cool bath | r.t. | cool bath |
| | Evaporation time | 7 min | 7 min | 10 min | 10 min | 15 min | 15 min |
| * % w/w based on PLGA weight | | | | | | | |

## Characterization of nanoparticles produced using lower amounts of solvent

We first started with the minimum volume of solvent to dissolve the organic phase components. In general, the particle size was found to decrease as the volume of the solvent was increased **(Table 9).** FIG. **11A** shows the size distribution by intensity and **FIG. 11B** shows the zeta potential distribution of sample F28. **FIG. 11C** shows the size distribution by intensity and **FIG. 11D** shows the zeta potential distribution of sample F31.

In the single emulsion method, low volumes of ethyl acetate of 0.125 (F25) and 0.25 ml (F26) resulted in NPs with large diameter that were not re-disposable after centrifugation. With 0.5 ml of ethyl acetate, the produced NPs were physically stable. F27 showed the largest diameter among stable NPs but also the highest drug loading efficiency of 0.486 mg (EE%: 6.08, DL%: 2.02). It is important to point out that this formula was centrifuged at 6,000 rpm and it could be collected at this speed due to its large diameter ≈ 670 nm, whereas all the other formulas were centrifuged at 10,000 rpm. It is possible that the higher centrifugation speed may cause deformation of the NPs resulting in the escape of drug. Although myrcene is not soluble in water it could be solubilized by the surfactant, PVA or Pluronic.

Similarly, in the nanoprecipitation method, F31 formulas with larger diameter (≈ 400 nm) showed higher myrcene loading efficiency of up to 0.188 mg in F31 (EE%: 2.09, DL%: 1.04). The use of cool bath during the addition of organic phase appeared to increase the particle size but it could not be concluded that it can increase drug loading (by preventing/reducing drug evaporation during preparation).

**Table 9.** Characteristics of NPs prepared by emulsification/homogenization or nanoprecipitation

| | Formula | Evap-time (min) | Diamet er (nm) | PdI | Zeta Potential (mV) | NPs weight (mg) | Yield (%) | Loaded myrcene (mg) | EE% | DL% |
|---|---|---|---|---|---|---|---|---|---|---|
| Emulsification | F25 | 7 | 1612.0 | 0.495 | - | Pellet not re-susp | | - | - | - |
| | F26 | 10 | 1136.0 | 0.275 | - | Pellet not re-susp | | - | - | - |
| | F27 | 12 | 671.0 | 0.242 | -25.1 | 24.11 | 50.23 | 0.486 | 6.08 | 2.02 |
| | F28 | 15 | 275.3 | 0.081 | -25.4 | 23.75 | 49.48 | 0.132 | 1.65 | 0.55 |
| | F29 | 25 | 279.4 | 0.148 | -24.9 | 24.97 | 52.02 | 0.049 | 0.61 | 0.2 |
| | F30 | 45 | 270.9 | 0.124 | -24.8 | 25.24 | 52.58 | 0.036 | 0.45 | 0.14 |
| Nano precipitation | F31 | 7 | 405.0 | 0.39 | -37.1 | 18.14 | 57.56 | 0.188 | 2.09 | 1.04 |
| | F32 | 7 | 439.9 | 0.397 | -40.9 | 17.92 | 56.89 | 0.150 | 1.67 | 0.84 |
| | F33 | 10 | 325.1 | 0.221 | -35.8 | 18.74 | 59.49 | 0.130 | 1.44 | 0.69 |
| | F34 | 10 | 339.2 | 0.39 | -36.2 | 18.43 | 58.51 | 0.147 | 1.63 | 0.8 |
| | F35 | 15 | 242.1 | 0.158 | -36.5 | 18.27 | 58.00 | 0.062 | 0.69 | 0.34 |
| | F36 | 15 | 261.0 | 0.193 | -39.7 | 16.94 | 52.21 | 0.045 | 0.50 | 0.27 |

**Example 5** - **Measurement of myrcene in the supernatant**

Because of the relatively low amount of loaded myrcene in the NPs, we sought to measure the drug in the supernatant to understand how the drug is lost. Table 10 shows that the total amount of detected myrcene, which includes both loaded myrcene in the NPs and non-loaded free myrcene that present in the supernatant, was between 0.06 - 3.44% of the starting amount of the drug. The results shown in **Table 10** indicate that the majority of myrcene is lost at some stage; during NPs preparation, during sample preparation for analysis, or during storage.

It is likely that the myrcene loss occurs by evaporation during production and not during storage, since the freeze-dried samples were stored in the freezer in well closed containers which should prevent the evaporation of the drug.

**Table 11.** Amounts of myrcene loaded in the NPs and in the supernatant.

| **Method** | **Myrcene** | | | |
|---|---|---|---|---|
| | **Starting amount (mg)** | **In supernatant (mg)** | **In NPs (mg)** | **Detected myrcene (in supernatant + NPs)/ starting amount (%)** |
| Nanoprecipitation | 9 | 0.108 | 0.155 | 2.92% |
| Microemulsion | 24 | 0.014 | 0.004 | 0.08% |
| Microemulsion | 48 | 0.025 | 0.006 | 0.06% |
| Emulsification, homogenizer | 8 | 0.096 | 0.179 | 3.44% |

**Example 6 - Factors Resulting in Myrcene Loss**

**Filtration during sample preparation for GC-MS analysis**

The effect of filtration on possible myrcene loss was studied on myrcene-loaded NPs prepared using the emulsification method (F27, F28, F29 and F30). Filtration was used to remove the non-dissolved components of the NPs in DCM, such as PVA, during the preparation of samples for GC-MS analysis. Insoluble impurities in the samples may result in clogging the injector during analysis and may impede the measurement. 5 mg of the lyophilized NPs were dissolved in DCM. To facilitate dissolution, samples were sonicated in the bath sonicator for 5 min. Samples were filtered using 1 $\mu$m syringe filter (compatible with DCM). Samples' solutions (1.5 mL each) were transferred into suitable vials and sealed. Another set of samples (each weighing 5 mg of NPs) were prepared in DCM without filtration.

Amounts of loaded myrcene in the NPs, EE% and DL% before and after filtration using a 1 $\mu$m syringe filter are displayed in **Table 11.**

**Table 11.** Amounts of loaded myrcene, EE% and DL% before and after filtration of NPs prepared by emulsification.

| **Formula** | **Before filtration (myrcene)** | | | **After filtration (myrcene)** | | |
|---|---|---|---|---|---|---|
| | **Amount loaded (mg)** | **EE%** | **DL%** | **Amount loaded (mg)** | **EE%** | **DL%** |
| F27 | 0.486 | 6.08 | 2.02 | 0.368 | 4.60 | 1.53 |
| F28 | 0.132 | 1.65 | 0.55 | 0.121 | 1.51 | 0.51 |
| F29 | 0.049 | 0.61 | 0.20 | 0.045 | 0.56 | 0.18 |
| F30 | 0.036 | 0.45 | 0.14 | 0.034 | 0.43 | 0.13 |

Filtration resulted in a relative reduction in the measured amounts of myrcene. Most samples showed little concentration reduction, however this effect was more significant in F27 which has the highest concentration of loaded myrcene. The amount of myrcene was reduced by about 25% from 0.486 mg before filtration to 0.368 mg after filtration. This may be due to an interaction between the drug and the filter resulting in entrapment of a proportion of the drug. Based on these findings, filtration was not the major cause of myrcene loss during preparation.

## Organic solvent evaporation

To study the effect of evaporation performed using rotary evaporator on the loss of myrcene, two different samples were prepared as in the table below. In the first sample, 40 mg of Resomer 502 was dissolved in 1 ml of ethyl acetate then 8 mg of myrcene was added. In the second sample, 8 mg of myrcene was mixed with 1 ml of ethyl acetate without the polymer. The two samples were subjected to evaporation under pressure using the rotary evaporator without heating for 1 h. Formulas are shown in **Table 12.**

**Table 12.** Formulas used to study the effect of evaporation performed using rotary evaporator on the loss of myrcene.

| Formula | F37 | F38 |
|---|---|---|
| Myrcene | 8 mg | 8 mg |
| PLGA (Reseomer 502) | 40 mg | - |
| Ethyl acetate | 1 mL | 1 mL |
| Evaporation time | 60 min | 60 min |

The amount of myrcene retained in the sample and percentage of myrcene loss are shown in **Table 13.**

**Table 13.** Amount of myrcene remained in the sample and percentage of lost myrcene after evaporation in the rotary evaporator for 1 h.

| Formula: Myrcene; PLGA; EA (solution) | Retained myrcene (mg) | Loss of myrcene (%) |
|---|---|---|
| F37: 8 mg; 40 mg; 1 mL | 3.27 mg | 59.1 |
| F38: 8 mg; 0 mg; 1 mL | < 0.001 | 100 |

Rotary evaporation of the myrcene and ethyl acetate sample without PLGA (F38) resulted in complete loss of the myrcene. However, when PLGA was added to myrcene (F37), about 40% of the starting drug was maintained which may be due to adsorption of the drug to the polymer. This result may importantly indicate that myrcene entrapped in the polymer (in the NPs) may be protected from evaporation whereas any free drug may be subject to loss during the evaporation step.

## Evaporation, Centrifugation, and Freeze-Drying

To investigate the possible loss of myrcene during the different steps of NPs production, samples were prepared using the nanoprecipitation method as described in Example 1 and according to the formulas in **Table 14.**

F39 was centrifuged at 10,000 rpm at 4°C for 30 min. The NPs pellet was resuspended in 2 mL MQ water, and the preparation was freeze-dried. F40 and F41 were freeze-dried immediately after evaporation of the solvent without centrifugation. The amount of myrcene in the samples was measured using GC-MS spectrometry. 5 mg of each sample was dissolved in 2 mL of DCM, and the solution was filtered using 1 μm syringe filter, filled in a suitable vial and sealed.

**Table 14.** Formulas used to study the effect of different production stages on the loss of myrcene with NPs prepared using emulsification.

| Formula | | F39 | F40 | F41 |
|---|---|---|---|---|
| **Organic phase** | Myrcene* | 20%<br>4.5 mg | 20%<br>4.5 mg | 20%<br>4.5 mg |
| | PLGA (Resomer 502) | 22.5 mg | 22.5 mg | - |
| | Span® 60 | 7.5 mg | 7.5 mg | - |
| | Acetone | 1 mL | 1 mL | 1 mL |
| **Aqueous phase** | PVA 0.5% w/v | 4.5 mL | 4.5 mL | 4.5 mL |

(continued)

| **Table 14.** Formulas used to study the effect of different production stages on the loss of myrcene with NPs prepared using emulsification. | | | | |
|---|---|---|---|---|
| **Formula** | | **F39** | **F40** | **F41** |
| **Conditions** | Temperature during addition of organic phase | cool bath | cool bath | cool bath |
| | Evaporation time | 10 min | 10 min | 10 min |
| * % w/w based on PLGA weight | | | | |

Mean diameter, size distribution, zeta potential, weight of lyophilizate, amount of myrcene remained in the sample, and percentage of myrcene loss of the different formulations are displayed in **Table 15. FIG. 9A** shows the size distribution by intensity and **FIG. 9B** shows the zeta potential distribution of sample F41.

**Table 15.** Characteristics of different formulations prepared to investigate myrcene loss during production stages (starting myrcene = 4.5 mg).

| **Formula** (micelles) | **Diameter (nm)** | **PdI** | **Zeta Potentia l (mV)** | **Weight of lyophilizate (mg)** | **Amount of myrcene (mg)** | **Myrcene loss (%)** |
|---|---|---|---|---|---|---|
| F39: centrifuged and lyophilized NPs | 285.9 | 0.174 | -36.2 | 19.17 | 0.136 | 97.0 |
| F40: lyophilized NPs without centrifugation | 274.4 | 0.157 | -40.1 | 52.57 | 0.376 | 91.6 |
| F41: lyophilized emulsion without centrifugation | 357.0 | 0.154 | -7.71 | 24.50 | 0.335 | 92.6 |

F41 was prepared by the addition of myrcene dissolved in acetone, without the polymer, to the aqueous phase. The measured size of 357 nm indicates the formation of an emulation of myrcene in the presence of the PVA as the surfactant. These are not polymeric nanoparticles because PLGA was not used, just micelles of PVA. F39 was prepared to explore whether centrifugation could be responsible for loss of drug from the NPs during centrifugation. However, even F40 in which NPs were prepared without centrifugation showed a low retention of the drug. Similarly, F3 which is an emulsion of myrcene (without the addition of PLGA) also exhibited similar retention of the drug after lyophilization. These findings indicate that a large proportion of the drug may be lost during lyophilization. There is a possibility that some of the drug escapes from NPs during centrifugation, but this could not be examined with the current method of preparation, since measurement of the drug should be done immediately after centrifugation, not after freeze drying.

Several methods were used to encapsulate myrcene in PLGA NPs including: (i) nanoprecipitation, (ii) emulsification using a high-speed homogenizer and (iii) microemusification. To improve the loading efficiency, we have also developed and studied several formulations. To reduce the possible evaporation of myrcene, the preparation method was optimized, with regards to temperature during NPs production, and use of a rotary evaporator without heating the samples, which resulted in improved drug loading capacity.

We obtained particles in the nanometric range with narrow size distribution (PdI < 0.2) and negative zeta potential (from -25 to -35 mv). The highest loading capacity was achieved with the single emulsion method, with entrapment efficiency up to 6 % of the starting drug (F27). The low amount of myrcene measured in the supernatants, collected after centrifugation of NPs, indicates that the drug is lost during the production process, and not due to a failure of the PLGA to encapsulate the myrcene.

Myrcene has a low molecular weight (136.23 g/mol), low vapor pressure (2.09 mm Hg at 25ºC) and although it has a boiling point of 167°C it is a volatile liquid at room temperature, which makes it difficult to maintain within the NPs and to prevent its evaporation. Although myrcene may be evaporated during the evaporation step of the production process, performed in the rotary evaporator without heating, its evaporation is significantly reduced by the presence of PLGA. Thus, the loading of the drug in the NPs can protect myrcene from evaporation to a large extant. The amount of solvent and time of evaporation was also optimized and reduced from 1 h down to 25 min to minimize possible evaporation of the drug. Myrcene was also lost during freeze drying. It is unclear if the loss is due to evaporation or sublimation. However, myrcene was not frozen even at -80°C.

**Example 7** - **Optimization of lyophilization and centrifugation of nanoparticles** Lyophilization

To investigate whether myrcene is lost during lyophilization, 3 samples of either free myrcene, a mixture of myrcene and water, or a mixture of myrcene, water and PLGA were prepared in duplicate as illustrated in **Table 16.** Samples were lyophilized for 24 h. The vials containing the samples were weighed before and after lyophilization to calculate the weight of the dried sample.

**Table 16.** Formulas of free myrcene, water, and PLGA to evaluate the loss of free myrcene during lyophilization.

| Formula | F42 | F43 | F44 |
|---|---|---|---|
| Myrcene | 8 mg | 8 mg | 8 mg |
| PLGA (Resomer 502) | - | - | 40 mg |
| Water | - | 2 mL | 2 mL |

The results of the lyophilization assay are shown in **Table 17.**

**Table 17.** Starting and remaining weight of samples containing myrcene before and after lyophilization, respectively.

| Formula | Starting weight (mg) | Weight after lyophilization (mg) |
|---|---|---|
| F42: Myrcene: 8 mg | 8 | 0.88 |
| F43: Myrcene: 8 mg; PLGA: 40 mg | 48 | 1.17 |
| F44: Myrcene: 8 mg; PLGA: 40 mg; Water: 2mL | 48 (+ 2 mL) | 39.71 |

The results demonstrate that most of the myrcene is lost during the process of lyophilization over 24 h, whether it is free or in combination with the polymer and water. Myrcene is not frozen at the temperature of lyophilization (around -80°C), which may indicate that it is lost by evaporation under vacuum in the freeze dryer, rather than by sublimation.

As lyophilization was a crucial step in the loss of myrcene from the NPs, we hypothesized that the reduction of lyophilization time may reduce the loss of myrcene during this process. An aliquot of 100 μL of NPs suspension of formulas F49 (myrcene: 100% w/w; centrifugation: Falcon tubes, 10,000 rpm, 30 min, 4°C) and F54 (myrcene: 100% w/w; centrifugation: Amicon tubes, 4,000 ×*g*, 30 min, 12°C) were transferred into an Eppendorf, frozen, and lyophilized for only 2 h; this time was sufficient to remove 100 μL of water. The dried product was then dissolved in DCM and the content of myrcene was analyzed using a GC-MS instrument as previously described. Samples were prepared in duplicates.

Centrifugation

After confirming that myrcene is lost during lyophilization, we measured the amount of myrcene loaded in the NPs before the lyophilization step. Two different centrifugation protocols were compared to determine the optimal conditions. PLGA NPs were prepared using the single emulsion homogenization method described in Example 3. Sample formulas are present in **Tables 18** and **19.** Formulas F45 - F49 were centrifuged in Falcon tubes at 10,000 rpm at 4°C for 30 min to collect the NPs. Formulas F50 - F54 were spun in Amicon tubes at 4,000 ×g at 12°C for 30 min. Samples were prepared in duplicates.

**Table 18.** Formulas of NPs prepared using the emulsification method and centrifuged in Falcon tubes at 10,000 rpm at 4°C for 30 min.

| Formula | | F45 | F46 | F47 | F48 | F49 |
|---|---|---|---|---|---|---|
| **Organic phase** | Myrcene* | 20%<br>8 mg | 40%<br>16 mg | 60%<br>24 mg | 80%<br>32 mg | 100%<br>40mg |
| | PLGA (Resomer 502) | 40 mg | 40 mg | 40 mg | 40 mg | 40 mg |
| | Ethyl acetate | 1 mL | 1 mL | 1 mL | 1 mL | 1 mL |

(continued)

| Table 18. Formulas of NPs prepared using the emulsification method and centrifuged in Falcon tubes at 10,000 rpm at 4°C for 30 min. | | | | | | |
|---|---|---|---|---|---|---|
| **Formula** | | **F45** | **F46** | **F47** | **F48** | **F49** |
| **Aqueous phase** | PVA 0.5% w/v | 5 mL | 5 mL | 5 mL | 5 mL | 5 mL |
| **Centrifugation** | Falcon tubes: 10,000 rpm, 30 min , 4°C | | | | | |
| * % (w/w) based on PLGA | | | | | | |

| Table 19. Formulas of NPs prepared using the emulsification method and centrifuged in Amicon tubes at 4,000 xg at 12°C for 30 min. | | | | | | |
|---|---|---|---|---|---|---|
| **Formula** | | **F50** | **F51** | **F52** | **F53** | **F54** |
| **Organic phase** | Myrcene* | 20%<br>8 mg | 40%<br>16 | 60%<br>24 mg | 80%<br>32 mg | 100%<br>40mg |
| | PLGA (Resomer 502) | 40 mg | 40 mg | 40 mg | 40 mg | 40 mg |
| | Ethyl acetate | 1 mL | 1 mL | 1 mL | 1 mL | 1 mL |
| **Aqueous phase** | PVA 0.5% w/v | 5 mL | 5 mL | 5 mL | 5 mL | 5 mL |
| **Centrifugation** | Amicon tubes: 4,000 xg, 30 min, 12°C | | | | | |
| * % (w/w) based on PLGA | | | | | | |

The results of increasing the starting amount of myrcene are presented in **Table 20.** Increasing the starting amount of myrcene was found to enhance the amount of loaded drug in the NPs up to drug load of around 5% when 100% (w/w, myrcene to PLGA; 40 mg myrcene and 40 mg PLGA) of myrcene was used. The amount of loaded myrcene is slightly higher when the nanoparticles are collected by centrifugation in a Falcon tube at 10,000 rpm in comparison with those collected by an Amicon tube at 4,000 ×*g.*

| Table 20. Characteristics of myrcene-loaded NPs prepared, using emulsification with a homogenizer, with various amounts of starting myrcene and centrifuged in either falcon or amicon tubes. | | | | | | | |
|---|---|---|---|---|---|---|---|
| **Formula: Myrcene concentration *** | **Centrifugation procedure** | **Diameter (nm)** | **PdI** | **Zeta Potential (mV)** | **Amount of loaded myrcene (mg)** | **EE %** | **DL %** |
| F45: 20% (w/w); 8 mg | Faclon tubes: 10,000 rpm, 4°C, 30 min | 217.7 | 0.099 | -25.8 | 0.871 | 10.89 | 2.17 |
| F46: 40% (w/w); 16 mg | | 227.6 | 0.093 | -25.9 | 1.030 | 6.44 | 2.58 |
| F47: 60% (w/w); 24 mg | | 245.0 | 0.130 | -24.8 | 1.228 | 5.12 | 3.07 |
| F48: 80% (w/w); 32 mg | | 253.6 | 0.193 | -26.1 | 1.483 | 4.861 | 3.71 |
| F49: 100% (w/w); 40 mg | | 285.6 | 1.61 | -26.9 | 1.754 | 4.39 | 4.39 |
| F50: 20% (w/w); 8 mg | Amicon tubes: 4,000 ×*g*, 12°C, 30 min | 222.9 | 0.109 | -21.8 | 0.884 | 11.05 | 2.21 |
| F51: 40% (w/w); 16 mg | | 234.2 | 0.132 | -20.7 | 1.222 | 7.64 | 3.06 |

(continued)

| **Table 20.** Characteristics of myrcene-loaded NPs prepared, using emulsification with a homogenizer, with various amounts of starting myrcene and centrifuged in either falcon or amicon tubes. | | | | | | | |
|---|---|---|---|---|---|---|---|
| **Formula: Myrcene concentration *** | **Centrifugation procedure** | **Diameter (nm)** | **PdI** | **Zeta Potential (mV)** | **Amount of loaded myrcene (mg)** | **EE %** | **DL %** |
| F52: 60% (w/w); 24 mg | | 264.6 | 0.151 | -22.9 | 1.275 | 5.31 | 3.19 |
| F53: 80% (w/w); 32 mg | | 287.9 | 0.167 | -22.2 | 1.298 | 4.06 | 3.25 |
| F54: 100% (w/w); 40 mg | | 326.2 | 0.13 | -23.7 | 1.476 | 3.69 | 3.69 |
| * % (w/w) based on PLGA | | | | | | | |

## Comparison of Production Methods

Once myrcene loss during lyophilization was confirmed, we repeated the preparation of NPs using the three preparation methods described previously herein to select the best method for optimal drug loading. The myrcene content in the NPs was measured after centrifugation but before lyophilization, to select the method that provided the highest myrcene loading efficiency and amount.

NPs were prepared using emulsification induced by a high-speed homogenizer, as described in Example 3; nanoprecipitation, as described in Example 1; and microemulsion, as described in Example 2; according to formulas presented in **Table 21.** After centrifugation of the preparations, NPs were resuspended in a total volume of 1 mL MQ water. Samples were prepared for GC-MS analysis as previously described. 100 μL of each NP suspension was transferred into an Eppendorf tube, and centrifuged at 10,000 rpm at 4°C for 30 min. The supernatant was discarded and 1 mL of DCM was added to dissolve the NPs and myrcene. The DCM solution was then diluted and the content of myrcene was analyzed using a GC-MS instrument as described previously. Samples were prepared in duplicates.

**Table 21.** Formulas of myrcene-loaded NPs prepared using different methods.

| **Formula** | **F49** | | **F54** | | **F55** | | **F56** | |
|---|---|---|---|---|---|---|---|---|
| **Method** | **Emulsification-homogenizer** | | **Emulsification-homogenizer** | | **Nanoprecipitation** | | **Microemulsion** | |
| | Myrcene | 40 mg | Myrcene | 40 mg | Myrcene | 13.5 mg | Myrcene | 24 mg |
| **Organic phase** | PLGA (Resomer 502) | 40 mg | PLGA (Resomer 502) | 40 mg | PLGA (Resomer 502) | 22.5 mg | PLGA (Resomer 502) | 11 mg |
| | Ethyl acetate | 1 mL | Ethyl acetate | 1 mL | Span 60 | 7.5 mg | Epikuron 200 | 13 mg |
| | | | | | Acetone | 1.5mL | Acetone | 2.5 |
| **Aqueous phase** | PVA 0.5% w/v | 5 mL | PVA 0.5% w/v | 5 mL | PVA 0.5% w/v | 4.5 mL | PVA 0.5% w/v | 5 mL |
| **Centrifugati on** | Falcon tubes: 10,000 rpm, 4°C, 30 min | | Amicon tubes: 4,000 $\times g$, 12°C, 30 min | | Falcon tubes: 10,000 rpm, 4°C, 30 min | | Amicon tubes: 4,000 $\times g$, 12°C, 30 min | |
| **Evaporation time** | 25 min | | 25 min | | 12 min | | 25 min | |

As demonstrated in **Table 22,** emulsification using a homogenizer, in particular after centrifugation with a falcon tube, resulted in the highest drug load with an EE% of 9%. Nanoprecipitation also produced NPs with a relatively good load, although the maximum concentration of starting myrcene was 60%, since higher concentrations promoted aggregation of NPs. In contrast, microemulsification gave NPs with poor drug load. In both emulsification using homogenizer and microemulsification methods it is expected that the liquid drug would be entrapped inside the NPs resulting in the so-called nanocapsules, which are nanoparticles with a liquid core surrounded by a polymer shell. In the nanoprecipitation method, solid NPs known as nanospheres, a matrix of the polymer in which the drug is dispersed, are produced. In the case of liquid myrcene, more drug load may be expected when it is load in nanocapsules in comparison to nanospheres. This may explain the higher drug load achieved by emulsification using homogenizer and suggest that the formula used in the microemulsification preparation may need optimization to produce stable particles with ability to entrap the drug.

**Table 22.** Characteristics of myrcene-loaded NPs prepared using various preparation methods.

| **Formula: Myrcene concentration *** | **Method** | **Diameter (nm)** | **PdI** | **Zeta Potential (mV)** | **Amount of loaded myrcene (mg)** | **EE %** | **DL %** |
|---|---|---|---|---|---|---|---|
| F49: 100% (w/w); 40 mg | **Emulsification-homogenizer (Falcon)** | 285.6 | 1.61 | -26.9 | 3.633 | 9.08 | 9.08 |
| F54: 100% (w/w); 40 mg | **Emulsification-homogenizer (Amicon)** | 344.6 | 0.225 | -18.7 | 3.201 | 8.00 | 8.00 |
| F55: 60% (w/w); 13.5 mg | **Nanoprecipitation** | 284.1 | 0.213 | -26.2 | 0.535 | 3.96 | 2.38 |
| F56: 100% (w/w); 24 mg | **Microemulsion** | 272.9 | 0.190 | -31.7 | 0.016 | 0.06 | 0.06 |

* % (w/w) based on PLGA

**Use of a cryoprotectant to stabilize the nanoparticles**

The myrcene-loaded NPs appeared to be damaged during freezing and freeze drying, resulting in increased loss of the drug during the process of lyophilization. To test the effect of a cryoprotectant, trehalose, on stabilizing the NPs, samples were prepared using the emulsification method as previously described using the two centrifuge methods described in section 5.8.2. 50 μL of NPs suspension was mixed with 50 μL of 10% trehalose solution (to give a final mixture of 5% trehalose). The product was frozen followed by lyophilization for 2 h. The dried product was then dissolved in DCM and the content of myrcene was analyzed using a GC-MS instrument as previously described. Samples were prepared in duplicates. The results of the assay are shown in **Table 23.**

**Table 23.** Characteristics of myrcene loading in NPs prepared by emulsification with a homogenizer before and after lyophilization, with and without trehalose.

| **Formula: Myrcene*** | **Method** | **Loaded myrcene** | | | | | | | | |
|---|---|---|---|---|---|---|---|---|---|---|
| | | **No lyophilization** | | | **After lyophilization** | | | **After lyophilization in the presence of trehalose** | | |
| | | **Amt (mg)** | **EE %** | **DL %** | **Amt (mg)** | **EE %** | **DL %** | **Amt (mg)** | **EE %** | **DL %** |
| F49: 100% (w/w); 40 mg | **Emulsification-homogenizer (Falcon)** | 3.633 | 9.08 | 9.08 | 0.252 | 0.63 | 0.63 | 0.633 | 1.58 | 1. 58 |

(continued)

| Table 23. Characteristics of myrcene loading in NPs prepared by emulsification with a homogenizer before and after lyophilization, with and without trehalose. | | | | | | | | | | |
|---|---|---|---|---|---|---|---|---|---|---|
| **Formula: Myrcene*** | **Method** | **Loaded myrcene** | | | | | | | | |
| | | **No lyophilization** | | | **After lyophilization** | | | **After lyophilization in the presence of trehalose** | | |
| | | **Amt (mg)** | **EE %** | **DL %** | **Amt (mg)** | **EE %** | **DL %** | **Amt (mg)** | **EE %** | **DL %** |
| F54: 100% (w/w); 40 mg | **Emulsification-homogenizer (Amicon)** | 3.201 | 8.00 | 8.00 | 1.358 | 3.40 | 3.40 | 2.813 | 7.03 | 7. 03 |
| * % (w/w) based on PLGA | | | | | | | | | | |

**Conclusion**

Myrcene was found to be lost during lyophilization. The measurement of myrcene in the NPs suspension before lyophilization indicates that the best method of preparation is emulsification with a high-speed homogenizer. This method can produce nanocapsules that entrap the liquid myrcene in their core. Optimization of the lyophilization conditions including duration and addition of a cryoprotectant resulted in optimal conditions to enhance drug load. Factors such as centrifugation speed and freezing cause damage to the NPs and may significantly contribute to the loss of myrcene during lyophilization. Duration of lyophilization also affected myrcene loss.

NPs prepared by emulsification with homogenizer, centrifuged in amicon tube at 4,000 $\times g$, cryoprotected with trehalose 5%, and lyophilized for 2 h successfully showed the highest drug load in lyophilized NPs of approximately 7%. These conditions allowed the production of lyophilized NPs with a relatively high drug load of up to 8% when 100% (w/w, myrcene to PLGA; 40 mg myrcene and 40 mg PLGA) of myrcene was used.

**Example 8 - The synthesis and characterizations of nanoparticles containing β-myrcene, β-caryophyllene, or nerolidol**

This example shows the syntheses and characterizations of PLGA-PEG nanoparticles containing β-myrcene, β-caryophyllene or nerolidol.

β-Myrcene, β-caryophyllene and nerolidol containing PLGA-PEG or poly(ethylene glycol) methyl ether-block-poly(lactide-co-glycolide) nanoparticles were prepared by emulsification and solvent evaporation method using a high-speed homogenizer (Kinematica Polytron™ PT 2500E homogenizer, Fisher Scientific). This method is based on the emulsification of polymer organic solution into a water phase, followed by organic solvent evaporation, as illustrated in **FIG. 3.** The organic phase is poured into the continuous or aqueous phase in which a surfactant is dissolved to impart stability to the emulsion. Emulsification is carried out under high-shear force to reduce the size of the emulsion droplet. This process will largely determine the final particle size of the nanoparticles. This emulsification step is followed by the evaporation of the solvent under reduced pressure to yield the desired nanoparticles.

*The synthesis of terpenoid-containing PLGA-PEG nanoparticles*

The formulas of blank and terpenoid-containing PLGA-PEG nanoparticles, as prepared using the emulsification method, are summarized in **Table 24.**

| Table 24. Formulas of blank and terpenoid-containing PEG-PLGA nanopartiples as prepared using the emulsification method | | | | | |
|---|---|---|---|---|---|
| **Formula** | | **F57 Blank NPs** | **F58 Myrcene NPs** | **F59 β-caryophyllene NPs** | **F60 Nerolidol NPs** |
| | Myrcene | - | 10 mg (25%, w/w) | - | - |

(continued)

| Table 24. Formulas of blank and terpenoid-containing PEG-PLGA nanopartiples as prepared using the emulsification method | | | | | |
|---|---|---|---|---|---|
| **Formula** | | **F57 Blank NPs** | **F58 Myrcene NPs** | **F59 β-caryophyllene NPs** | **F60 Nerolidol NPs** |
| **Organic Phase** | β-Caryophyllene | - | - | 10 mg (25%, w/w) | - |
| | Nerolidol | - | - | - | 10 mg (25%, w/w) |
| | PLGA-PEG | 40 mg | 40 mg | 40 mg | 40 mg |
| | Ethyl acetate | 1 mL | 1 mL | 1 mL | 1 mL |
| **Aqueous Phase** | PVA 0.5% w/v | 5 mL | 5 mL | 5 mL | 5 mL |

In a container, 40 mg of PLGA-PEG (PEG average Mn 2,000, PLGA average Mn 11,500; lactide:glycolide 50:50) was dissolved in 1 mL of ethyl acetate, followed by the addition of 10 mg of either myrcene, β-caryophyllene, or nerolidol (based on 12.5% w/w of PLGA weight) to give the organic phase solution. In a separate falcon tube containing 5-mL of PVA (0.5% w/v) and the probe of the homogenizer, the terpenoid-containing organic solution was added drop-wise using a pipette over 1 minute under homogenization conditions operating at 24,000 rpm and 0 °C. The organic solvent was then removed using a rotary evaporator without heating over at least 30 minutes to give a mixture comprising the terpenoid-containing nanoparticles. In order to remove non-encapsulated terpenoids and the remaining surfactant (PVA), the mixture was diluted with 10 mL of deionized and purified water, transferred to Amicon tubes (Ultracel-100 kDa regenerated cellulose membrane, 15 mL sample volume), and evaporated under centrifugation conditions (4,000 ×*g* at 12°C for 30 min); this process was repeated for a total of three times. The resulting nanoparticle concentrated suspension, as a mixture with at least 1 mL of deionized and purified water, is further mixed with 1 mL of trehalose solution (10 w/v) and the resulting mixture was lyophilized or freeze-dried at - 80 °C and <0.100 mbar (TESLAR Cryodos). The lyophilized terpenoid-containing nanoparticles were collected as white cotton-like solids. For each terpenoid, three samples of nanoparticles were prepared as triplicates for further testing and studies.

*Sample preparations for GC-MS analysis*

For GC-MS analysis and characterizations, the terpenoid-containing nanoparticle lyophalates were dissolved in 1 mL of dichloromethane (DCM) and the resulting mixture was sonicated for at least 5 minutes. The samples were then centrifuged for 4 minutes to precipitate the undissolved materials, such as trehalose and traces of PVA surfactant. From the supernatant, either 0.25 or 0.5 mL of this solution was then diluted to a final volume of 2 mL with dichloromethane (DCM), of which 1.5 mL of the final solution was transferred into a vial suitable for GC-MS analysis.

*Mean diameter and size distributions*

The mean diameter, size distribution, zeta potential, weight, yield of terpenoid-loaded PLGA-PEG NPs prepared by emulsification are presented in **Table 25.** The encapsulations terpenoids inside the nanoparticles resulted in an increase in the particle sizes. This increase in particle size was more significant with β-caryophyllene- and nerolidol-containing nanoparticles which had a particle size of approximately 350 and 323 nm, respectively, as compared to myrcene-containing nanoparticles with a diameter of 266 nm.

| Table 25. Characteristics of terpenoid-containing PLGA-PEG nanoparticles. | | | | |
|---|---|---|---|---|
| **Formula** | **Mean Size (nm)** | **PDI** | **Zeta Potential (mV)** | **Yield (mg)** |
| **F57: Blank NPs**<br>PLGA-PEG (40 mg) | 234.2 ± 2.4 | 0.159 ± 0.015 | -36.0 ± 1.3 | 36.8 ± 1.1 (92.0%) |
| **F58: Myrcene NPs**<br>- Myrcene (10 mg; 25% w/w)<br>- PLGA-PEG: (40 mg) | 266.1 ± 8.4 | 0.243 ± 0.015 | -32.4± 1.5 | 38.7 ± 1.0 (77.4%) |

(continued)

| **Table 25.** Characteristics of terpenoid-containing PLGA-PEG nanoparticles. | | | | |
|---|---|---|---|---|
| **Formula** | **Mean Size (nm)** | **PDI** | **Zeta Potential (mV)** | **Yield (mg)** |
| **F59: β-Caryophyllene NPs**<br>- β-Caryophyllene: (10 mg; 25% w/w)<br>- PLGA-PEG: (40 mg) | 350.1 ± 12.8 | 0.223 ± 0.012 | -36.5± 1.3 | 42.1 ± 0.6 (84.2%) |
| **F60: Nerolidol NPs**<br>- Nerolidol: (10 mg; 25% w/w)<br>-PLGA-PEG: (40 mg) | 322.9 ± 8.3 | 0.228 ± 0.026 | -33.9± 1.4 | 40.9 ± 0.8 (81.8%) |

Although the encapsulation of the terpenoids has increased the polydispersity of the nanoparticles, the polydispersity index (PDI) was < 0.3 for all nanoparticles. The nanoparticles also showed a negative surface charge with a measured zeta potential of around -35 mV. Furthermore, between 77-92% of the starting materials, as measured by weight, were recovered.

*Scanning Electron Microscope (SEM) Images*

SEM images confirmed the globular structure of the terpenoid-containing nanoparticles, as illustrated in **FIGs 13A-B** (myrcene) **FIG. 14A-B** (β-caryophyllene), and **FIG. 15A-B** (nerolidol). Furthermore, similar structures and size distribution of the nanoparticles were observed regardless of which terpenoid was encapsulated. As illustrated in **FIGs. 13A** and **15B,** ruptured large nanoparticles were detected and may provide an evidence that the terpenoid-containing nanoparticles were of the core-shell nano-capsule class as shown in **FIG. 16.** One possible explanation is that the nanoparticles ruptured while under vacuum during sample preparation for SEM imaging.

*Gas Chromatography-Mass Spectrometry (GC-MS) analyses*

Using the GC-MS method and conditions as described herein, the retention times for myrcene **(FIG. 4A),** β-caryophyllene **(FIG. 5A),** *cis*-nerolidol **(FIG. 6),** and *trans*-nerolidol **(FIG. 6)** were measured to be 6.93, 5.72, 6.77, and 7.16 minutes respectively, and their mass spectra are shown in **FIGs. 4B, 5B, 6A,** and **6C** respectively. The calibration curves of myrcene **(FIG. 4C)** and β-caryophyllene **(FIG. 5C)** within a concentration range of 1-60 ppm were linear with $r^2$ values of 0.9990 and 0.9993, respectively. The ratio of the *cis* to *trans* isomer of nerolidol was found to be 1.02:0.98, or about 1:1, and the calibration curves of the *cis* **(FIG. 7B)** and *trans* **(FIG. 7D)** isomer of nerolidol within a concentration range of 0.5-30 ppm were linear with $r^2$ values of 0.9998, and 0.9997, respectively.

*Terpenoid Encapsulations*

The results of terpenoids encapsulations are presented in **Table 26.** The starting mass for all terpenoids was 10 mg. The amount of encapsulated myrcene was 1.8 mg with EE = 18.1% and DL = 4.7%, possibly due to its low boiling point and volatile nature. Both β-caryophyllene and nerolidol showed higher encapsulated amounts of 6.3 (EE = 64.9% and DL = 15.1%) and 5.7 mg (EE = 55.7% and DL = 13.9%) respectively. The increase in encapsulation efficiency of β-caryophyllene and nerolidol as compared to myrcene may be due to increases in the diameters of the as the respective terpenoid-containing nanoparticle, as summarized in **Table 25.**

| **Table 26.** Analysis of terpenoids encapsulation in the prepared nanoparticles (NPs) | | | |
|---|---|---|---|
| **Formula** | **Loaded terpenoid** | | |
| | **Amount (μg)** | **EE %** | **DL %** |
| **F57: Blank NPs**<br>PLGA-PEG (40 mg) | - | - | - |
| **F58: Myrcene NPs**<br>- Myrcene (10 mg; 25% w/w)<br>- PLGA-PEG: (40 mg) | 1811.0 ± 174.7 | 18.11 ± 1.75 | 4.67 ± 0.33 |

(continued)

| Table 26. Analysis of terpenoids encapsulation in the prepared nanoparticles (NPs) | | | |
|---|---|---|---|
| **Formula** | **Loaded terpenoid** | | |
| | **Amount (μg)** | **EE %** | **DL %** |
| **F59:** β-Caryophyllene NPs<br>- β-Caryophyllene: (10 mg; 25% w/w)<br>- PLGA-PEG: (40 mg) | 6338.7 ± 251.2 | 63.94 ± 3.14 | 15.07 ± 0.57 |
| **F60: Nerolidol NPs**<br>- Nerolidol: (10 mg; 25% w/w)<br>- PLGA-PEG: (40 mg) | 5677.1 ± 314.3 | 55.67 ± 2.51 | 13.88 ± 0.86 |

**Example 9 - Calcium responses in HEK TRPV1 cells as induced by terpenoid-encapsulating PLGA nanoparticles**

This example shows the investigation of the ability of terpenoid-containing PLGA-PEG or poly(ethylene glycol) methyl ether-block-poly(lactide-co-glycolide) nanoparticles (NPs) to improve the interaction of the encapsulated species with TRPV1 receptors, as an *in vitro* assessment of their potential application in pain management.

A calcium signaling assay was utilized to evaluate the ion influx into HEK TRPV1 cells. Cells were dispersed in 1 mM calcium assay buffer and the intracellular calcium levels were measured using a plate reader in the presence of Fluo-4, as the fluorophore. Fluo-4 exhibits an increase in fluorescence upon binding of $Ca^{2+}$ ions and is used to measure intracellular $Ca^{2+}$ concentrations in living cells. The experiments conducted were carried out for 1 hour to provide sufficient time for the encapsulated terpenoids to be released from the nanoparticles. Fluorescence changes in response to the free terpenoids, encapsulated terpenoids, and combinations of free and encapsulated terpenoids were detected and compared.

*Non-encapsulated vs. encapsulated individual terpenoids*

As shown in **FIG. 17,** all non-encapsulated terpenoids induced a calcium response, with free myrcene giving the most intense response, followed by free nerolidol and free β-caryophyllene. Surprisingly, all nanoparticle formulations containing terpenoids remarkably increased the intensity of fluorescence in comparison with the non-encapsulated terpenoids in bulk solution. While both free and encapsulated β-caryophyllene displayed the lowest calcium responses as compared to the rest **(FIG. 18C),** nerolidol demonstrated a remarkable but delayed increase in its calcium response at TRPV1 when encapsulated inside the nanoparticle as compared to in bulk solution **(FIG. 18B).** Calcium responses of free and encapsulated myrcene are shown in **FIG. 18A.**

*Combination of non-encapsulated terpenoids*

Calcium responses in HEK TRPV1 cells to combinations of non-encapsulated terpenoids was also investigated. A total of 4 combinations were carried out, namely myrcene plus nerolidol **(FIG. 19A),** myrcene plus β-caryophyllene **(FIG. 19B),** nerolidol plus β-caryophyllene **(FIG. 19C),** and myrcene plus nerolidol plus β-caryophyllene **(FIG. 19D),** with a concentration of 40 μg/ml of each terpenoid. Surprising, all combinations of non-encapsulated terpenoids have improved calcium influx in comparison with the use of individual terpenoids in bulk solution.

*Combinations of different terpene-encapsulating nanoparticles*

Combinations of various populations of nanoparticles, each population of nanoparticles encapsulating a different terpenoid, were also tested and compared to treatments with a single population of nanoparticles, at a concentration of 40 μg/ml of each terpenoid. As illustrated by **FIGs. 20A-20D,** combinations of different populations of nanoparticles led to higher and improved calcium responses as compared to a single population of nanoparticles containing individual terpenoids.

*Combinations of encapsulated terpenoids as compared to combinations of non-encapsulated terpenoids*

Lastly, combinations of terpenoid-encapsulating nanoparticles were tested and compared with combinations of non-encapsulated terpenoids at a concentration of 40 μg/ml of each terpenoid. Across all the combinations tested,

namely myrcene plus nerolidol **(FIG. 21A),** myrcene plus β-caryophyllene **(FIG. 21B),** nerolidol plus β-caryophyllene **(FIG. 21C),** and myrcene plus β-caryophyllene plus nerolidol **(FIG. 21D),** combinations of terpenoid-encapsulating nanoparticles led to much improved calcium responses as compared to combinations of non-encapsulated terpenoids.

*Conclusion*

The calcium responses of HEK TRPV1 cells in the presence of free terpenoids in bulk solution, terpenoids-containing PLGA-PEG nanoparticles, and combinations of both non-encapsulated and nanoparticle encapsulated terpenoids were explored using the calcium signaling assay described herein. As demonstrated in **FIGs. 17, 18-18C, 19A-19D, 20A-20D, 21A-21D,** and described above, terpenoid combinations improved calcium responses regardless of encapsulation within nanoparticles. More importantly, whether administered individually or in combinations, terpenoid-containing nanoparticles were found to produce higher and improved calcium cellular influxes as compared to non-encapsulated terpenoids in bulk solutions. These data and results provide strong evidence that the encapsulation of terpenoids within nanoparticles significantly improves the *in vitro* pharmacological activity of said terpenoids. Furthermore, these results highlight the advantages of formulations and pharmaceutical compositions comprising combinations of terpenoids for their use in future *in vivo* studies and as potential therapeutics.

While the invention has been particularly shown and described with reference to a preferred embodiment and various alternate embodiments, it will be understood by persons skilled in the relevant art that various changes in form and details can be made therein without departing from the spirit and scope of the invention.

All references, issued patents and patent applications cited within the body of the instant specification are hereby incorporated by reference in their entirety, for all purposes.

It will be understood that when an aspect of the invention is drafted as a method of treating a disease or condition comprising administering to a subject the pharmaceutical composition of the invention, it is also intended to encompass a pharmaceutical composition of the invention for use in the treatment of said disease or condition.

## Claims

1. A terpenoid-encapsulating PLGA nanoparticle, comprising: a PLGA nanoparticle and a first terpenoid encapsulated in the PLGA nanoparticle.

2. The terpenoid-encapsulating PLGA nanoparticle of claim 1, wherein the PLGA nanoparticle comprises PLGA copolymer and the weight ratio between the first terpenoid and the PLGA copolymer is between 1:50 and 1:4.

3. The terpenoid-encapsulating PLGA nanoparticle of claim 2, wherein the weight ratio between the first terpenoid and the PLGA copolymer is between 1:25 and 1:5.

4. The terpenoid-encapsulating PLGA nanoparticle of any one of claims 1-3, wherein the first terpenoid is selected from the group consisting of myrcene, β-caryophyllene, and nerolidol.

5. The terpenoid-encapsulating PLGA nanoparticle of claim 4, wherein the first terpenoid is myrcene, and the weight ratio between the first terpenoid and the PLGA copolymer is about 1:22.

6. The terpenoid-encapsulating PLGA nanoparticle of claim 4, wherein the first terpenoid is β-caryophyllene, and the weight ratio between the first terpenoid and the PLGA copolymer is between 1:5 and 1:7, optionally wherein the nanoparticle further comprises PEG.

7. The terpenoid-encapsulating PLGA nanoparticle of claim 4, wherein the first terpenoid is nerolidol, and the weight ratio between the first terpenoid and the PLGA copolymer is between 1:5 and 1:7, optionally wherein the nanoparticle further comprises PEG.

8. The terpenoid-encapsulating PLGA nanoparticle of any one of claims 1-7, wherein the nanoparticle further encapsulates at least a second compound, wherein the second encapsulated compound is (i) a cannabinoid or (ii) a second terpenoid other than the first terpenoid.

9. The terpenoid-encapsulating PLGA nanoparticle of claim 4, wherein the second encapsulated compound is cannabigerolic acid (CBGA).

**10.** The terpenoid-encapsulating PLGA nanoparticle of claim 4, wherein the second encapsulated compound is cannabidiol (CBD).

**11.** The terpenoid-encapsulating PLGA nanoparticle of claim 4, wherein the second encapsulated compound is cannabinol (CBN).

**12.** The terpenoid-encapsulating PLGA nanoparticle of claim 4, wherein the second encapsulated compound is cannabidivarin (CBDV).

**13.** The terpenoid-encapsulating PLGA nanoparticle of claim 4, wherein the second encapsulated compound is cannabichromene (CBC).

**14.** The terpenoid-encapsulating PLGA nanoparticle of claim 4, wherein the second encapsulated compound is cannabidiolic acid (CBDA).

**15.** The terpenoid-encapsulating PLGA nanoparticle of claim 4, wherein the second encapsulated compound is cannabigerol (CBG).

**16.** The terpenoid-encapsulating PLGA nanoparticle of claim 4, wherein the second encapsulated compound is myrcene, β-caryophyllene, or nerolidol.

**17.** The terpenoid-encapsulating PLGA nanoparticle of claim 4, wherein the second encapsulated compound is limonene.

**18.** The terpenoid-encapsulating PLGA nanoparticle of claim 4, wherein the second encapsulated compound is phytol.

**19.** The terpenoid-encapsulating PLGA nanoparticle of claim 4, wherein the second encapsulated compound is pinene.

**20.** The terpenoid-encapsulating PLGA nanoparticle of claim 4, wherein the second encapsulated compound is linalool.

**21.** The terpenoid-encapsulating PLGA nanoparticle of any one of above claims, wherein the PLGA nanoparticles have an average diameter between 200-350 nm.

**22.** The terpenoid-encapsulating PLGA nanoparticle of any one of above claims, wherein the PLGA nanoparticles comprise PLGA copolymer having a ratio of lactic acid to glycolic acid between about 10-90% lactic acid and about 90-10% glycolic acid.

**23.** The terpenoid-encapsulating PLGA nanoparticle of claim 22, wherein the ratio of lactic acid to glycolic acid is about 10% lactic acid to about 90% glycolic acid.

**24.** The terpenoid-encapsulating PLGA nanoparticle of claim 22, wherein the ratio of lactic acid to glycolic acid is about 25% lactic acid to about 75% glycolic acid.

**25.** The terpenoid-encapsulating PLGA nanoparticle of claim 22, wherein the ratio of lactic acid to glycolic acid is about 50% lactic acid to about 50% glycolic acid.

**26.** The terpenoid-encapsulating PLGA nanoparticle of claim 22, wherein the ratio of lactic acid to glycolic acid is about 75% lactic acid to about 25% glycolic acid.

**27.** The terpenoid-encapsulating PLGA nanoparticle of claim 22, wherein the ratio of lactic acid to glycolic acid is about 90% lactic acid to about 10% glycolic acid.

**28.** A pharmaceutical composition comprising a first population of terpenoid-encapsulating PLGA nanoparticles according to any one of the above claims, and a pharmaceutically acceptable carrier or diluent.

**29.** The pharmaceutical composition of claim 28, further comprising trehalose.

**30.** The pharmaceutical composition of any one of claims 28-29, wherein the composition is lyophilized.

**31.** The pharmaceutical composition of any one of claims 28-30 wherein the composition further comprises a second population of PLGA nanoparticles, wherein the second population of nanoparticles encapsulates a third compound, wherein the third compound is a cannabinoid or terpenoid different from the cannabinoids and terpenoids encapsulated in the first population of nanoparticles.

**32.** The pharmaceutical composition of claim 31, wherein the third encapsulated compound is cannabigerolic acid (CBGA).

**33.** The pharmaceutical composition of claim 31, wherein the third encapsulated compound is cannabidiol (CBD).

**34.** The pharmaceutical composition of claim 31, wherein the third encapsulated compound is cannabinol (CBN).

**35.** The pharmaceutical composition of claim 31, wherein the third encapsulated compound is cannabidivarin (CBDV).

**36.** The pharmaceutical composition of claim 31, wherein the third encapsulated compound is cannabichromene (CBC).

**37.** The pharmaceutical composition of claim 31, wherein the third encapsulated compound is cannabidiolic acid (CBDA).

**38.** The pharmaceutical composition of claim 31, wherein the third encapsulated compound is cannabigerol (CBG).

**39.** The pharmaceutical composition of claim 31, wherein the third encapsulated compound is myrcene, β-caryophyllene, or nerolidol.

**40.** The pharmaceutical composition of claim 31, wherein the third encapsulated compound is limonene.

**41.** The pharmaceutical composition of claim 31, wherein the third encapsulated compound is phytol.

**42.** The pharmaceutical composition of claim 31, wherein the third encapsulated compound is pinene.

**43.** The pharmaceutical composition of claim 31, wherein the third encapsulated compound is linalool.

**44.** The pharmaceutical composition of any one of claims 31-43, wherein the composition further comprises a third population of PLGA nanoparticles, wherein the third population of nanoparticles encapsulates a fourth compound, wherein the fourth compound is a cannabinoid or terpenoid different from the cannabinoids and terpenoids encapsulated in the first population of nanoparticles or the second population of nanoparticles.

**45.** The pharmaceutical composition of claim 44, wherein the composition further comprises a fourth population of PLGA nanoparticles, wherein the fourth population of nanoparticles encapsulates a fifth compound, wherein the fifth compound is a cannabinoid or terpenoid different from the cannabinoids and terpenoids encapsulated in the first population of nanoparticles, the second population of nanoparticles or the third population of nanoparticles.

**46.** A method for obtaining terpenoid-encapsulating PLGA nanoparticles, wherein the method comprises the steps of:

(a) providing an organic solution comprising terpenoid, a PLGA copolymer, and a solvent, and an aqueous solution comprising a surfactant;
(b) mulsifying the two solutionsto form a suspension of the terpenoid-encapsulating PLGA nanoparticles;
(c) evaporating the solvent from theemulsion; and
(d) obtaining the terpenoid-encapsulating PLGA nanoparticles.

**47.** The method of claim 46, wherein the weight ratio of the first terpenoid to PLGA copolymer in the solution of step (a) is from about 1:5 to about 1:1.

**48.** The method of claim 47, wherein the weight ratio of the first terpenoid to PLGA copolymer in the solution of step (a) is about 1:5.

**49.** The method of claim 47, wherein the weight ratio of the first terpenoid to PLGA copolymer in the solution of step (a) is about 1:4.

**50.** The method of claim 47, wherein the weight ratio of the first terpenoid to PLGA copolymer in the solution of step (a) is about 1:3.

**51.** The method of claim 47, wherein the weight ratio of the first terpenoid to PLGA copolymer in the solution of step (a) is about 1:2.

**52.** The method of claim 47, wherein the weight ratio of the first terpenoid to PLGA copolymer in the solution of step (a) is about 1:1.

**53.** The method of any of one claims 46-52, wherein the entrapment efficiency of the first terpenoid in step (b) is between about 4% to about 10%.

**54.** The method of claim 53, wherein the entrapment efficiency is at least 4%.

**55.** The method of claim 53, wherein the entrapment efficiency is at least 5%.

**56.** The method of claim 53, wherein the entrapment efficiency is at least 6%.

**57.** The method of claim 53, wherein the entrapment efficiency is at least 7%.

**58.** The method of claim 53, wherein the entrapment efficiency is at least 8%.

**59.** The method of claim 53, wherein the entrapment efficiency is at least 9%.

**60.** The method of claim 53, wherein the entrapment efficiency is at least 10%.

**61.** The method of any one of claims 46-60, wherein the average weight ratio between encapsulated terpenoid and PLGA copolymer in the terpenoid-encapsulating PLGA nanoparticles of step (d) is between about 1:50 and about 1:10.

**62.** The method of claim 61, wherein the average weight ratio between encapsulated terpenoid and PLGA copolymer in the terpenoid-encapsulating PLGA nanoparticles is at least about 1:50.

**63.** The method of claim 61, wherein the average weight ratio between encapsulated terpenoid and PLGA copolymer in the terpenoid-encapsulating PLGA nanoparticles is at least about 1:40.

**64.** The method of claim 61, wherein the average weight ratio between encapsulated terpenoid and PLGA copolymer in the terpenoid-encapsulating PLGA nanoparticles is at least about 1:30.

**65.** The method of claim 61, wherein the average weight ratio between encapsulated terpenoid and PLGA copolymer in the terpenoid-encapsulating PLGA nanoparticles is at least about 1:20.

**66.** The method of claim 61, wherein the average weight ratio between encapsulated terpenoid and PLGA copolymer in the terpenoid-encapsulating PLGA nanoparticles is at least about 1:10.

**67.** The method of any one of claims 46-66, wherein the PLGA copolymer has a ratio of lactic acid to glycolic acid between about 10-90% lactic acid and about 90-10% glycolic acid.

**68.** The method of claim 67, wherein the ratio of lactic acid to glycolic acid is about 10% lactic acid to about 90% glycolic acid.

**69.** The method of claim 67, wherein the ratio of lactic acid to glycolic acid is about 25% lactic acid to about 75% glycolic acid.

**70.** The method of claim 67, wherein the ratio of lactic acid to glycolic acid is about 50% lactic acid to about 50% glycolic acid.

**71.** The method of claim 67, wherein the ratio of lactic acid to glycolic acid is about 75% lactic acid to about 25% glycolic

acid.

**72.** The method of claim 67, wherein the ratio of lactic acid to glycolic acid is about 90% lactic acid to about 10% glycolic acid.

**73.** The method of any one of claims 46-72, wherein the solution of step (a) further comprises at least one cannabinoid or terpenoid other than the first terpenoid.

**74.** The method of claim 73, wherein the at least one cannabinoid or terpenoid is selected from the group consisting of: cannabidiol (CBD), cannabinol (CBN), cannabidivarin (CBDV), cannabichromene (CBC), cannabidiolic acid (CBDA), and cannabigerol (CBG).

**75.** The method of claim 73, wherein the at least one cannabinoid or terpenoid is selected from the group consisting of: myrcene, β-caryophyllene, nerolidol, phytol, limonene, linalool, and pinene.

**76.** The method of any one of claims 46-75, wherein the solvent is acetone, dichloromethane, or ethyl acetate.

**77.** The method of any one of claims 46-76, wherein the surfactant is polyethylene glycol, a poloxamer, or polyvinyl alcohol (PVA).

**78.** The method of claim 77, wherein the surfactant is polyvinyl alcohol (PVA).

**79.** The method of any one of claims 46-78, wherein the step of emulsifying comprises homogenization or sonication.

**80.** The method of claim 79, wherein the step of emulsifying is homogenization.

**81.** The method of claim 80, wherein the homogenization is performed at 20,000 to 30,000 rpm.

**82.** The method of claim 80, wherein the homogenization is performed at 24,000 rpm.

**83.** The method of any one of claims 80-82, wherein the solution is homogenized for 30s to 10 min.

**84.** The method of claim 83, wherein the solution is homogenized for 1 min.

**85.** The method of any one of claims 46-84, wherein the step of evaporating the solvent comprises at least one of stirring the solvent, applying gas streams, applying heat, maintaining cold temperature of 10°C, or creating a vacuum.

**86.** The method of claim 85, wherein the step of evaporating the solvent comprises stirring the suspension at room temperature.

**87.** The method of claim 86, wherein the suspension is stirred for 5 min to 120 min to evaporate the solvent.

**88.** The method of claim 87, wherein the suspension is stirred for 60 min.

**89.** The method of any one of claims 46-88, wherein the step of obtaining the terpenoid-encapsulating PLGA nanoparticles comprises centrifugation, filtration, or centrifugation and filtration.

**90.** The method of claim 89, wherein the step of obtaining comprises centrifugation.

**91.** The method of claim 90, wherein the centrifugation is performed between 2,000 x g and 15,000 x g.

**92.** The method of claim 91, wherein the centrifugation is at 4,000 x g.

**93.** The method of any one of claims 46-92, further comprising the subsequent step of adding a cryoprotectant to the terpenoid-encapsulating PLGA nanoparticles.

**94.** The method of claim 93, wherein the cryoprotectant is trehalose.

**95.** The method of any one of claims 93-94, wherein the cryoprotectant is added in an amount of 1-10% (w/v) of the terpenoid-encapsulating PLGA nanoparticles.

**96.** The method of claim 95, wherein the cryoprotectant is added in an amount of 5% (w/v) of the terpenoid-encapsulating PLGA nanoparticles.

**97.** The method of any one of claims 44-96, further comprising lyophilizing the terpenoid-encapsulating PLGA nanoparticles.

**98.** A terpenoid-encapsulating PLGA nanoparticle, obtained by the method of any one of claims 46-97.

**99.** The pharmaceutical composition of any one of claims 28-45 for use in the desensitization of TRPV1 in cells of a mammalian subject, wherein said pharmaceutical composition is administered to the mammalian subject in an amount, by a route of administration, and for a time sufficient to cause TRPV1 desensitization in cells within the mammalian subject.

**100.** The pharmaceutical composition for use according to claim 99, wherein the cells are nociceptors.

**101.** The pharmaceutical composition for use according to claim 100, wherein the nociceptors are peripheral nociceptors.

**102.** The pharmaceutical composition for use according to claim 100, wherein the nociceptors are visceral nociceptors.

**103.** The pharmaceutical composition for use according to any one of claims 99-102, wherein the pharmaceutical composition is administered orally.

**104.** The pharmaceutical composition for use according to any one of claims 99-102, wherein the pharmaceutical composition is administered topically.

**105.** The pharmaceutical composition for use according to any one of claims 99-102, wherein the pharmaceutical composition is administered systemically.

**106.** The pharmaceutical composition for use according to any one of claims 99-102, wherein the pharmaceutical composition is administered intravenously.

**107.** The pharmaceutical composition for use according to any one of claims 99-102, wherein the pharmaceutical composition is administered subcutaneously.

**108.** The pharmaceutical composition for use according to any one of claims 99-102, wherein the pharmaceutical composition is administered by inhalation.

**109.** The pharmaceutical composition of any one of claims 28-45 for use in the treatment of pain in a mammalian subject, wherein said pharmaceutical composition is administered to the subject in an amount, by a route of administration, and for a time sufficient to cause TRPV1 desensitization in nociceptors within the subject.

**110.** The pharmaceutical composition for use according to claim 109, wherein the nociceptors are peripheral nociceptors, and the pharmaceutical composition is administered topically.

**111.** The pharmaceutical composition for use according to claim 109, wherein the nociceptors are visceral nociceptors, and the pharmaceutical composition is administered systemically.

**112.** The pharmaceutical composition for use according to any one of claims 109-111, wherein the pain is neuropathic pain.

**113.** The pharmaceutical composition for use according to claim 112, wherein the neuropathic pain is diabetic peripheral neuropathic pain.

**114.** The pharmaceutical composition for use according to any one of claims 109-111, wherein the pain is post-herpetic neuralgia.

**115.** The pharmaceutical composition for use according to any one of claims 109-114, wherein the pharmaceutical composition is administered at least once a day for at least 3 days.

**116.** The pharmaceutical composition for use according to claim 115, wherein the pharmaceutical composition is administered at least once a day for at least 5 days.

**117.** The pharmaceutical composition for use according to claim 115, wherein the pharmaceutical composition is administered at least once a day for at least 7 days.

**118.** The pharmaceutical composition for use according to any one of claims 109-117, wherein the pharmaceutical composition is administered at a dose, by a route of administration, and on a schedule sufficient to maintain levels of the first terpenoid at the nociceptors for at least 3 days that are effective to desensitize TRPV1 in nociceptors.

**119.** The pharmaceutical composition for use according to claim 118, wherein the pharmaceutical composition is administered at a dose, by a route of administration, and on a schedule sufficient to maintain levels of the first terpenoid at the nociceptors for at least 5 days that are effective to desensitize TRPV1 in nociceptors.

**120.** The pharmaceutical composition for use according to of claim 118, wherein the pharmaceutical composition is administered at a dose, by a route of administration, and on a schedule sufficient to maintain levels of the first terpenoid at the nociceptors for at least 7 days that are effective to desensitize TRPV1 in nociceptors.

**121.** The pharmaceutical composition of any one of claims 28-45 for use in the treatment of cardiac hypertrophy in a mammalian subject, wherein an anti-hypertrophic effective amount of said pharmaceutical composition is administered to the subject.

**122.** The pharmaceutical composition for use according to claim 121, wherein the pharmaceutical composition is administered orally.

**123.** The pharmaceutical composition for use according to claim 121, wherein the pharmaceutical composition is administered systemically.

**124.** The pharmaceutical composition for use according to claim 121, wherein the pharmaceutical composition is administered intravenously.

**125.** The pharmaceutical composition for use according to claim 121, wherein the pharmaceutical composition is administered subcutaneously.

**126.** The pharmaceutical composition for use according to claim 121, wherein the pharmaceutical composition is administered by inhalation.

**127.** The pharmaceutical composition for use according to claim 121, wherein the pharmaceutical composition is administered orally.

**128.** The pharmaceutical composition of any one of claims 28-45 for use in the prophylactic treatment of cardiac hypertrophy in a mammalian subject, wherein an anti-hypertrophic effective amount of said pharmaceutical composition is administered to a subject at risk of cardiac hypertrophy.

**129.** The pharmaceutical composition of any one of claims 28-45 for use in the treatment of overactive bladder in a mammalian subject, wherein a therapeutically effective amount of said pharmaceutical composition is administered to the subject.

**130.** The pharmaceutical composition for use according to claim 129, wherein the pharmaceutical composition is administered systemically.

**131.** The pharmaceutical composition for use according to claim 129, wherein the pharmaceutical composition is administered by bladder irrigation.

**132.** The pharmaceutical composition of any one of claims 28-45 for use in the treatment of refractory chronic cough in

a mammalian subject, wherein a therapeutically effective amount of said pharmaceutical composition is administered to the subject.

**133.** The pharmaceutical composition for use according to claim 132, wherein the pharmaceutical composition is administered systemically.

**134.** The pharmaceutical composition for use according to claim 132, wherein the pharmaceutical composition is administered by inhalation.

**135.** A cannabinoid-encapsulating PLGA nanoparticle, comprising: a PLGA nanoparticle and a first cannabinoid encapsulated in the PLGA nanoparticle.

**136.** The cannabinoid-encapsulating PLGA nanoparticle of claim 135, wherein the PLGA nanoparticle comprises PLGA copolymer and the weight ratio between the first cannabinoid and the PLGA copolymer is between 1:50 and 1:4.

**137.** The cannabinoid-encapsulating PLGA nanoparticle of claim 136, wherein the weight ratio between the first cannabinoid and the PLGA copolymer is between 1:25 and 1:5.

**138.** The cannabinoid-encapsulating PLGA nanoparticle of any one of claims 135-137, wherein the first cannabinoid is selected from the group consisting of cannabidiol, cannabidivarin, cannabinol, cannabigerol, and cannabichromene.

**139.** The cannabinoid-encapsulating PLGA nanoparticle of claim 138, wherein the first cannabinoid is cannabidiol, and the weight ratio between the first cannabinoid and the PLGA copolymer is about 1:14.

**140.** The cannabinoid-encapsulating PLGA nanoparticle of claim 138, wherein the first cannabinoid is cannabidiol, and the weight ratio between the first cannabinoid and the PLGA copolymer is between 1:5 and 1:7.

**141.** The cannabinoid-encapsulating PLGA nanoparticle of claim 138, wherein the first cannabinoid is cannabidiol, and the weight ratio between the first cannabinoid and the PLGA copolymer is between 1:5 and 1:7.

**142.** The cannabinoid-encapsulating PLGA nanoparticle of any one of claims 135-141, wherein the nanoparticle further encapsulates at least a second compound, wherein the second encapsulated compound is (i) a terpenoid or (ii) a second cannabinoid other than the first cannabinoid.

**143.** The cannabinoid-encapsulating PLGA nanoparticle of claim 142, wherein the second encapsulated compound is cannabigerolic acid (CBGA).

**144.** The cannabinoid-encapsulating PLGA nanoparticle of claim 142, wherein the second encapsulated compound is cannabidivarin (CBV).

**145.** The cannabinoid-encapsulating PLGA nanoparticle of claim 142, wherein the second encapsulated compound is cannabinol (CBN).

**146.** The cannabinoid-encapsulating PLGA nanoparticle of claim 142, wherein the second encapsulated compound is cannabidivarin (CBDV).

**147.** The cannabinoid-encapsulating PLGA nanoparticle of claim 142, wherein the second encapsulated compound is cannabichromene (CBC).

**148.** The cannabinoid-encapsulating PLGA nanoparticle of claim 142, wherein the second encapsulated compound is cannabidiolic acid (CBDA).

**149.** The cannabinoid-encapsulating PLGA nanoparticle of claim 142, wherein the second encapsulated compound is cannabigerol (CBG).

**150.** The cannabinoid-encapsulating PLGA nanoparticle of claim 142, wherein the second encapsulated compound is myrcene, β-caryophyllene, or nerolidol.

**151.** The cannabinoid-encapsulating PLGA nanoparticle of claim 142, wherein the second encapsulated compound is limonene.

**152.** The cannabinoid-encapsulating PLGA nanoparticle of claim 142, wherein the second encapsulated compound is phytol.

**153.** The cannabinoid-encapsulating PLGA nanoparticle of claim 142, wherein the second encapsulated compound is pinene.

**154.** The cannabinoid-encapsulating PLGA nanoparticle of claim 142, wherein the second encapsulated compound is linalool.

**155.** The cannabinoid-encapsulating PLGA nanoparticle of claim 142, wherein the second encapsulated compound is myrcene.

**156.** The cannabinoid-encapsulating PLGA nanoparticle of any one of claims 135-155, wherein the PLGA nanoparticles have an average diameter between 200-350 nm.

**157.** The cannabinoid-encapsulating PLGA nanoparticle of any one of claims 135-156, wherein the PLGA nanoparticles comprise PLGA copolymer having a ratio of lactic acid to glycolic acid between about 10-90% lactic acid and about 90-10% glycolic acid.

**158.** The cannabinoid-encapsulating PLGA nanoparticle of claim 157, wherein the ratio of lactic acid to glycolic acid is about 10% lactic acid to about 90% glycolic acid.

**159.** The cannabinoid-encapsulating PLGA nanoparticle of claim 157, wherein the ratio of lactic acid to glycolic acid is about 25% lactic acid to about 75% glycolic acid.

**160.** The cannabinoid-encapsulating PLGA nanoparticle of claim 157, wherein the ratio of lactic acid to glycolic acid is about 50% lactic acid to about 50% glycolic acid.

**161.** The cannabinoid-encapsulating PLGA nanoparticle of claim 157, wherein the ratio of lactic acid to glycolic acid is about 75% lactic acid to about 25% glycolic acid.

**162.** The cannabinoid-encapsulating PLGA nanoparticle of claim 157, wherein the ratio of lactic acid to glycolic acid is about 90% lactic acid to about 10% glycolic acid.

**163.** A pharmaceutical composition comprising a first population of cannabinoid-encapsulating PLGA nanoparticles according to any one of claims 135-162, and a pharmaceutically acceptable carrier or diluent.

**164.** The pharmaceutical composition of claim 163, further comprising trehalose.

**165.** The pharmaceutical composition of any one of claims 163-164, wherein the composition is lyophilized.

**166.** The pharmaceutical composition of any one of claims 163-165, wherein the composition further comprises a second population of PLGA nanoparticles, wherein the second population of nanoparticles encapsulates a third compound, wherein the third compound is a terpenoid or a cannabinoid different from the terpenoids and cannabinoids encapsulated in the first population of nanoparticles.

**167.** The pharmaceutical composition of claim 166, wherein the third encapsulated compound is cannabigerolic acid (CBGA).

**168.** The pharmaceutical composition of claim 166, wherein the third encapsulated compound is cannabidiol (CBD).

**169.** The pharmaceutical composition of claim 166, wherein the third encapsulated compound is cannabinol (CBN).

**170.** The pharmaceutical composition of claim 166, wherein the third encapsulated compound is cannabidivarin (CBDV).

**171.** The pharmaceutical composition of claim 166, wherein the third encapsulated compound is cannabichromene (CBC).

**172.** The pharmaceutical composition of claim 166, wherein the third encapsulated compound is cannabidiolic acid (CBDA).

**173.** The pharmaceutical composition of claim 166, wherein the third encapsulated compound is cannabigerol (CBG).

**174.** The pharmaceutical composition of claim 166, wherein the third encapsulated compound is myrcene, β-caryophyllene, or nerolidol.

**175.** The pharmaceutical composition of claim 166, wherein the third encapsulated compound is limonene.

**176.** The pharmaceutical composition of claim 166, wherein the third encapsulated compound is phytol.

**177.** The pharmaceutical composition of claim 166, wherein the third encapsulated compound is pinene.

**178.** The pharmaceutical composition of claim 166, wherein the third encapsulated compound is linalool.

**179.** The pharmaceutical composition of claim 166, wherein the third encapsulated compound is myrcene.

**180.** The pharmaceutical composition of any one of claims 163-179, wherein the composition further comprises a third population of PLGA nanoparticles, wherein the third population of nanoparticles encapsulates a fourth compound, wherein the fourth compound is a cannabinoid or a terpenoid different from the cannabinoids and terpenoids encapsulated in the first population of nanoparticles or the second population of nanoparticles.

**181.** The pharmaceutical composition of claim 180, wherein the composition further comprises a fourth population of PLGA nanoparticles, wherein the fourth population of nanoparticles encapsulates a fifth compound, wherein the fifth compound is a cannabinoid or a terpenoid different from the cannabinoids and terpenoids encapsulated in the first population of nanoparticles, the second population of nanoparticles or the third population of nanoparticles

**182.** A method for obtaining cannabinoid-encapsulating PLGA nanoparticles, wherein the method comprises the steps of:

(a) providing an organic solution comprising a first cannabinoid, a PLGA copolymer, and a solvent, and an aqueous solution comprising a surfactant;
(b) emulsifying the two solutions to form a suspension of the cannabinoid-encapsulating PLGA nanoparticles;
(c) evaporating the solvent from the emulsion; and
(d) obtaining the cannabinoid-encapsulating PLGA nanoparticles.

**183.** The method of claim 182, wherein the weight ratio of the first cannabinoid to PLGA copolymer in the solution of step (a) is from about 1:5 to about 1:1.

**184.** The method of claim 182, wherein the weight ratio of the first cannabinoid to PLGA copolymer in the solution of step (a) is about 1:5.

**185.** The method of claim 182, wherein the weight ratio of the first cannabinoid to PLGA copolymer in the solution of step (a) is about 1:4.

**186.** The method of claim 182, wherein the weight ratio of the first cannabinoid to PLGA copolymer in the solution of step (a) is about 1:3.

**187.** The method of claim 182, wherein the weight ratio of the first cannabinoid to PLGA copolymer in the solution of step (a) is about 1:2.

**188.** The method of claim 182, wherein the weight ratio of the first cannabinoid to PLGA copolymer in the solution of step (a) is about 1:1.

**189.** The method of any of one claims 182-188, wherein the entrapment efficiency of the first cannabinoid in step (b) is between about 4% to about 10%.

**190.** The method of claim 189, wherein the entrapment efficiency is at least 4%.

**191.** The method of claim 189, wherein the entrapment efficiency is at least 5%.

**192.** The method of claim 189, wherein the entrapment efficiency is at least 6%.

**193.** The method of claim 189, wherein the entrapment efficiency is at least 7%.

**194.** The method of claim 189, wherein the entrapment efficiency is at least 8%.

**195.** The method of claim 189, wherein the entrapment efficiency is at least 9%.

**196.** The method of claim 189, wherein the entrapment efficiency is at least 10%.

**197.** The method of any one of claims 182-196, wherein the average weight ratio between encapsulated cannabinoid and PLGA copolymer in the cannabinoid-encapsulating PLGA nanoparticles of step (d) is between about 1:50 and about 1:10.

**198.** The method of claim 197, wherein the average weight ratio between encapsulated cannabinoid and PLGA copolymer in the cannabinoid-encapsulating PLGA nanoparticles is at least about 1:50.

**199.** The method of claim 197, wherein the average weight ratio between encapsulated cannabinoid and PLGA copolymer in the cannabinoid-encapsulating PLGA nanoparticles is at least about 1:40.

**200.** The method of claim 197, wherein the average weight ratio between encapsulated cannabinoid and PLGA copolymer in the cannabinoid-encapsulating PLGA nanoparticles is at least about 1:30.

**201.** The method of claim 197, wherein the average weight ratio between encapsulated cannabinoid and PLGA copolymer in the cannabinoid-encapsulating PLGA nanoparticles is at least about 1:20.

**202.** The method of claim 197, wherein the average weight ratio between encapsulated cannabinoid and PLGA copolymer in the cannabinoid-encapsulating PLGA nanoparticles is at least about 1:10.

**203.** The method of any one of claims 182-202, wherein the PLGA copolymer has a ratio of lactic acid to glycolic acid between about 10-90% lactic acid and about 90-10% glycolic acid.

**204.** The method of claim 203, wherein the ratio of lactic acid to glycolic acid is about 10% lactic acid to about 90% glycolic acid.

**205.** The method of claim 203, wherein the ratio of lactic acid to glycolic acid is about 25% lactic acid to about 75% glycolic acid.

**206.** The method of claim 203, wherein the ratio of lactic acid to glycolic acid is about 50% lactic acid to about 50% glycolic acid.

**207.** The method of claim 203, wherein the ratio of lactic acid to glycolic acid is about 75% lactic acid to about 25% glycolic acid.

**208.** The method of claim 203, wherein the ratio of lactic acid to glycolic acid is about 90% lactic acid to about 10% glycolic acid.

**209.** The method of any one of claims 182-208, wherein the solution of step (a) further comprises at least one cannabinoid or terpenoid other than the first cannabinoid.

**210.** The method of claim 209, wherein the at least one cannabinoid or terpenoid is selected from the group consisting of: cannabinol (CBN), cannabidivarin (CBDV), cannabichromene (CBC), cannabidiolic acid (CBDA), and cannabigerol (CBG).

**211.** The method of any one of claims 208-209, wherein the at least one cannabinoid or terpenoid is selected from the group consisting of: myrcene, β-caryophyllene, nerolidol, phytol, limonene, linalool, and pinene.

**212.** The method of any one of claims 182-211, wherein the solvent is acetone, dichloromethane, or ethyl acetate.

**213.** The method of any one of claims 182-212, wherein the surfactant is polyethylene glycol, a poloxamer, or polyvinyl alcohol (PVA).

**214.** The method of claim 213, wherein the surfactant is polyvinyl alcohol (PVA).

**215.** The method of any one of claims 182-214, wherein the step of emulsifying comprises homogenization or sonication.

**216.** The method of claim 215, wherein the step of emulsifying is homogenization.

**217.** The method of claim 216, wherein the homogenization is performed at 20,000 to 30,000 rpm.

**218.** The method of claim 217, wherein the homogenization is performed at 24,000 rpm.

**219.** The method of any one of claims 216-218, wherein the solution is homogenized for 30 seconds to 10 min.

**220.** The method of claim 219, wherein the solution is homogenized for 1 min.

**221.** The method of any one of claims 182-220, wherein the step of evaporating the solvent comprises at least one of stirring the solvent, applying gas streams, applying heat, maintaining cold temperature of 10°C, or creating a vacuum.

**222.** The method of claim 221, wherein the step of evaporating the solvent comprises stirring the suspension at room temperature.

**223.** The method of claim 222, wherein the suspension is stirred for 5 min to 120 min to evaporate the solvent.

**224.** The method of claim 223, wherein the suspension is stirred for 60 min.

**225.** The method of any one of claims 182-224, wherein the step of obtaining the cannabinoid-encapsulating PLGA nanoparticles comprises centrifugation, filtration, or centrifugation and filtration.

**226.** The method of claim 225, wherein the step of obtaining comprises centrifugation.

**227.** The method of claim 226, wherein the centrifugation is performed between 2,000 x g and 15,000 x g.

**228.** The method of claim 224, wherein the centrifugation is at 4,000 x g.

**229.** The method of any one of claims 182-228, further comprising the subsequent step of adding a cryoprotectant to the cannabinoid-encapsulating PLGA nanoparticles.

**230.** The method of claim 229, wherein the cryoprotectant is trehalose.

**231.** The method of any one of claims 229-230, wherein the cryoprotectant is added in an amount of 1-10% (w/v) of the cannabinoid-encapsulating PLGA nanoparticles.

**232.** The method of claim 231, wherein the cryoprotectant is added in an amount of 5% (w/v) of the cannabinoid-encapsulating PLGA nanoparticles.

**233.** The method of any one of claims 182-232, further comprising lyophilizing the cannabinoid-encapsulating PLGA nanoparticles.

**234.** A cannabinoid-encapsulating PLGA nanoparticle, obtained by the method of any one of claims 182-233.

**235.** The pharmaceutical composition of any one of claims 163-181 for use in the desensitization of TRPV1 in cells of a

mammalian subject, wherein said pharmaceutical composition is administered to the mammalian subject in an amount, by a route of administration, and for a time sufficient to cause TRPV1 desensitization in cells within the mammalian subject.

**236.** The pharmaceutical composition for use according to claim 235, wherein the cells are nociceptors.

**237.** The pharmaceutical composition for use according to claim 236, wherein the nociceptors are peripheral nociceptors.

**238.** The pharmaceutical composition for use according to claim 236, wherein the nociceptors are visceral nociceptors.

**239.** The pharmaceutical composition for use according to any one of claims 235-238, wherein the pharmaceutical composition is administered orally.

**240.** The pharmaceutical composition for use according to any one of claims 235-238, wherein the pharmaceutical composition is administered topically.

**241.** The pharmaceutical composition for use according to any one of claims 235-238, wherein the pharmaceutical composition is administered systemically.

**242.** The pharmaceutical composition for use according to any one of claims 235-238, wherein the pharmaceutical composition is administered intravenously.

**243.** The pharmaceutical composition for use according to any one of claims 235-238, wherein the pharmaceutical composition is administered subcutaneously.

**244.** The pharmaceutical composition for use according to any one of claims 235-238, wherein the pharmaceutical composition is administered by inhalation.

**245.** The pharmaceutical composition of any one of claims 163-181 for use in the treatment of pain in a mammalian subject, wherein said pharmaceutical composition is administered to the subject in an amount, by a route of administration, and for a time sufficient to cause TRPV1 desensitization in nociceptors within the subject.

**246.** The pharmaceutical composition for use according to claim 245, wherein the nociceptors are peripheral nociceptors, and the pharmaceutical composition is administered topically.

**247.** The pharmaceutical composition for use according to claim 245, wherein the nociceptors are visceral nociceptors, and the pharmaceutical composition is administered systemically.

**248.** The pharmaceutical composition for use according to any one of claims 245-247, wherein the pain is neuropathic pain.

**249.** The pharmaceutical composition for use according to claim 248, wherein the neuropathic pain is diabetic peripheral neuropathic pain.

**250.** The pharmaceutical composition for use according to any one of claims 245-247, wherein the pain is post-herpetic neuralgia.

**251.** The pharmaceutical composition for use according to any one of claims 245-250, wherein the pharmaceutical composition is administered at least once a day for at least 3 days.

**252.** The pharmaceutical composition for use according to claim 251, wherein the pharmaceutical composition is administered at least once a day for at least 5 days.

**253.** The pharmaceutical composition for use according to claim 252, wherein the pharmaceutical composition is administered at least once a day for at least 7 days.

**254.** The pharmaceutical composition for use according to claim 253, wherein the pharmaceutical composition is administered at least once a day for more than 7 days.

**255.** The pharmaceutical composition for use according to any one of claims 245-254, wherein the pharmaceutical composition is administered at a dose, by a route of administration, and on a schedule sufficient to maintain levels of cannabinoid at the nociceptors for at least 3 days that are effective to desensitize TRPV1 in nociceptors.

**256.** The pharmaceutical composition for use according to claim 255, wherein the pharmaceutical composition is administered at a dose, by a route of administration, and on a schedule sufficient to maintain levels of cannabinoid at the nociceptors for at least 5 days that are effective to desensitize TRPV1 in nociceptors.

**257.** The pharmaceutical composition for use according to claim 256, wherein the pharmaceutical composition is administered at a dose, by a route of administration, and on a schedule sufficient to maintain levels of cannabinoid at the nociceptors for at least 7 days that are effective to desensitize TRPV1 in nociceptors.

**258.** The pharmaceutical composition of any one of claims 163-181 for use in the treatment of cardiac hypertrophy in a mammalian subject, wherein an anti-hypertrophic effective amount of said pharmaceutical composition is administered to the subject.

**259.** The pharmaceutical composition for use according to claim 258, wherein the pharmaceutical composition is administered orally.

**260.** The pharmaceutical composition for use according to claim 258, wherein the pharmaceutical composition is administered systemically.

**261.** The pharmaceutical composition for use according to claim 258, wherein the pharmaceutical composition is administered intravenously.

**262.** The pharmaceutical composition for use according to claim 258, wherein the pharmaceutical composition is administered subcutaneously.

**263.** The pharmaceutical composition for use according to claim 258, wherein the pharmaceutical composition is administered by inhalation.

**264.** The pharmaceutical composition for use according to claim 258, wherein the pharmaceutical composition is administered orally.

**265.** The pharmaceutical composition of any one of claims 163-181 for use in the prophylactic treatment of cardiac hypertrophy in a mammalian subject, wherein an anti-hypertrophic effective amount of said pharmaceutical composition is administered to a subject at risk of cardiac hypertrophy.

**266.** The pharmaceutical composition of any one of claims 163-181 for use in the treatment of overactive bladder in a mammalian subject, wherein a therapeutically effective amount of said pharmaceutical composition is administered to the subject.

**267.** The pharmaceutical composition for use according to claim 266, wherein the pharmaceutical composition is administered systemically.

**268.** The pharmaceutical composition for use according to claim 266, wherein the pharmaceutical composition is administered by bladder irrigation.

**269.** The pharmaceutical composition of any one of claims 163-181 for use in the treatment of refractory chronic cough in a mammalian subject, wherein a therapeutically effective amount of said pharmaceutical composition is administered to the subject.

**270.** The pharmaceutical composition for use according to claim 269, wherein the pharmaceutical composition is administered systemically.

**271.** The pharmaceutical composition for use according to claim 269, wherein the pharmaceutical composition is administered by inhalation.

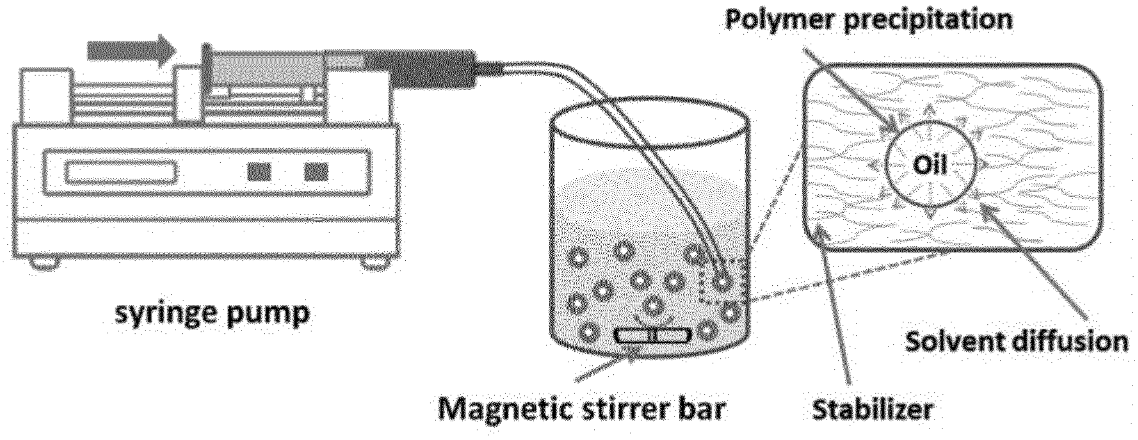


FIG. 1

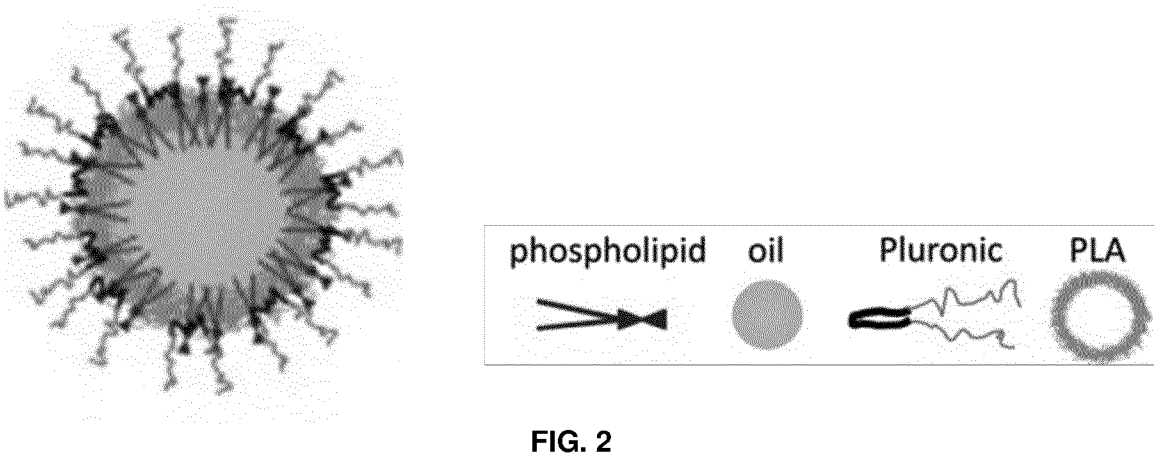


FIG. 2

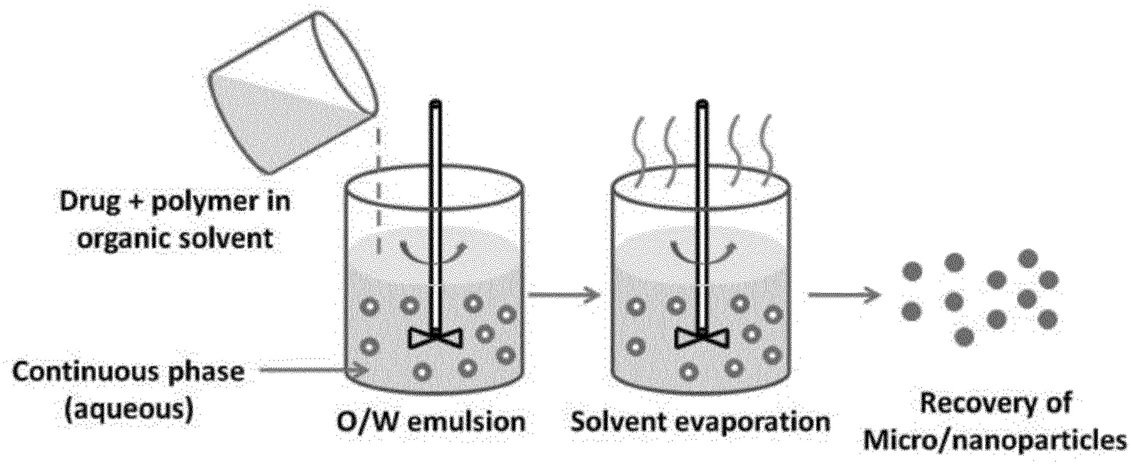


FIG. 3

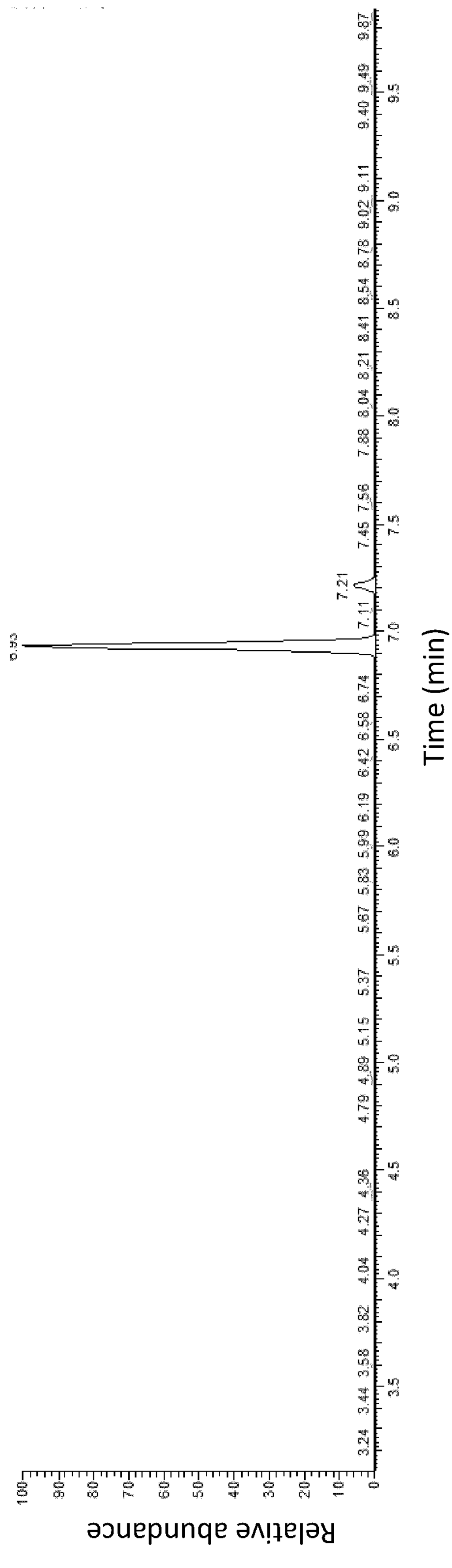
Relative abundance
0
10
20
30
40
50
60
70
80
90
100
Time (min)
3.5
4.0
4.5
5.0
5.5
6.0
6.5
7.0
7.5
8.0
8.5
9.0
9.5
3.24 3.44 3.58 3.82 4.04 4.27 4.36 4.79 4.89 5.15 5.37 5.67 5.83 5.99 6.19 6.42 6.58 6.74 7.11 7.21 7.45 7.56 7.88 8.04 8.21 8.41 8.54 8.78 9.02 9.11 9.40 9.49 9.87

**FIG. 4A**

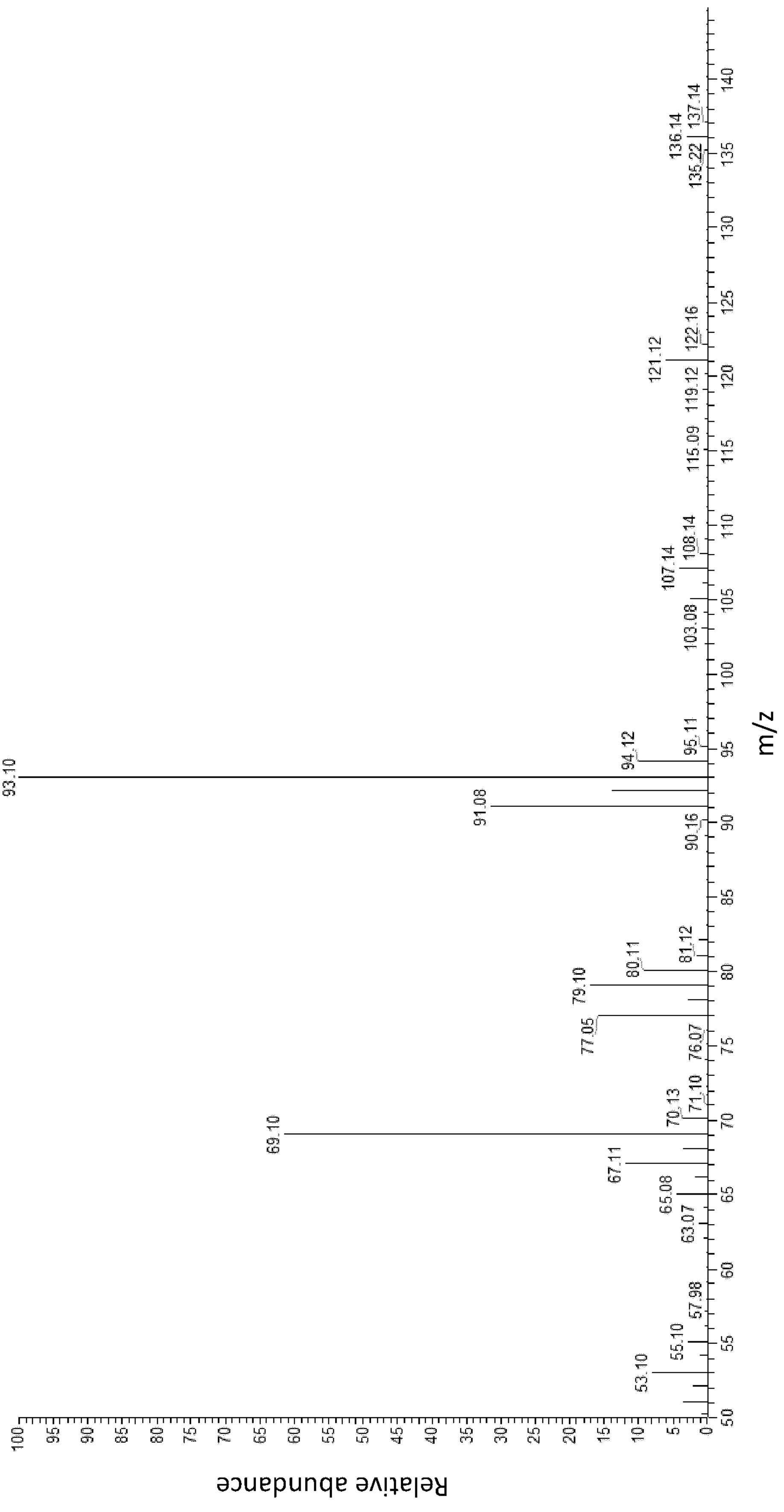
Relative abundance
m/z
93.10
91.08
69.10
67.11
65.08
63.07
57.98
55.10
53.10
70.13
71.10
76.07
77.05
79.10
80.11
81.12
90.16
94.12
95.11
103.08
107.14
108.14
115.09
119.12
121.12
122.16
135.22
136.14
137.14

FIG. 4B

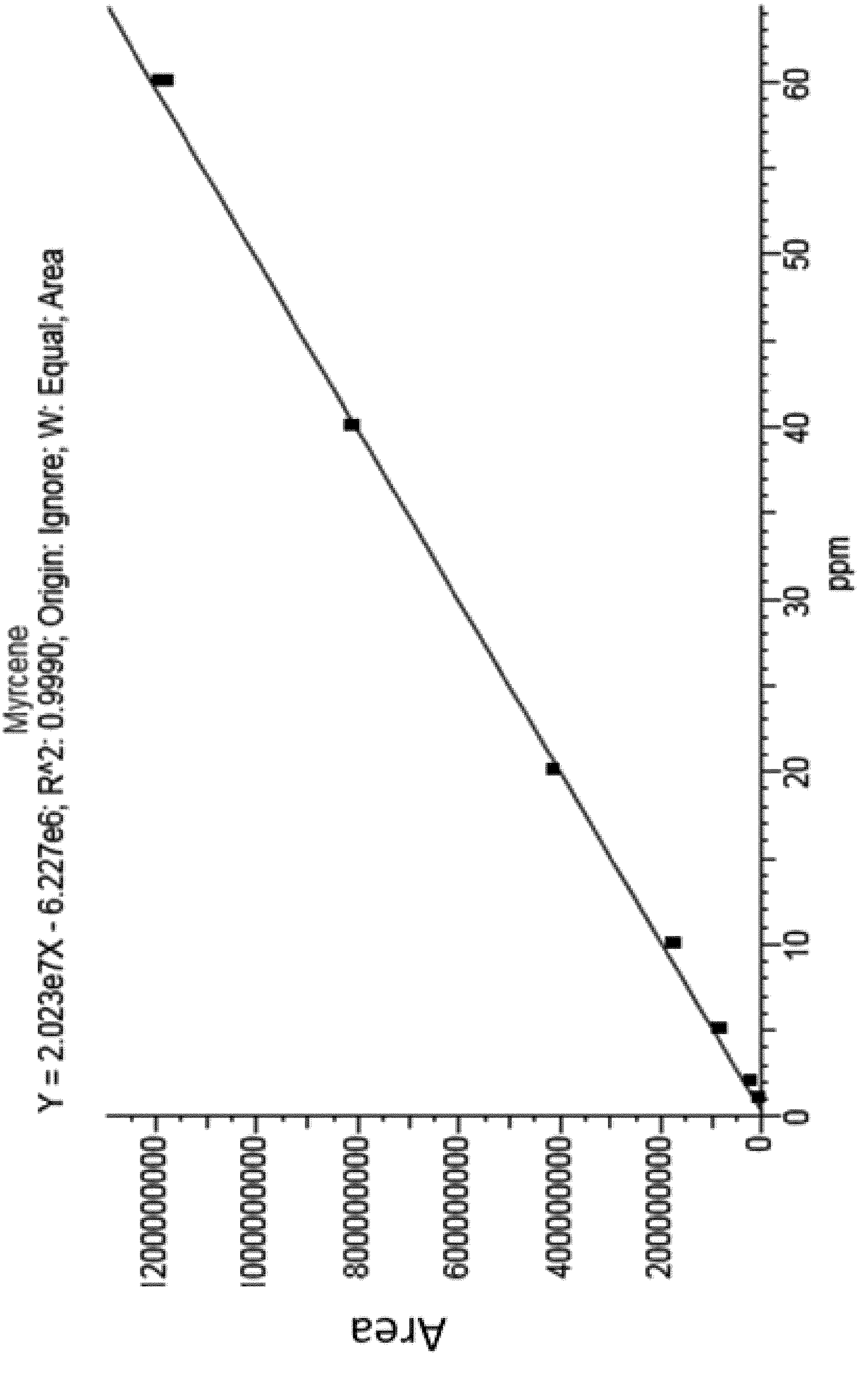
Myrcene
Y = 2.023e7X - 6.227e6; R^2: 0.9990; Origin: Ignore; W: Equal; Area
Area
1200000000
1000000000
800000000
600000000
400000000
200000000
0
0
10
20
30
40
50
60
ppm

FIG. 4C

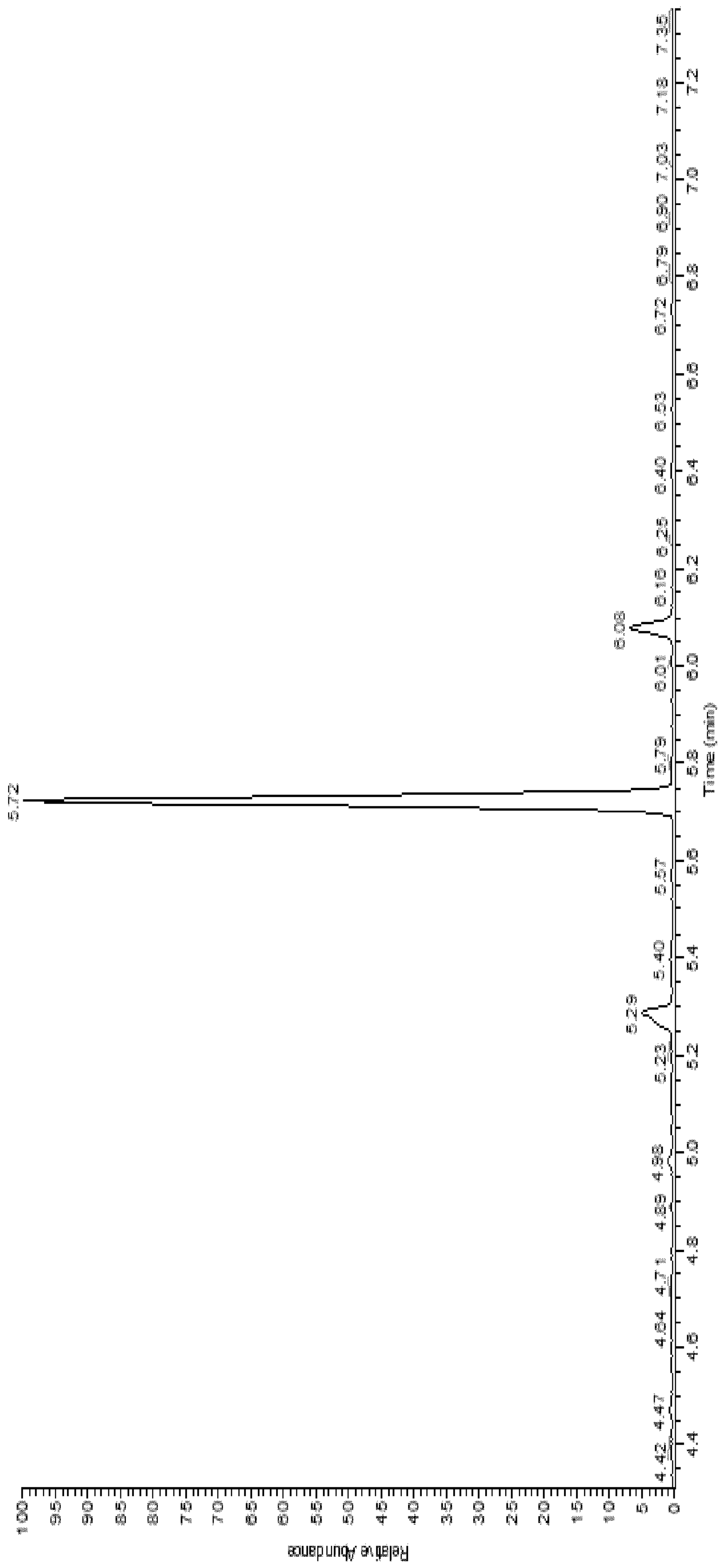
5.72
5.29
6.08
Relative Abundance
Time (min)
4.42 4.47 4.64 4.71 4.89 4.98 5.23 5.40 5.57 5.79 6.01 6.18 6.25 6.40 6.53 6.72 6.79 6.90 7.03 7.18 7.35
4.4 4.6 4.8 5.0 5.2 5.4 5.6 5.8 6.0 6.2 6.4 6.6 6.8 7.0 7.2
0 5 10 15 20 25 30 35 40 45 50 55 60 65 70 75 80 85 90 95 100

FIG. 5A

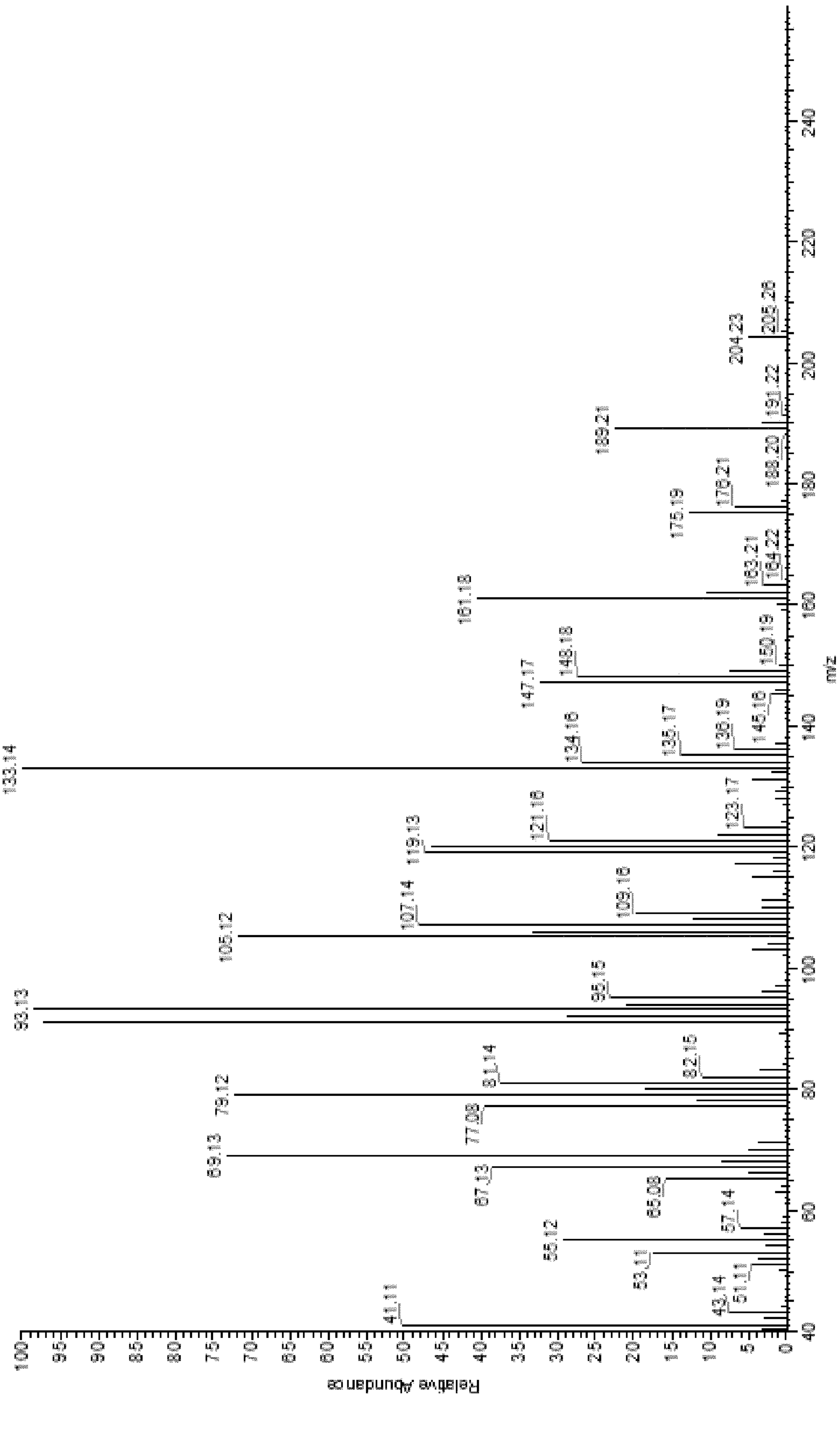
Relative Abundance
m/z
41.11
43.14
51.11
53.11
55.12
57.14
65.08
67.13
69.13
77.08
79.12
81.14
82.15
93.13
95.15
105.12
107.14
109.16
119.13
121.18
123.17
133.14
134.16
135.17
136.19
145.16
147.17
148.18
150.19
161.18
163.21
164.22
175.19
176.21
188.20
189.21
191.22
204.23
205.28

FIG. 5B

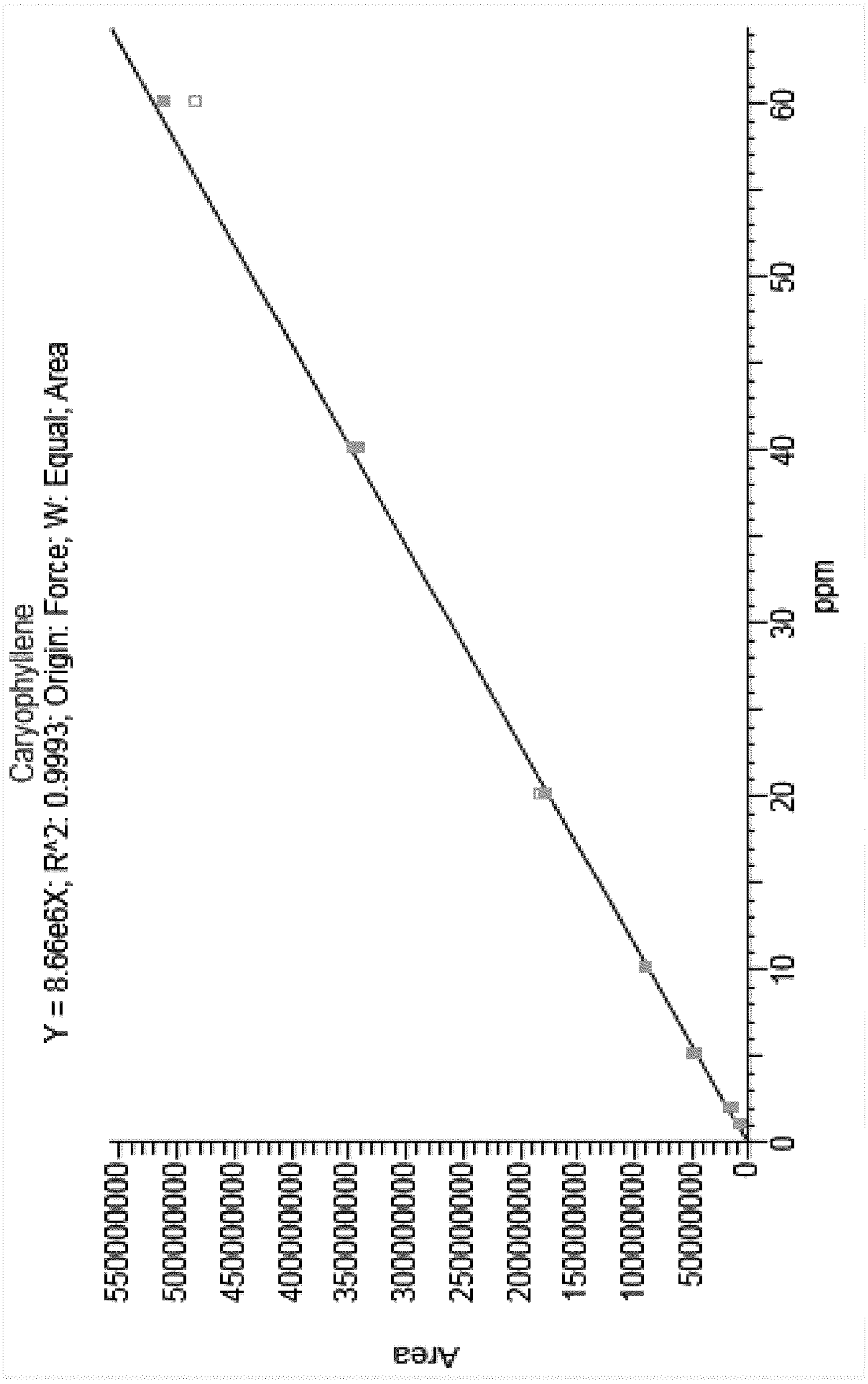
Caryophyllene
Y = 8.66e6X; R^2: 0.9993; Origin: Force; W: Equal; Area
Area
550000000
500000000
450000000
400000000
350000000
300000000
250000000
200000000
150000000
100000000
50000000
0
0
10
20
30
40
50
60
ppm

FIG. 5C

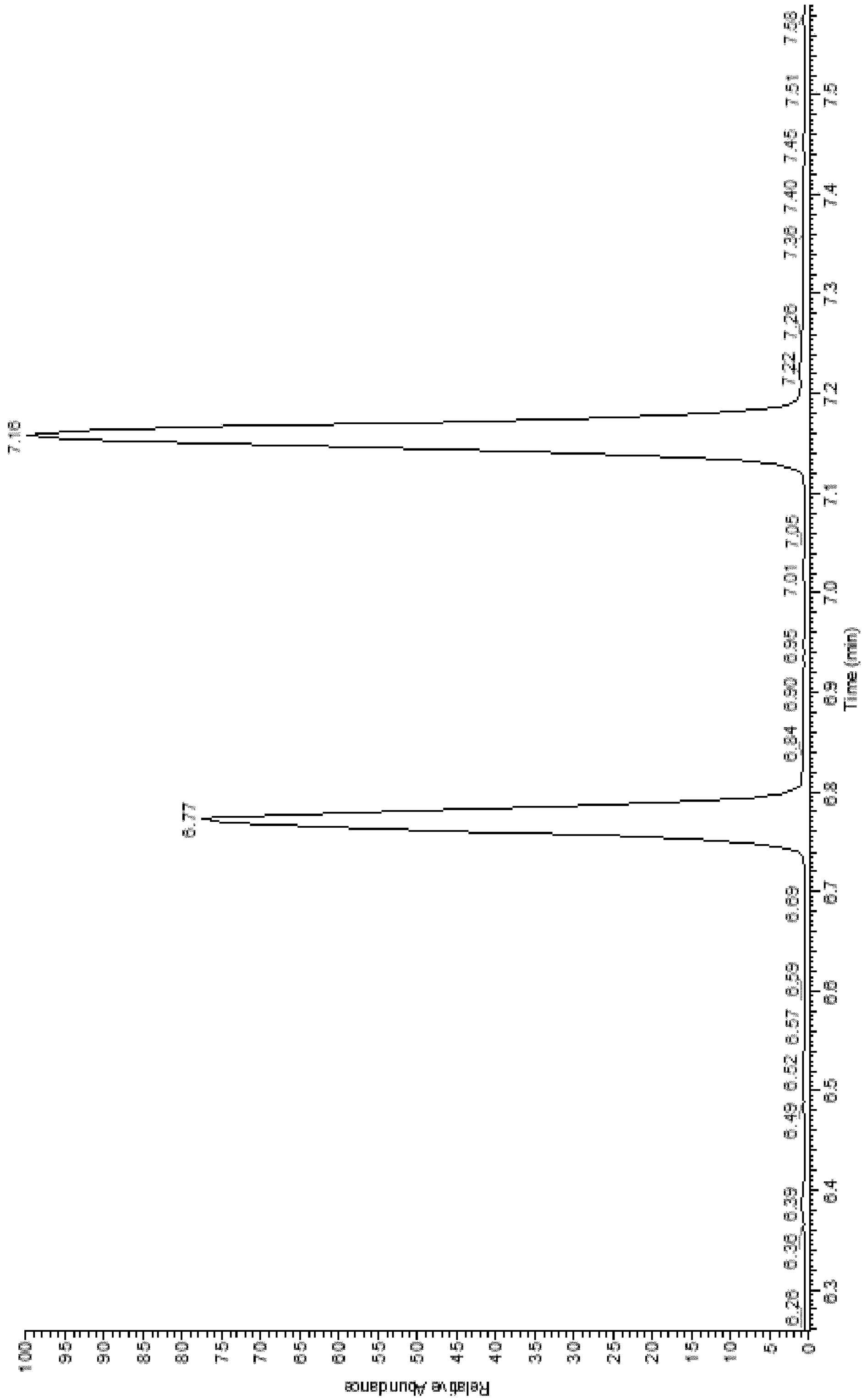


**FIG. 6**

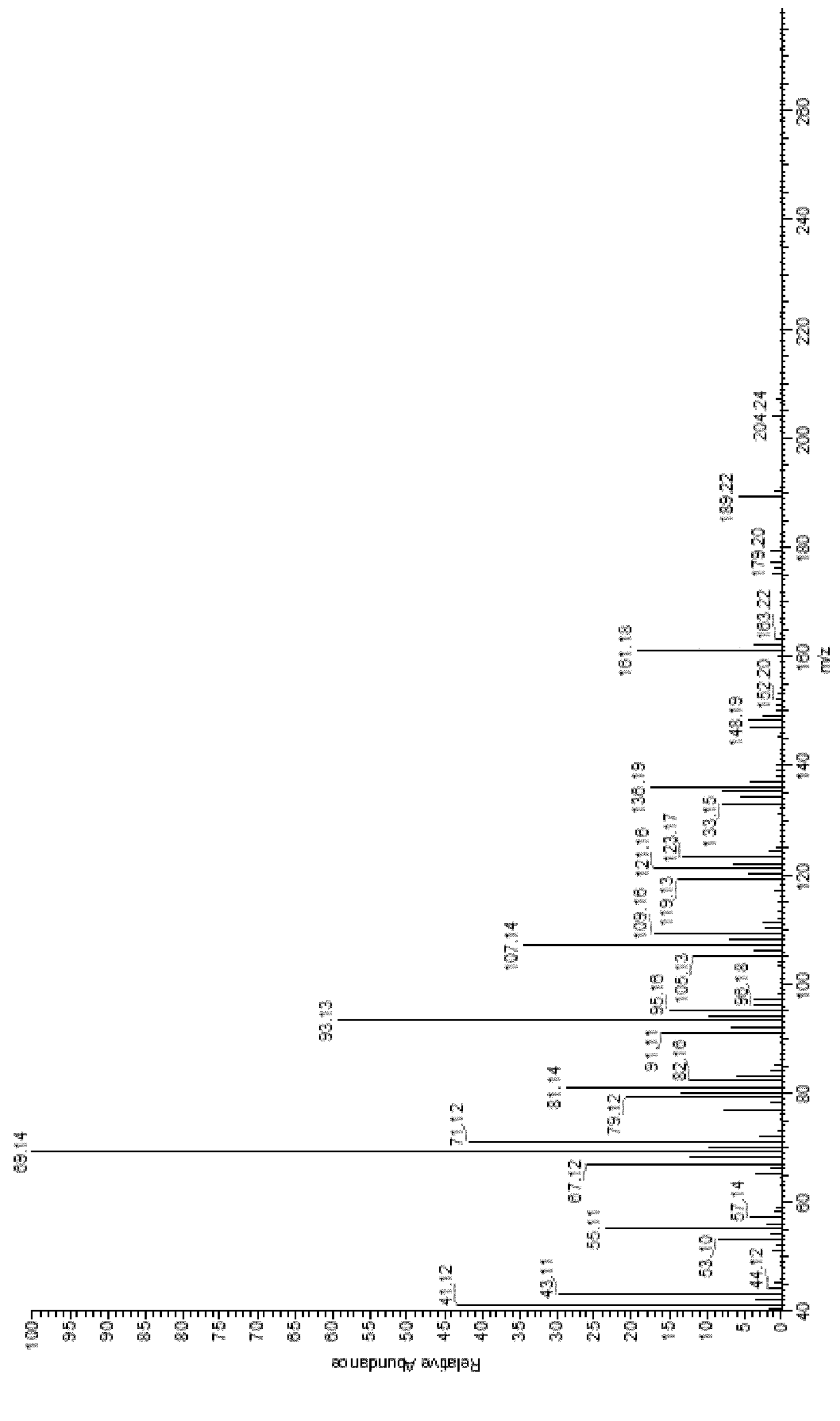
Relative Abundance
m/z
69.14
93.13
41.12
43.11
44.12
53.10
55.11
57.14
67.12
71.12
79.12
81.14
82.16
91.11
95.16
96.18
105.13
107.14
109.16
119.13
121.16
123.17
133.15
136.19
148.19
152.20
161.18
163.22
179.20
189.22
204.24

FIG. 7A

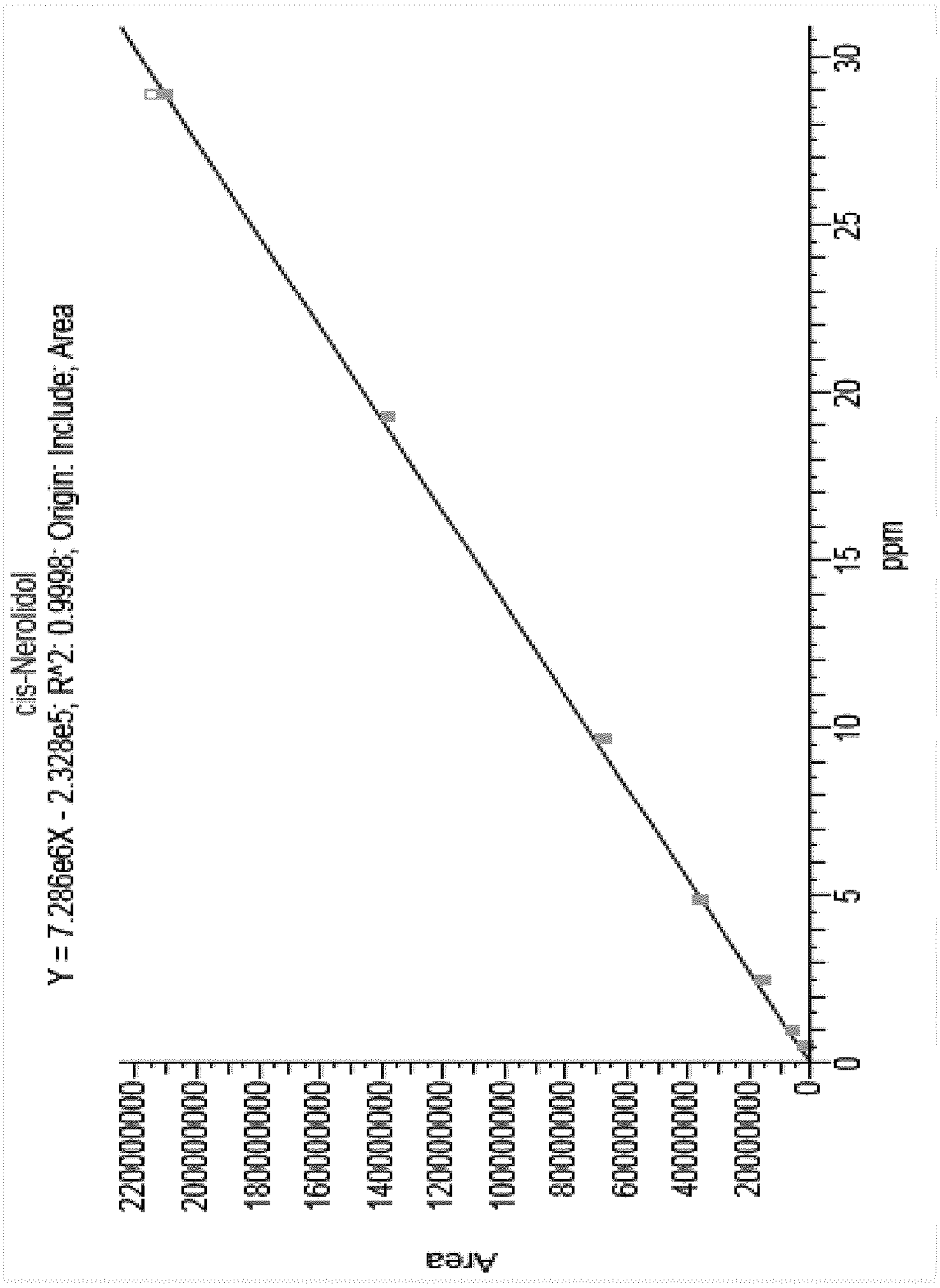
cis-Nerolidol
Y = 7.286e6X - 2.328e5; R^2: 0.9998; Origin: Include; Area
Area
220000000
200000000
180000000
160000000
140000000
120000000
100000000
80000000
60000000
40000000
20000000
0
0
5
10
15
20
25
30
ppm

FIG. 7B

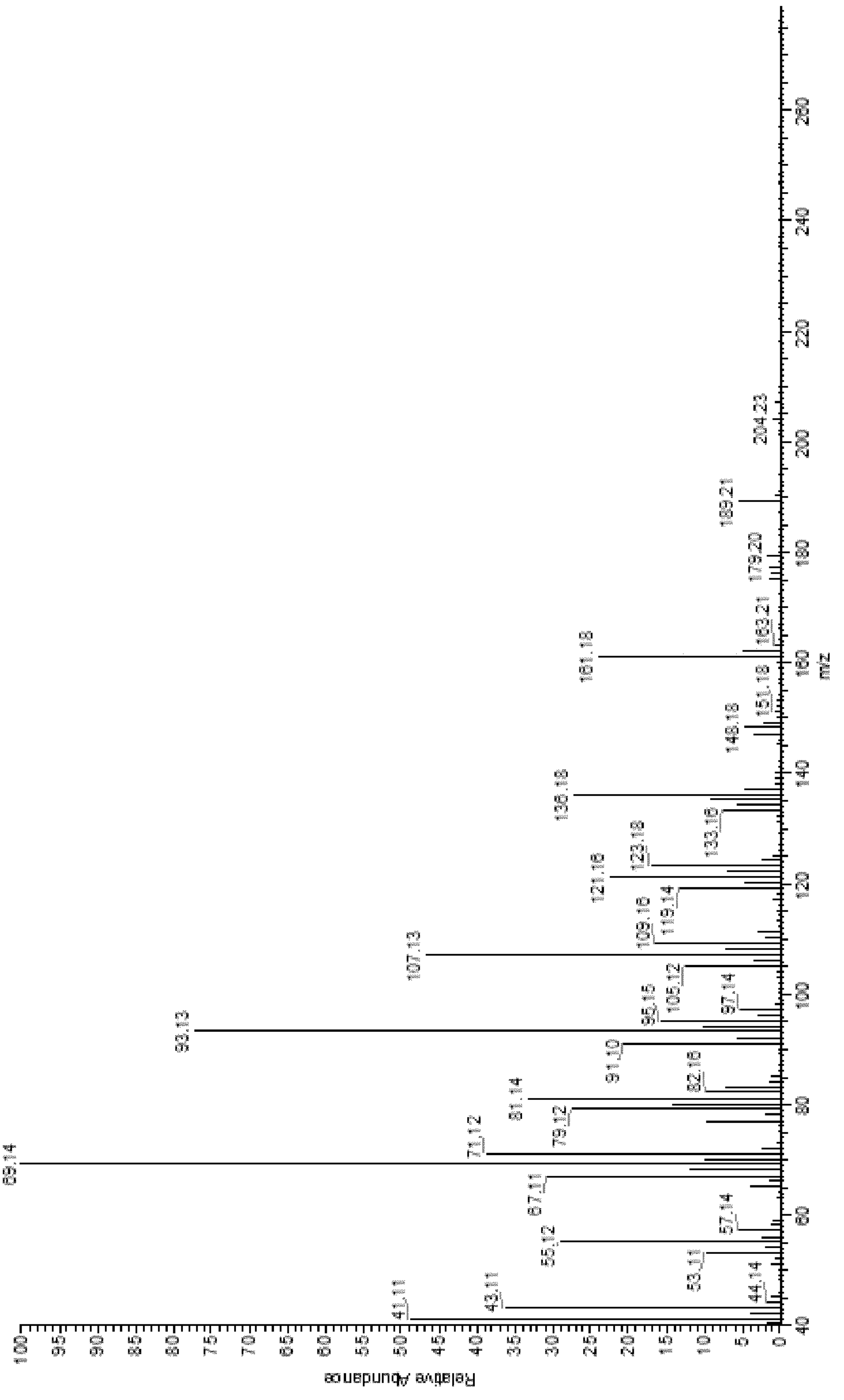
69.14
93.13
107.13
41.11
43.11
55.12
67.11
71.12
79.12
81.14
91.10
121.16
136.18
161.18
95.15
105.12
109.16
119.14
123.18
133.16
149.18
151.18
163.21
179.20
189.21
204.23
53.11
44.14
57.14
82.16
97.14
Relative Abundance
m/z

FIG. 7C

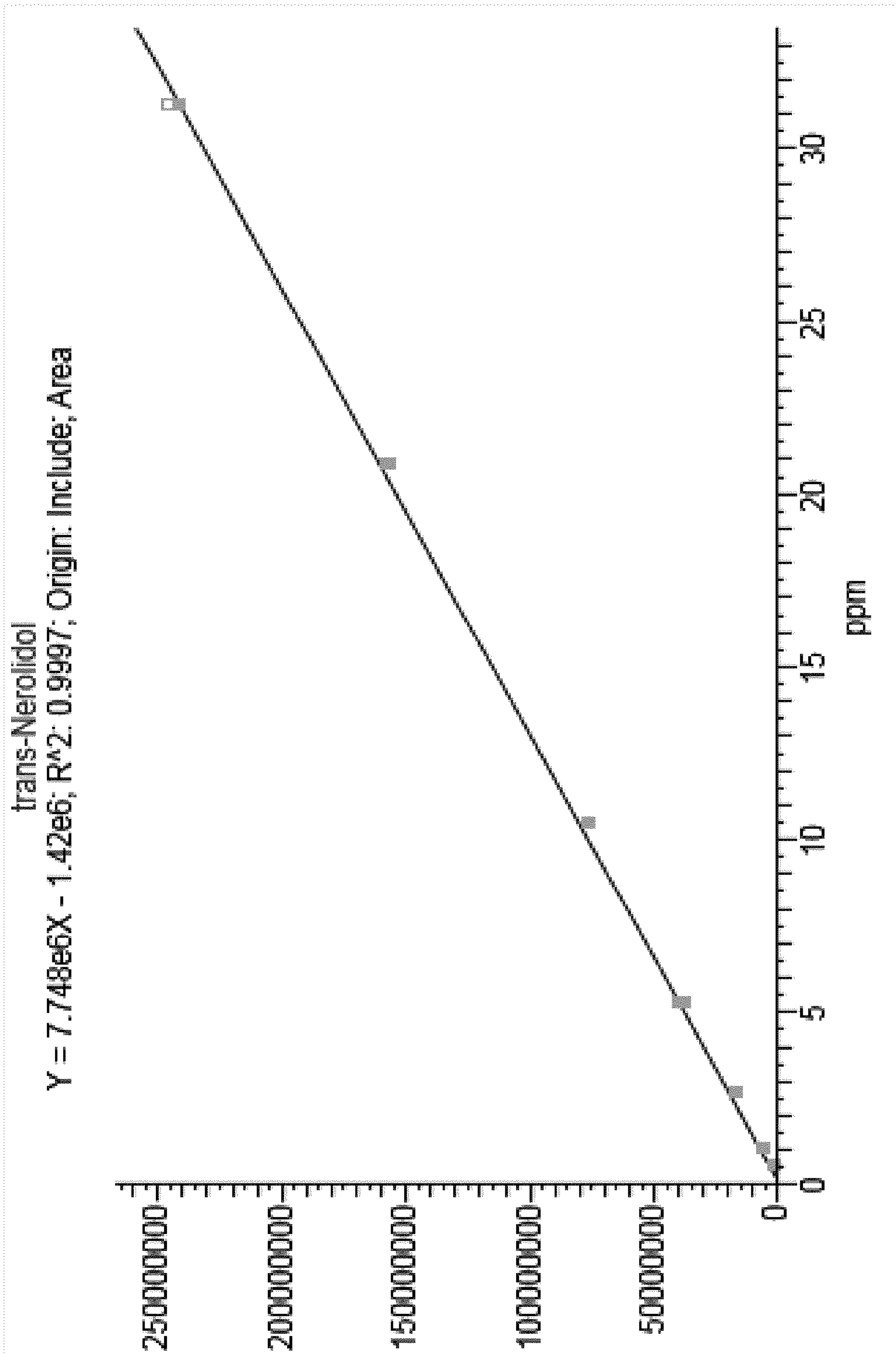
trans-Nerolidol
Y = 7.748e6X - 1.42e6; R^2: 0.9997; Origin: Include; Area
Area
250000000
200000000
150000000
100000000
50000000
0
0
5
10
15
20
25
30
ppm

FIG. 7D

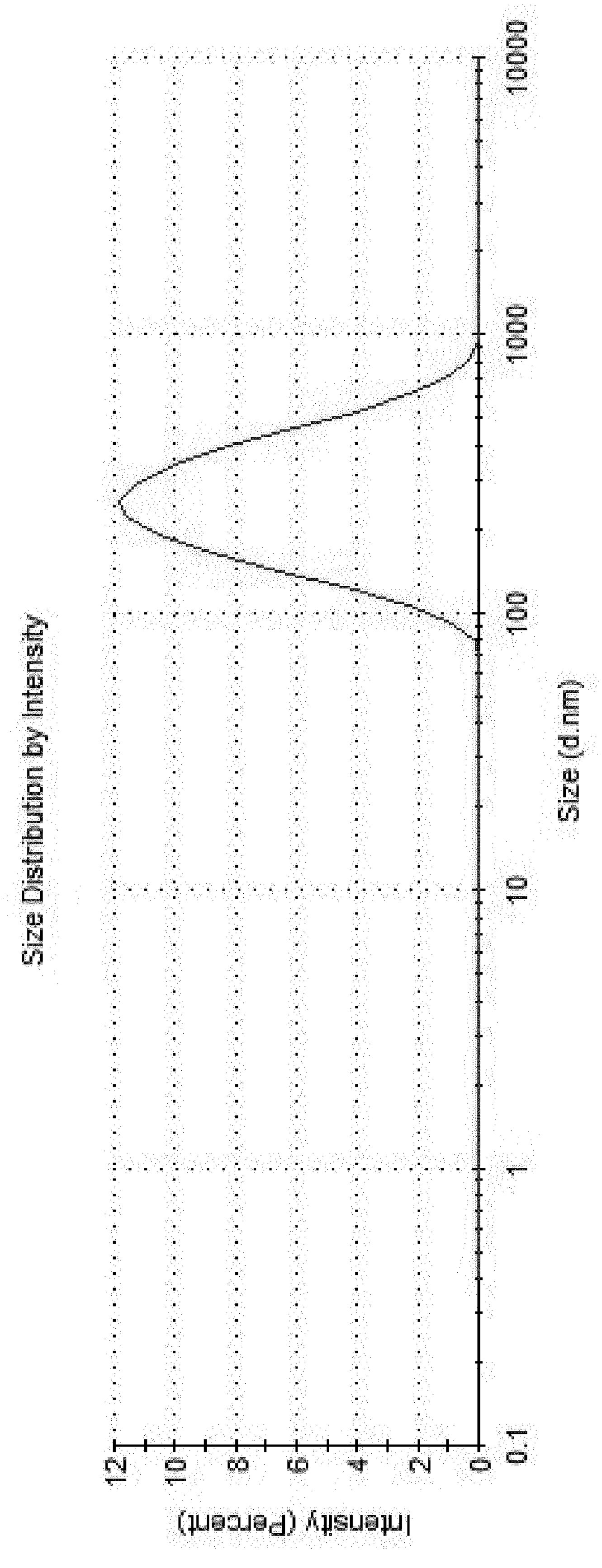
Size Distribution by Intensity
Intensity (Percent)
12
10
8
6
4
2
0
0.1
1
10
100
1000
10000
Size (d.nm)

FIG. 8A

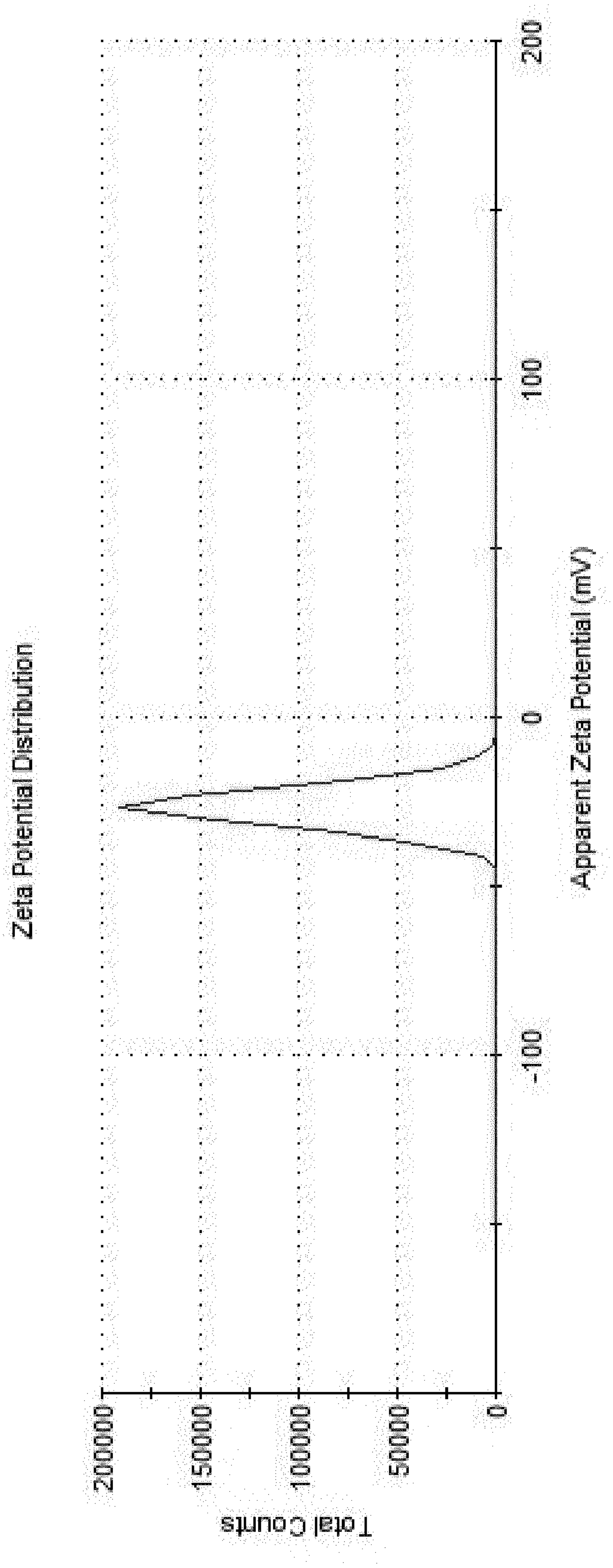
Zeta Potential Distribution
Total Counts
200000
150000
100000
50000
0
-100
0
100
200
Apparent Zeta Potential (mV)

FIG. 8B

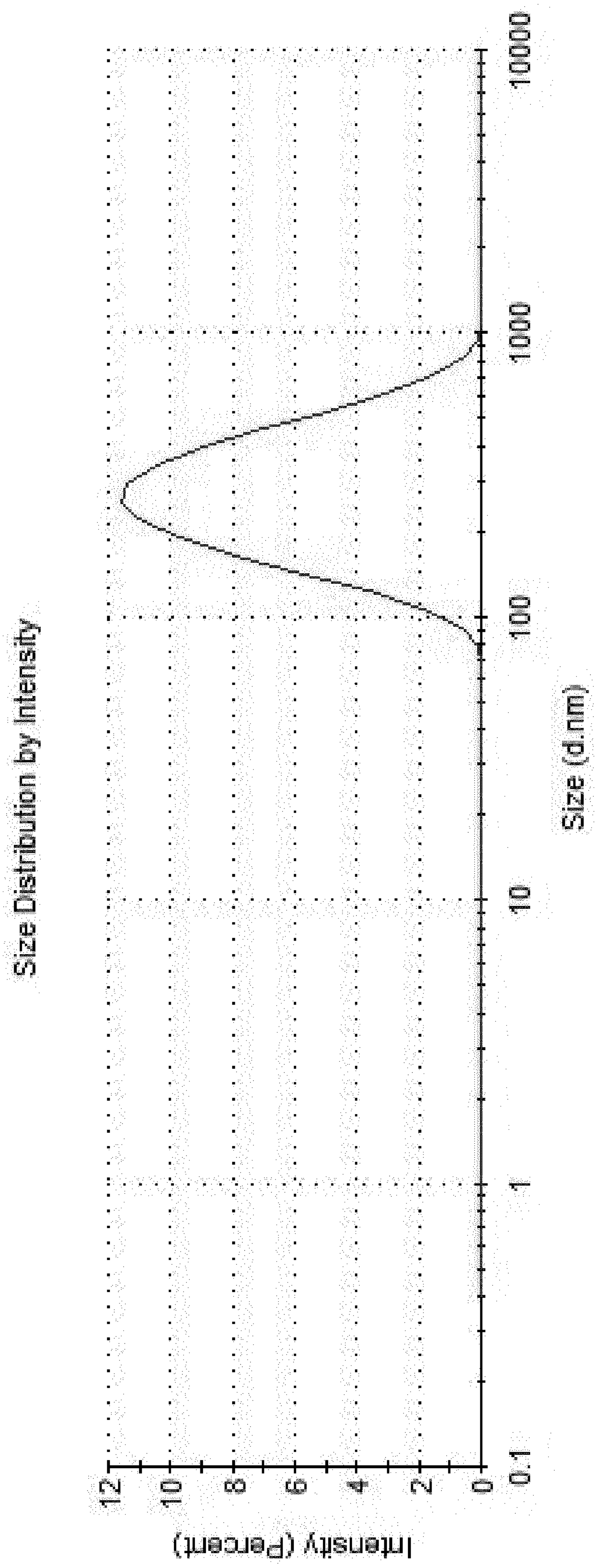
Size Distribution by Intensity
Intensity (Percent)
12
10
8
6
4
2
0
0.1
1
10
100
1000
10000
Size (d.nm)

FIG. 8C

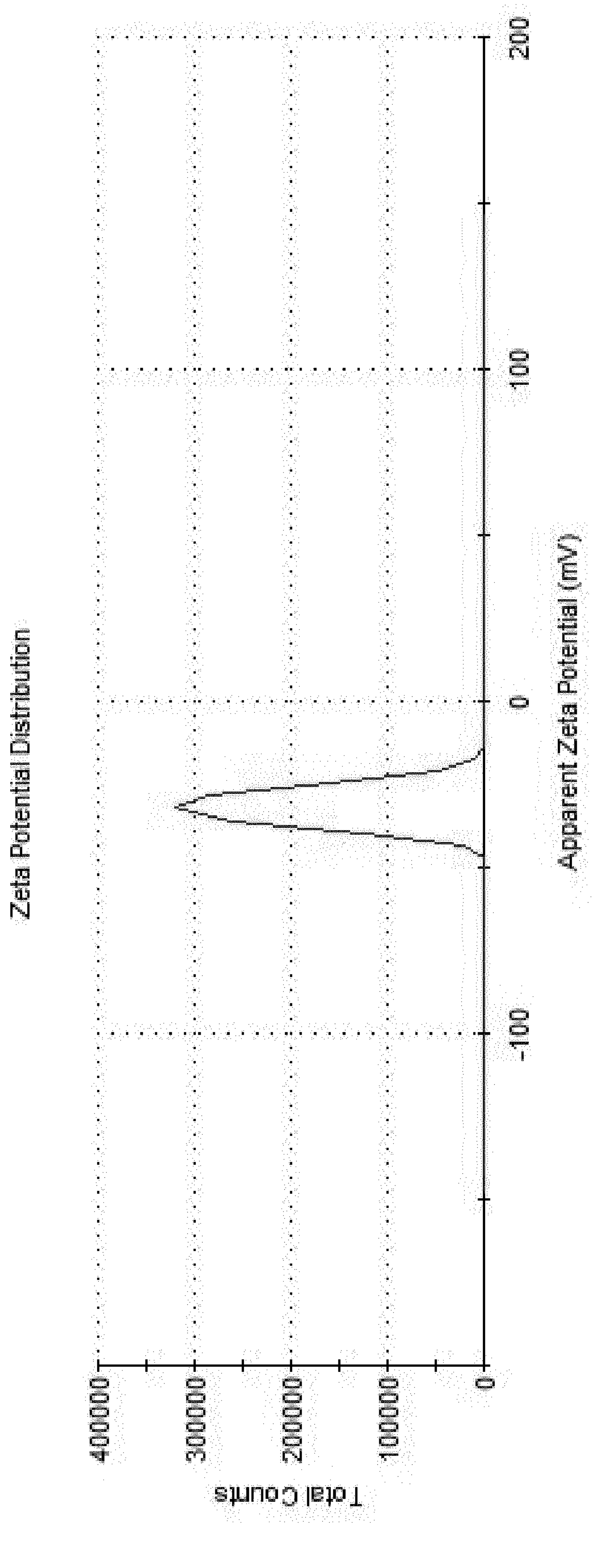
Zeta Potential Distribution
Total Counts
400000
300000
200000
100000
0
-100
0
100
200
Apparent Zeta Potential (mV)

FIG. 8D

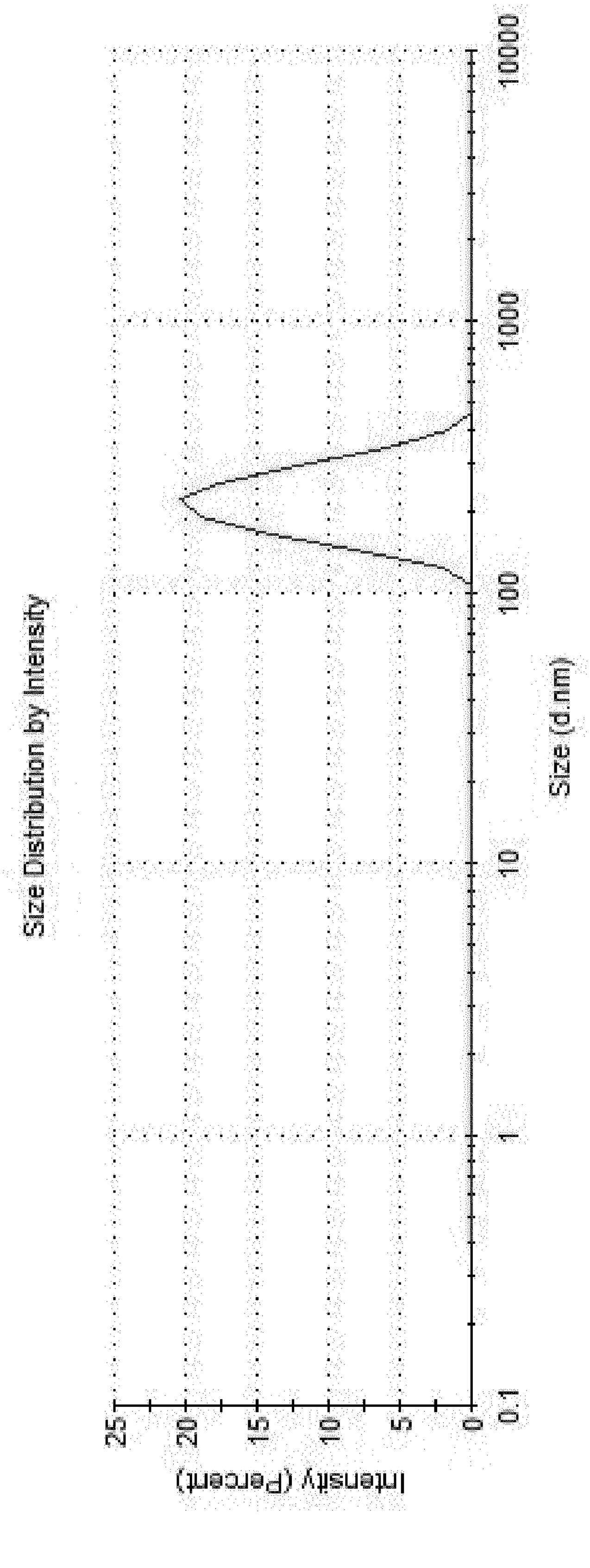
Size Distribution by Intensity
Intensity (Percent)
25
20
15
10
5
0
0.1
1
10
100
1000
10000
Size (d.nm)

FIG. 8E

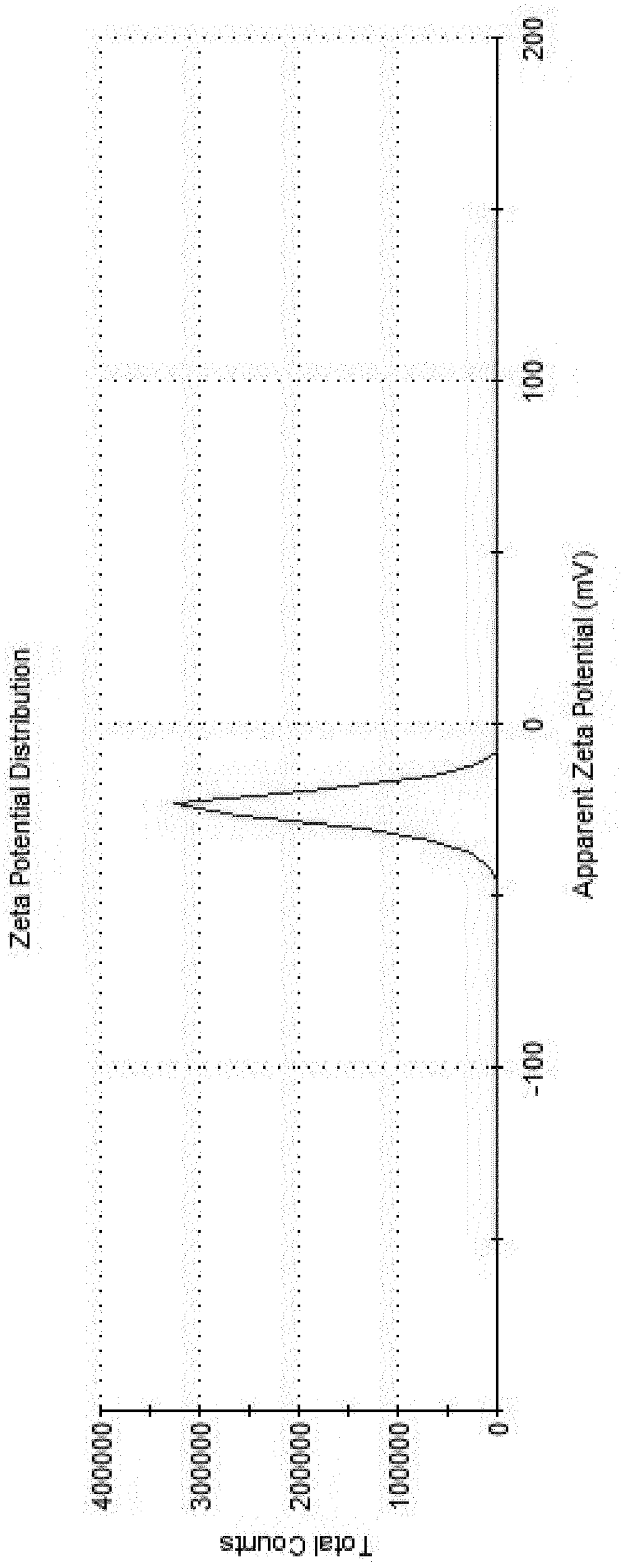
Zeta Potential Distribution
Total Counts
400000
300000
200000
100000
0
-100
0
100
200
Apparent Zeta Potential (mV)

FIG. 8F

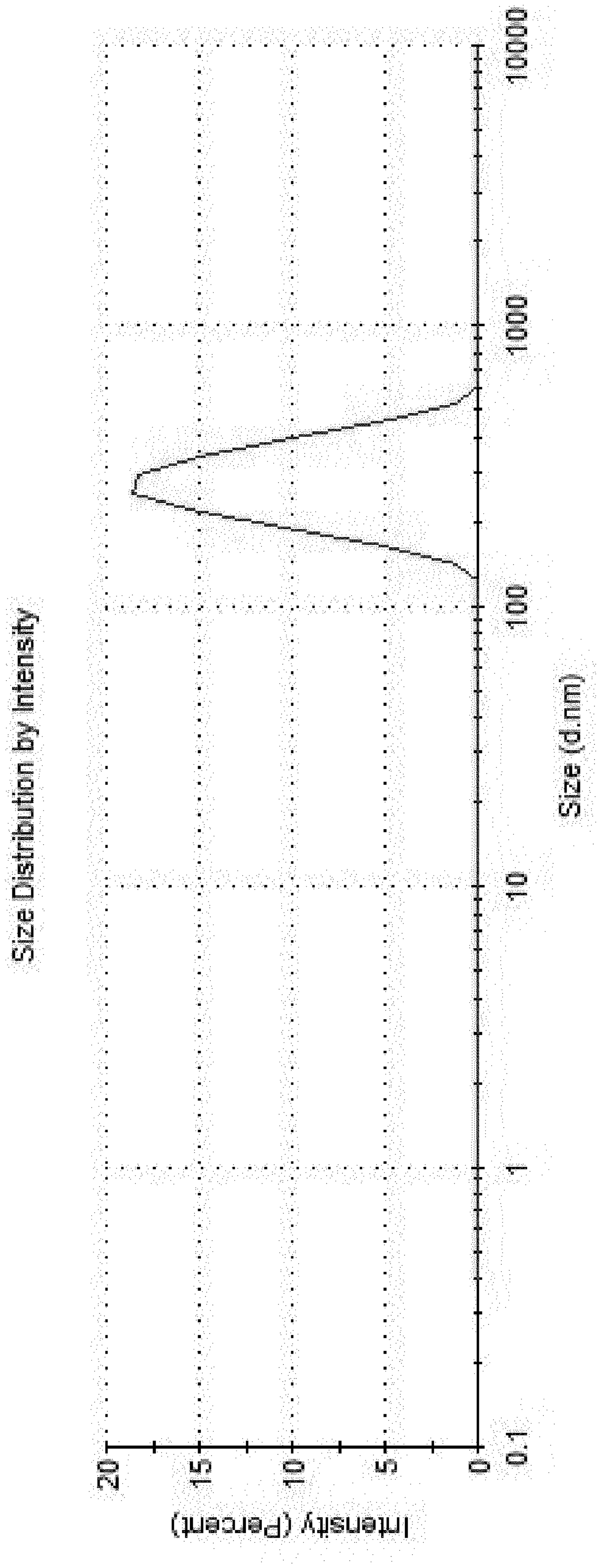
Size Distribution by Intensity
Intensity (Percent)
20
15
10
5
0
0.1
1
10
100
1000
10000
Size (d.nm)

FIG. 9A

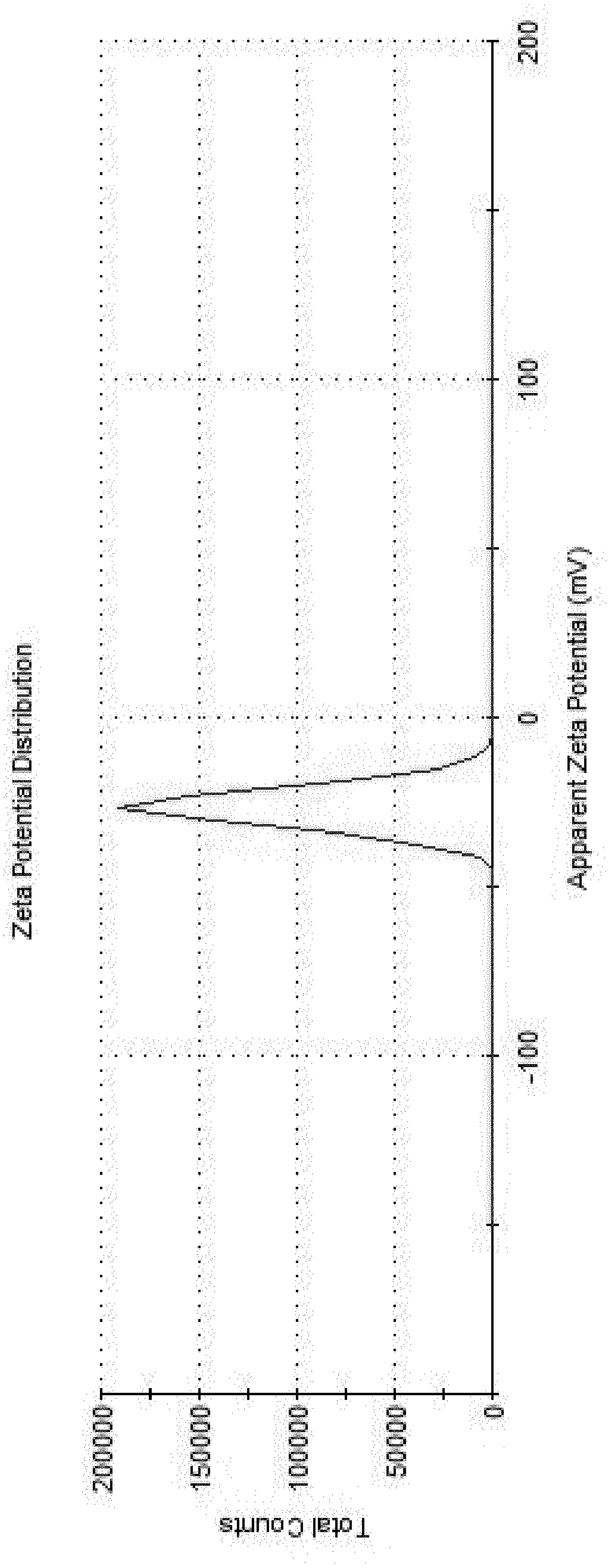
Zeta Potential Distribution
Total Counts
200000
150000
100000
50000
0
-100
0
100
200
Apparent Zeta Potential (mV)

FIG. 9B

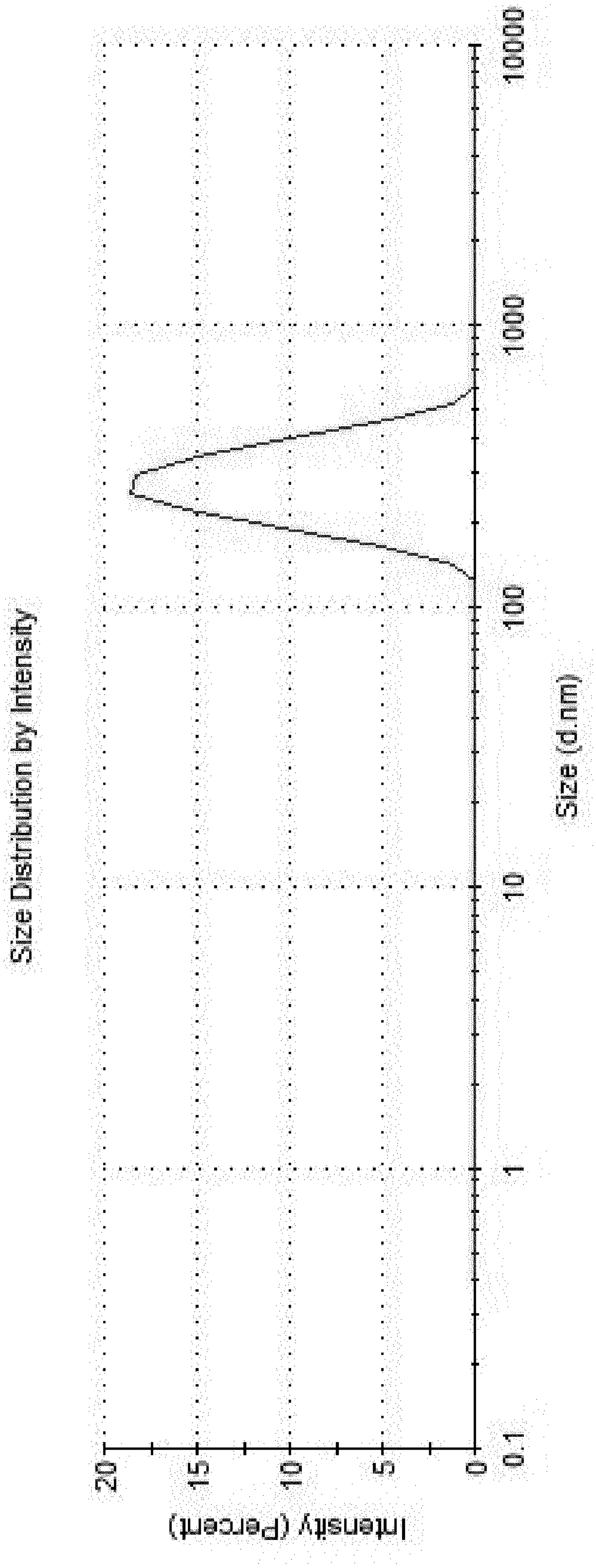
Size Distribution by Intensity
Intensity (Percent)
20
15
10
5
0
0.1
1
10
100
1000
10000
Size (d.nm)

**FIG. 10A**

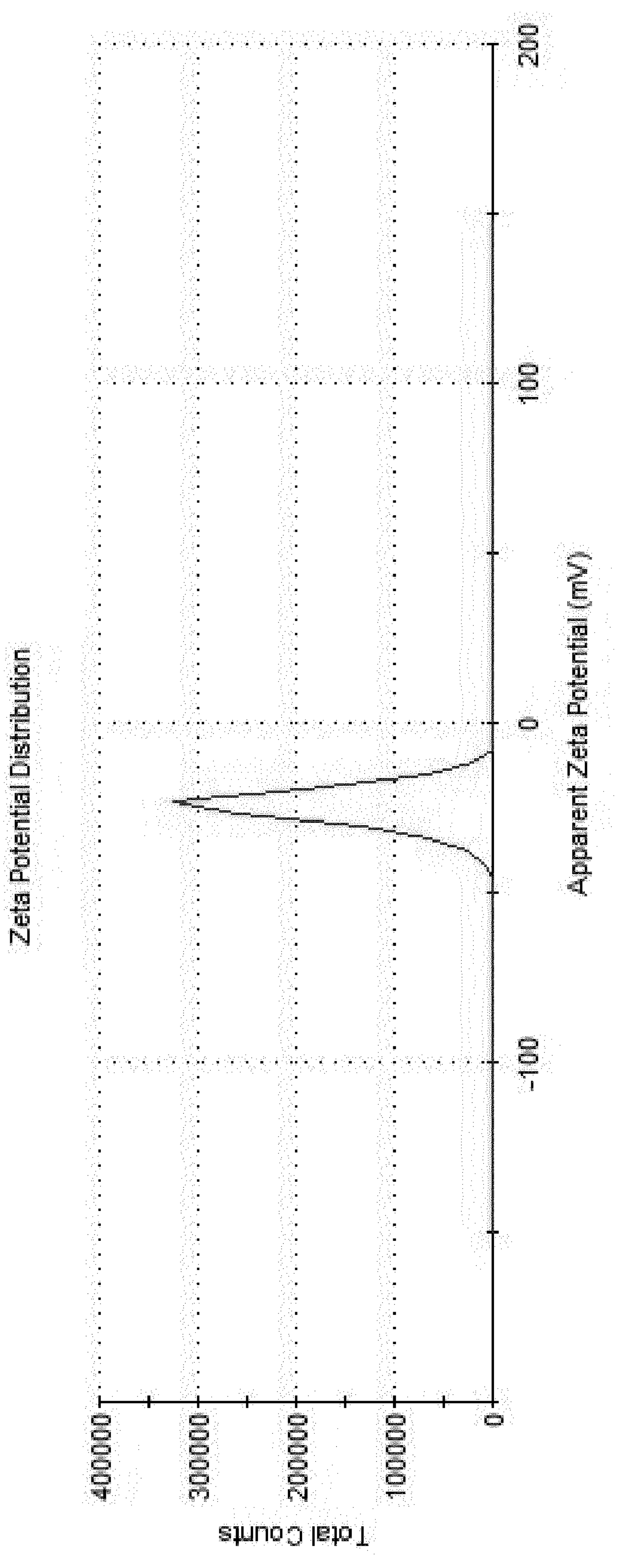
Zeta Potential Distribution
Total Counts
400000
300000
200000
100000
0
-100
0
100
200
Apparent Zeta Potential (mV)

FIG. 10B

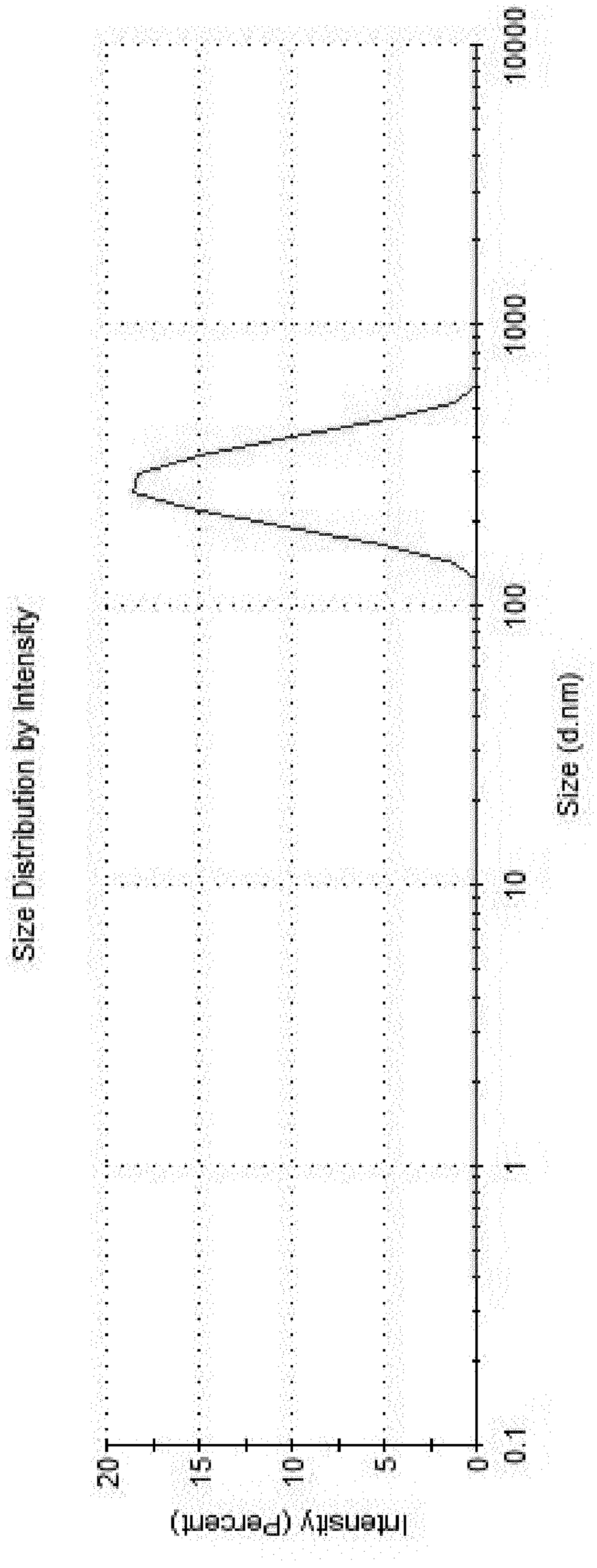
Size Distribution by Intensity
Intensity (Percent)
20
15
10
5
0
0.1
1
10
100
1000
10000
Size (d.nm)

FIG. 11A

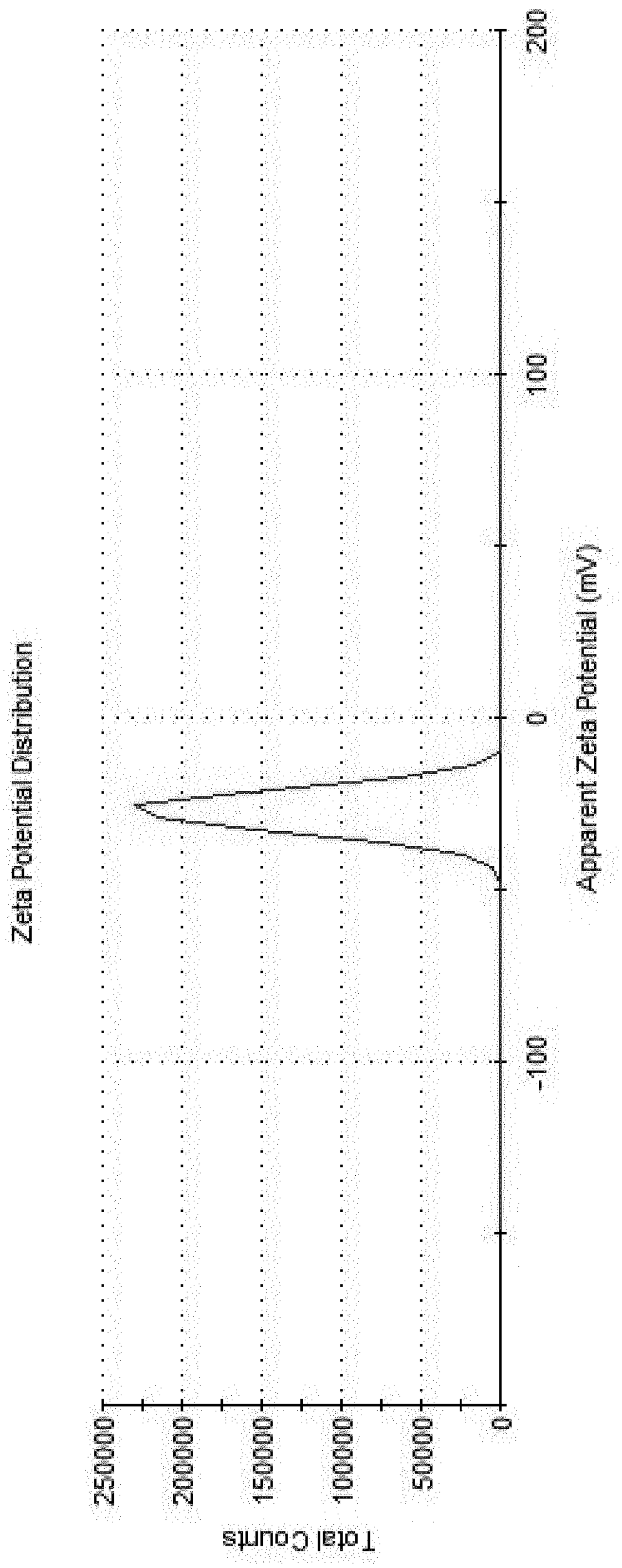
Zeta Potential Distribution
Total Counts
250000
200000
150000
100000
50000
0
-100
0
100
200
Apparent Zeta Potential (mV)

FIG. 11B

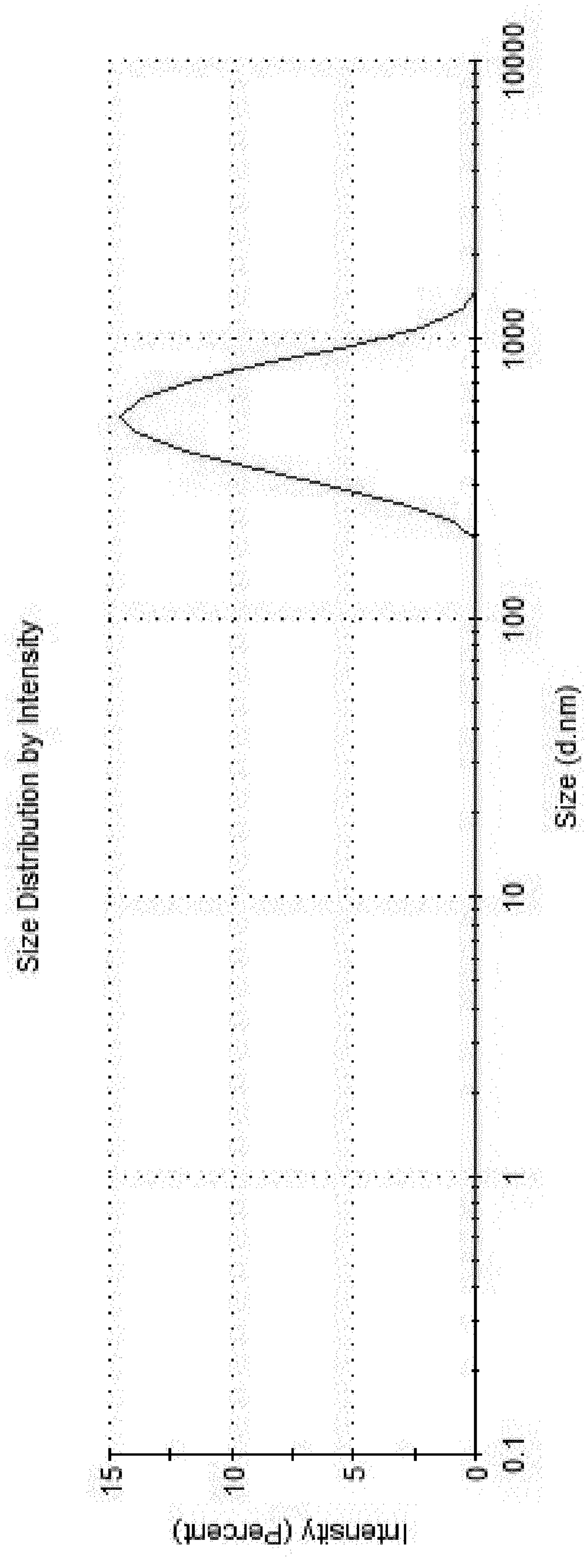
Size Distribution by Intensity
Intensity (Percent)
15
10
5
0
0.1
1
10
100
1000
10000
Size (d.nm)

FIG. 11C

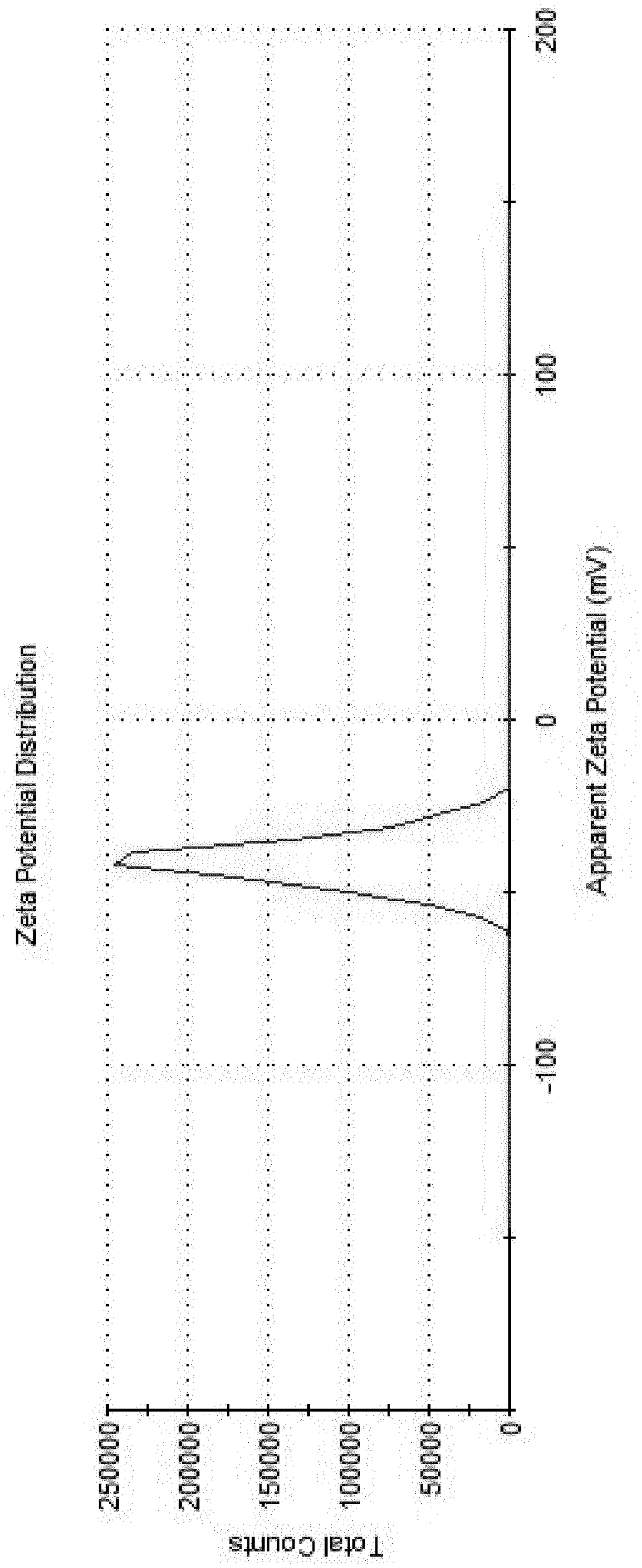
Zeta Potential Distribution
Total Counts
250000
200000
150000
100000
50000
0
-100
0
100
200
Apparent Zeta Potential (mV)

FIG. 11D

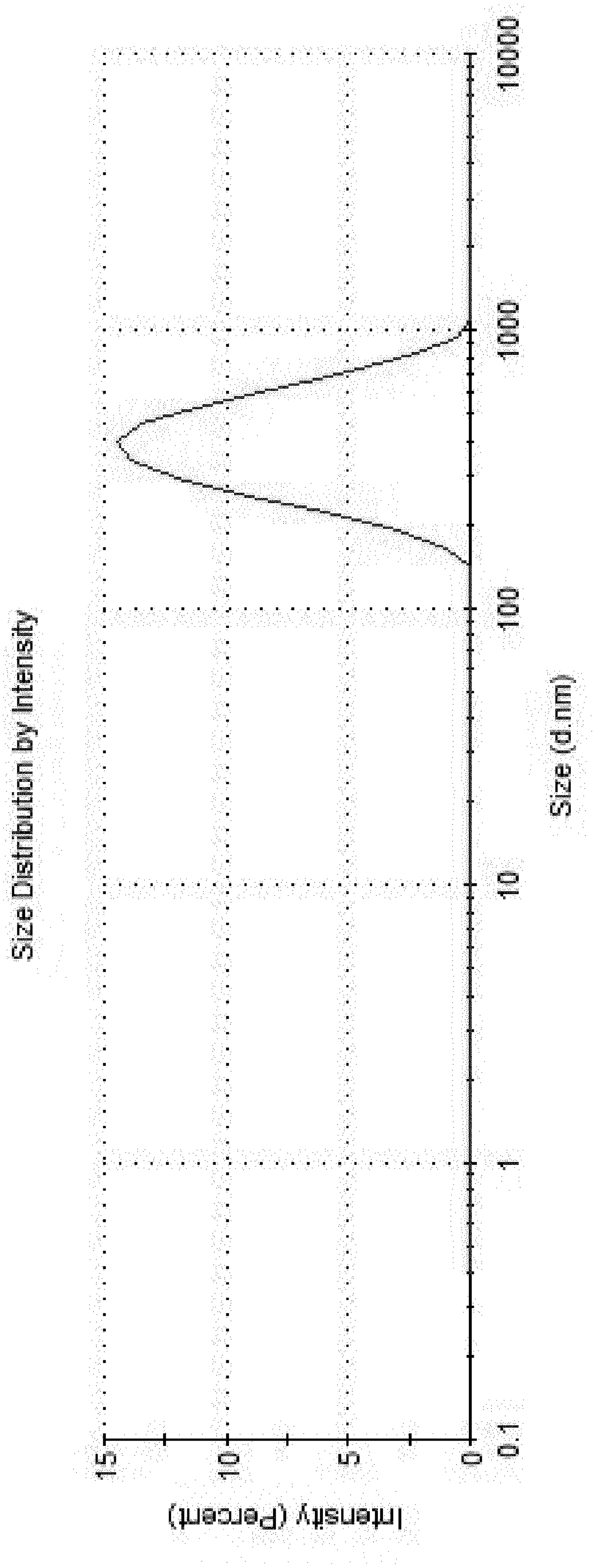
Size Distribution by Intensity
Intensity (Percent)
15
10
5
0
0.1
1
10
100
1000
10000
Size (d.nm)

FIG. 12A

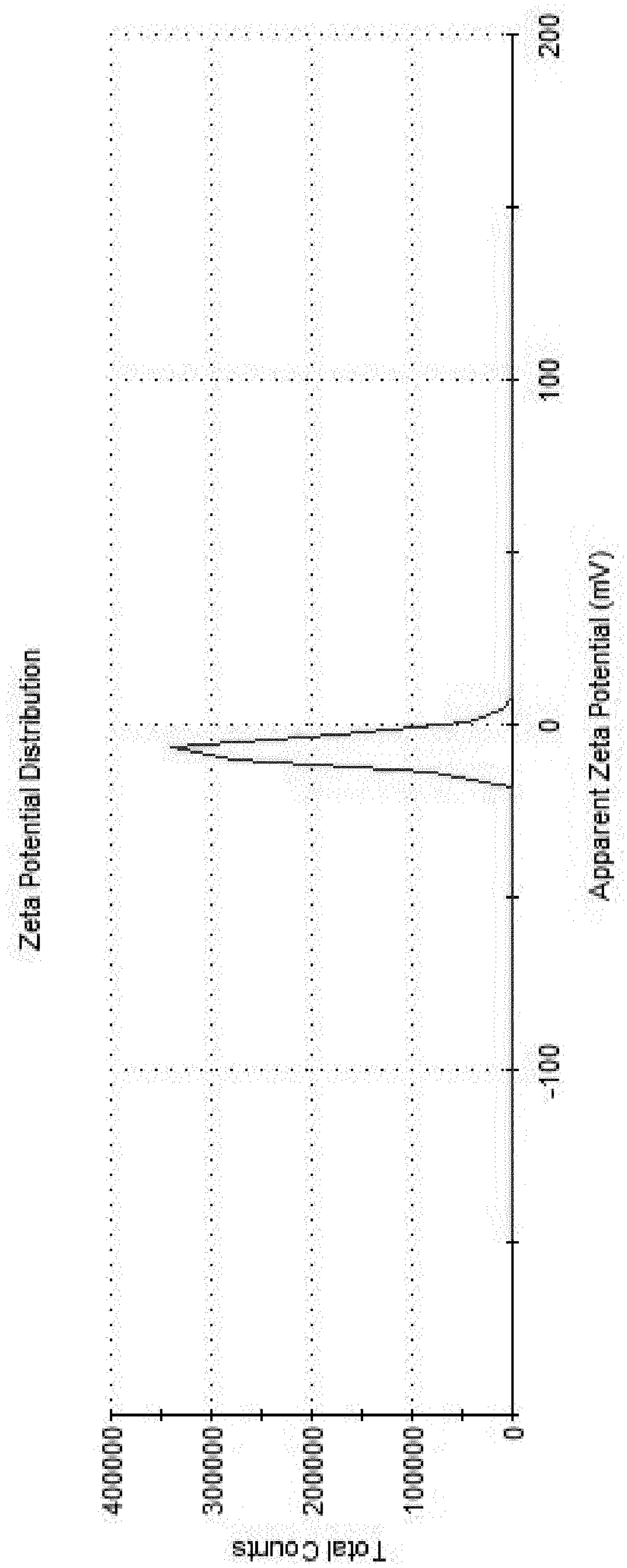
Zeta Potential Distribution
Total Counts
400000
300000
200000
100000
0
-100
0
100
200
Apparent Zeta Potential (mV)

FIG. 12B

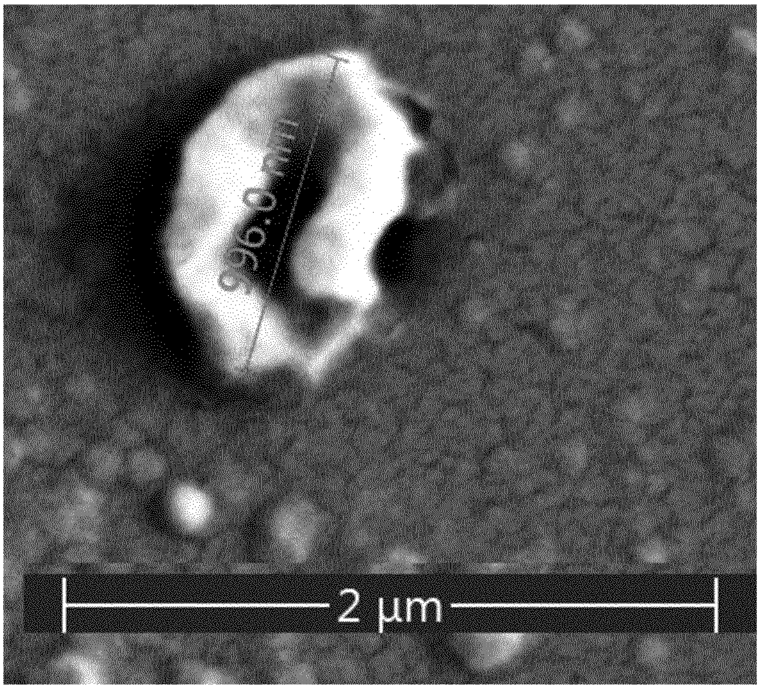


FIG. 13A

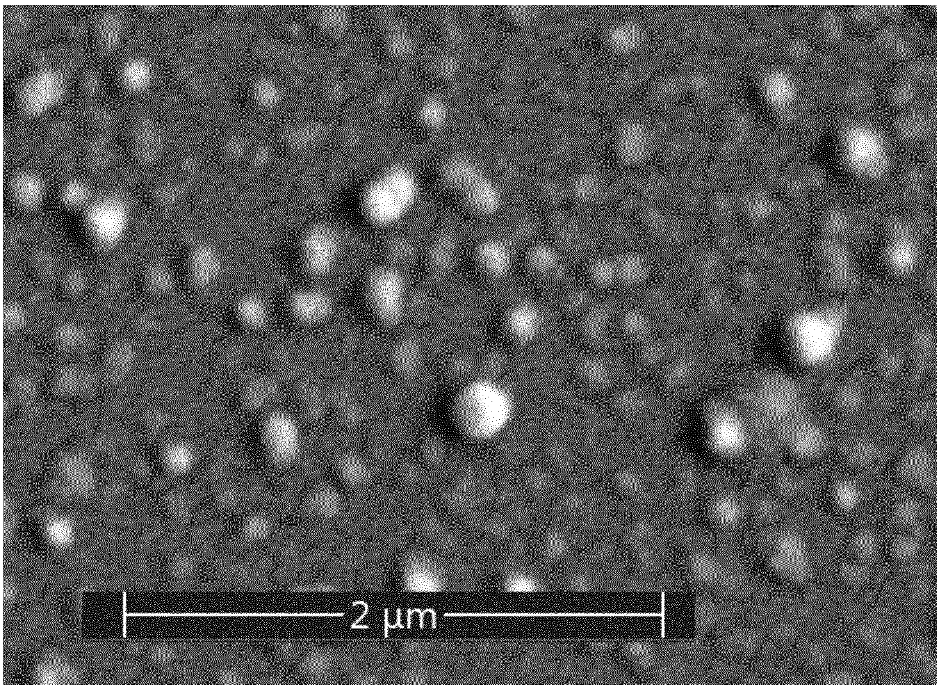


FIG. 13B

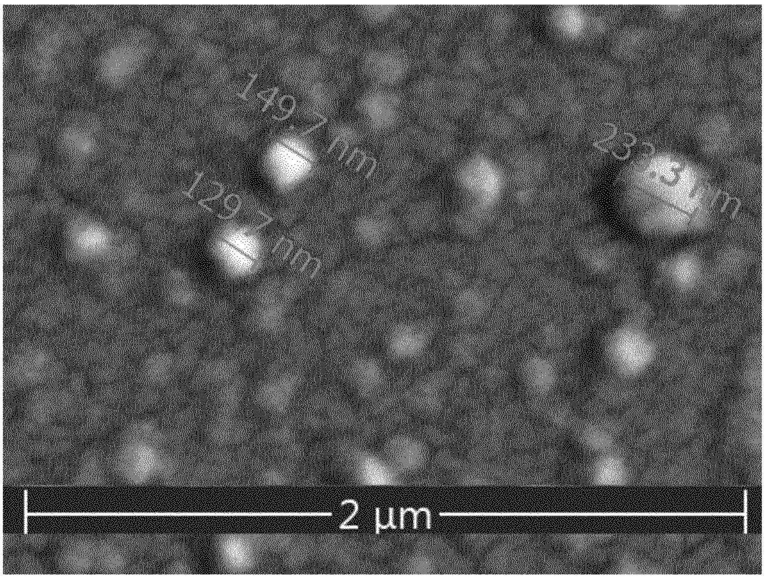


FIG. 14A

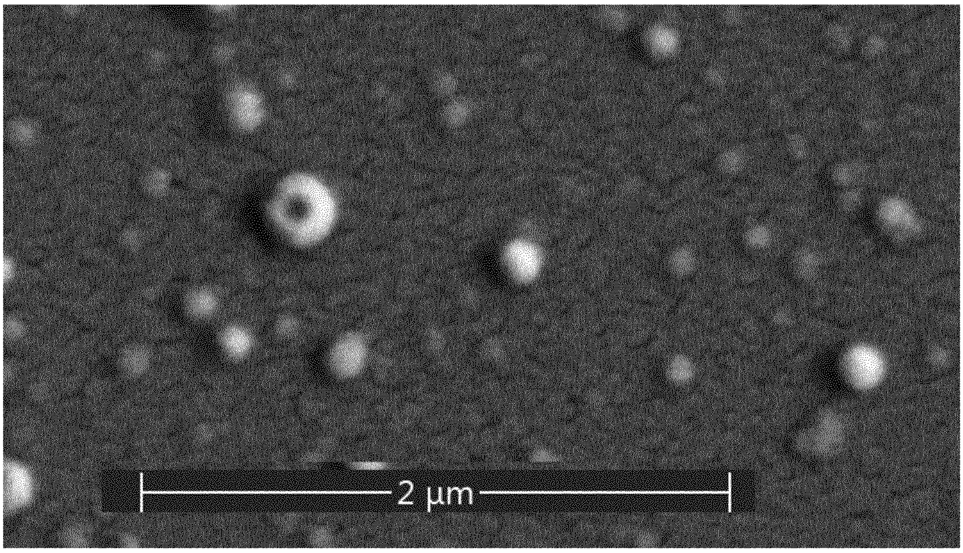


FIG. 14B

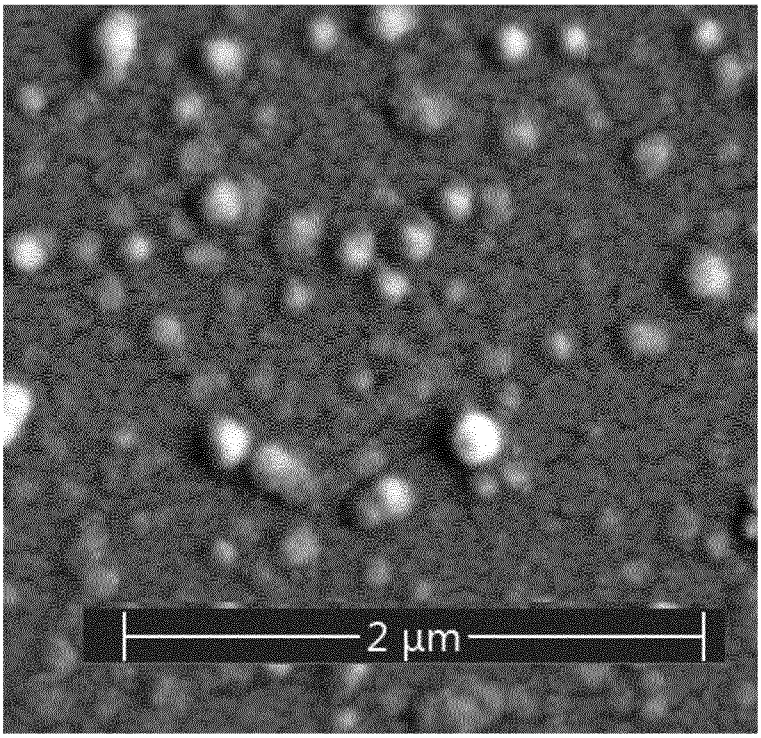


FIG. 15A

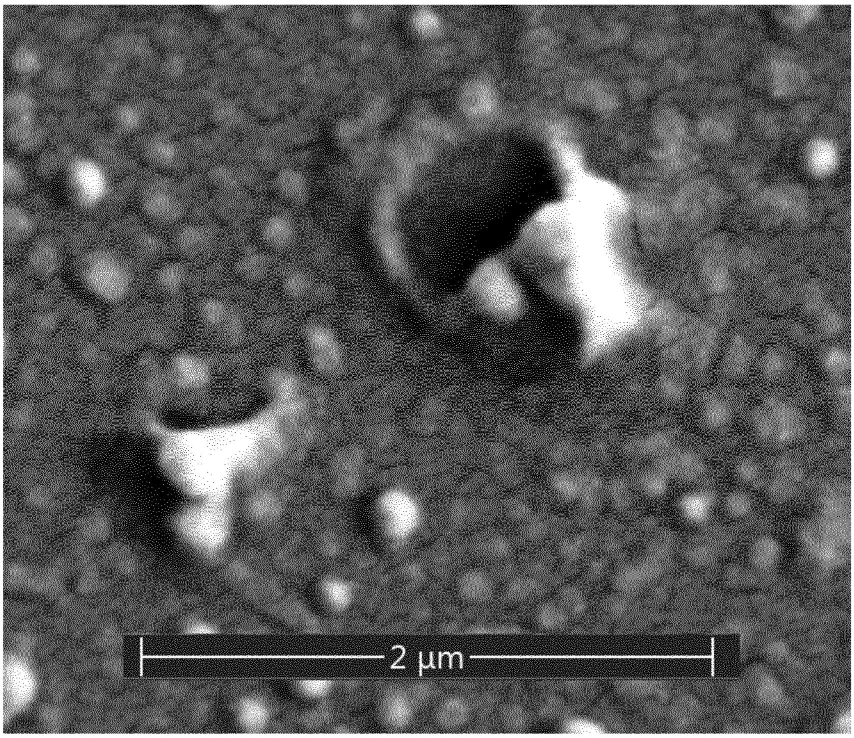


FIG. 15B

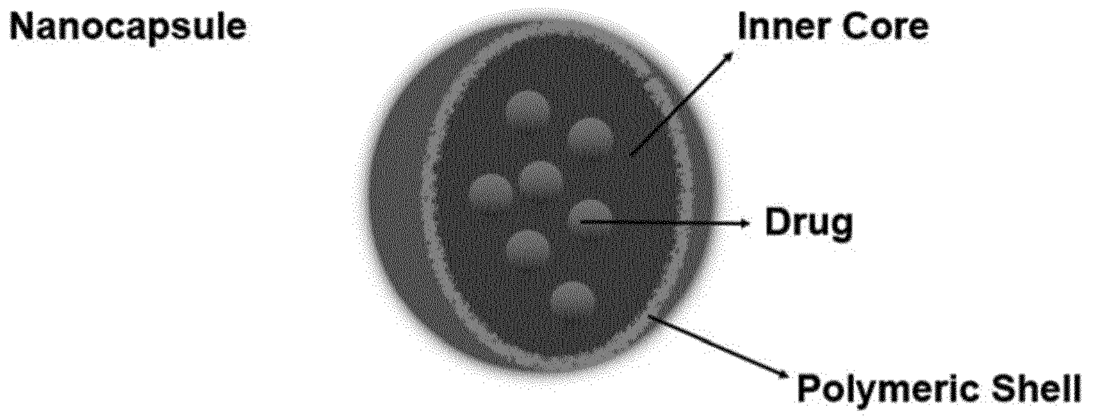
Nanocapsule
Inner Core
Drug
Polymeric Shell

FIG. 16

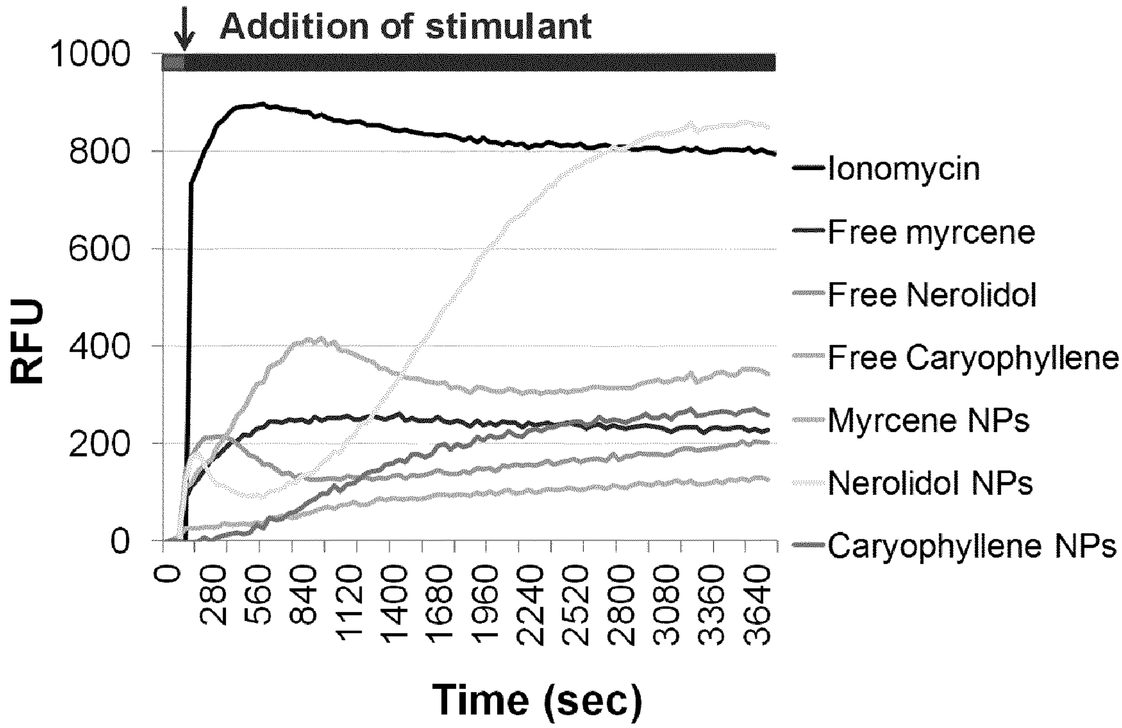
Addition of stimulant
1000
800
600
400
200
0
RFU
0
280
560
840
1120
1400
1680
1960
2240
2520
2800
3080
3360
3640
Time (sec)
Ionomycin
Free myrcene
Free Nerolidol
Free Caryophyllene
Myrcene NPs
Nerolidol NPs
Caryophyllene NPs

FIG. 17

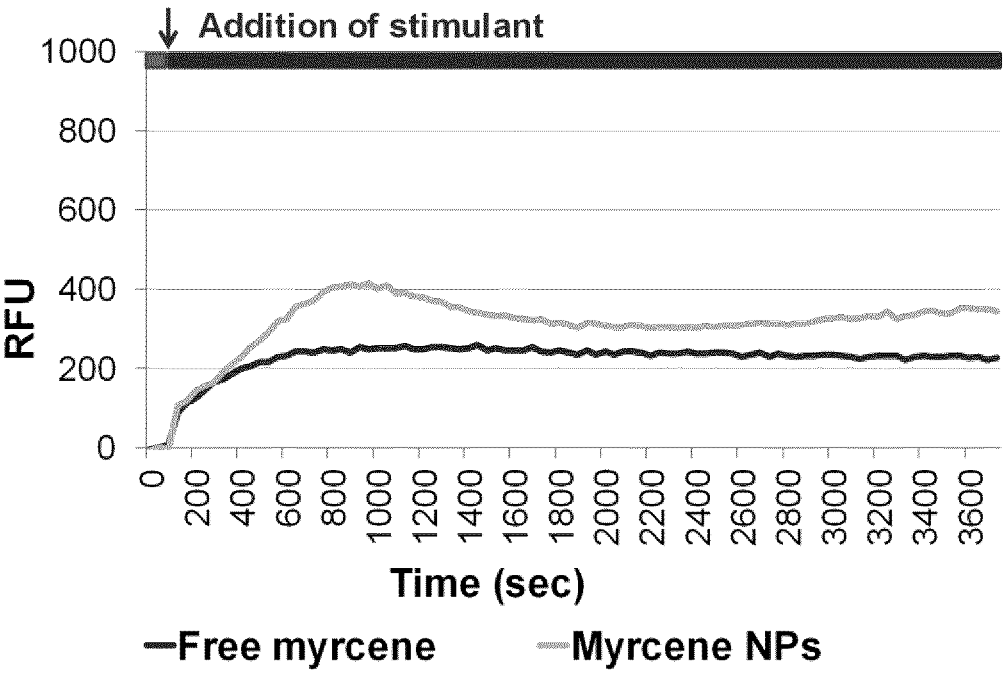


FIG. 18A

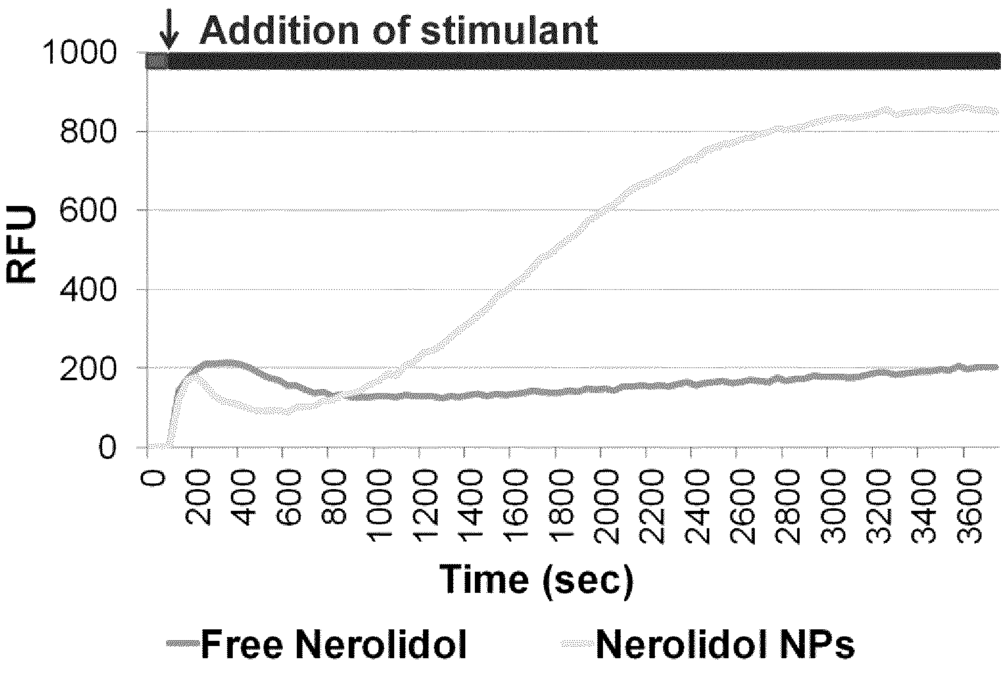


FIG. 18B

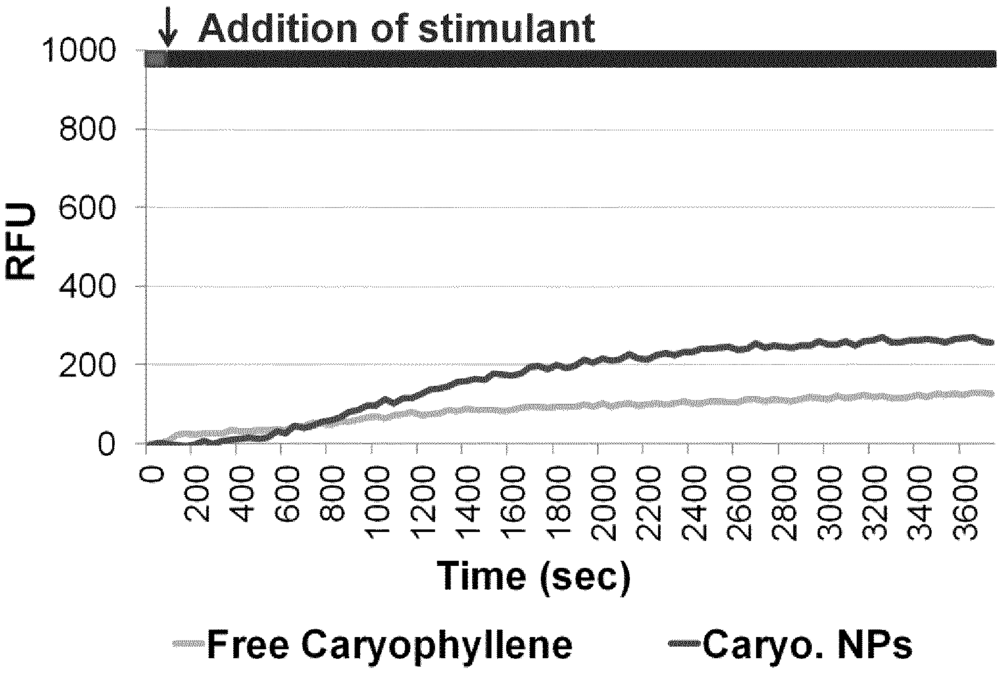


FIG. 18C

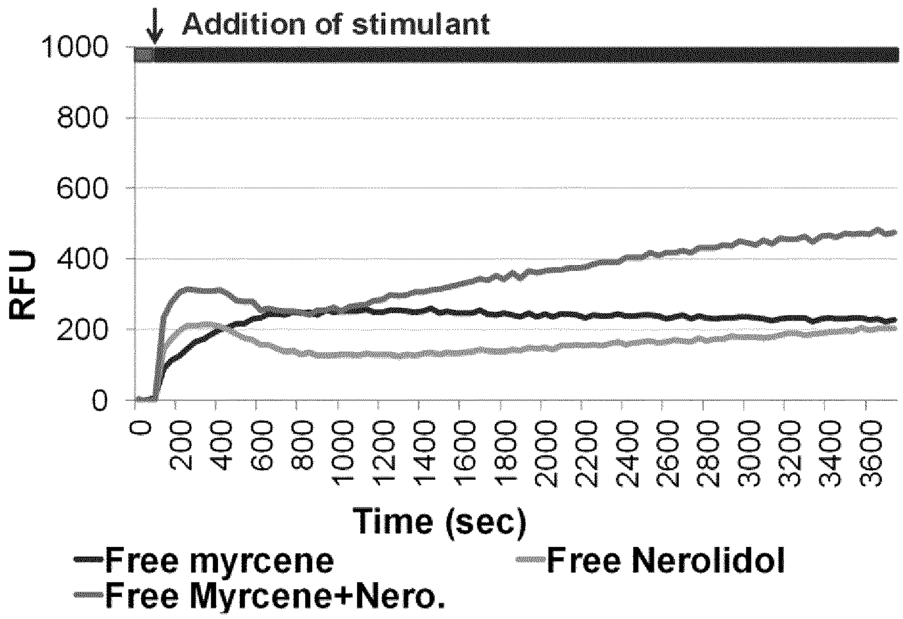


FIG. 19A

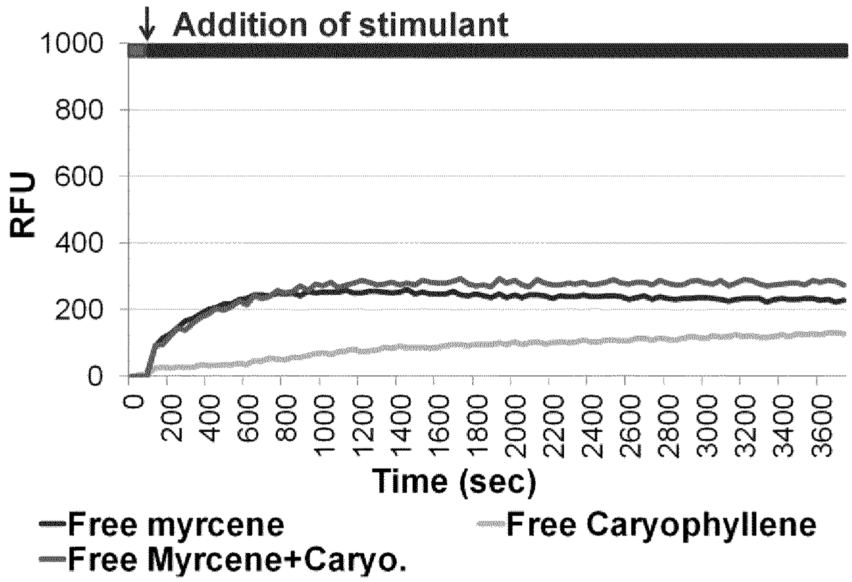


FIG. 19B

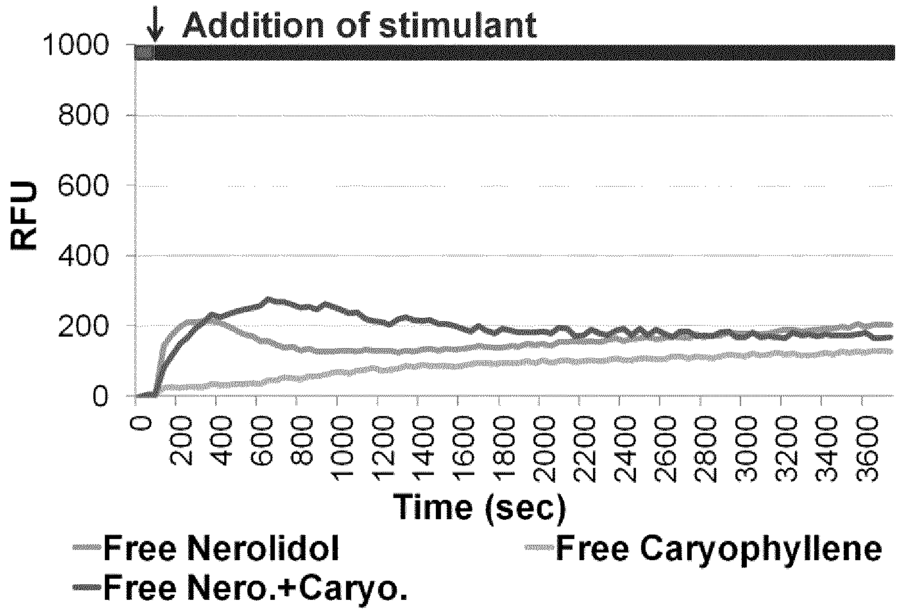


FIG. 19C

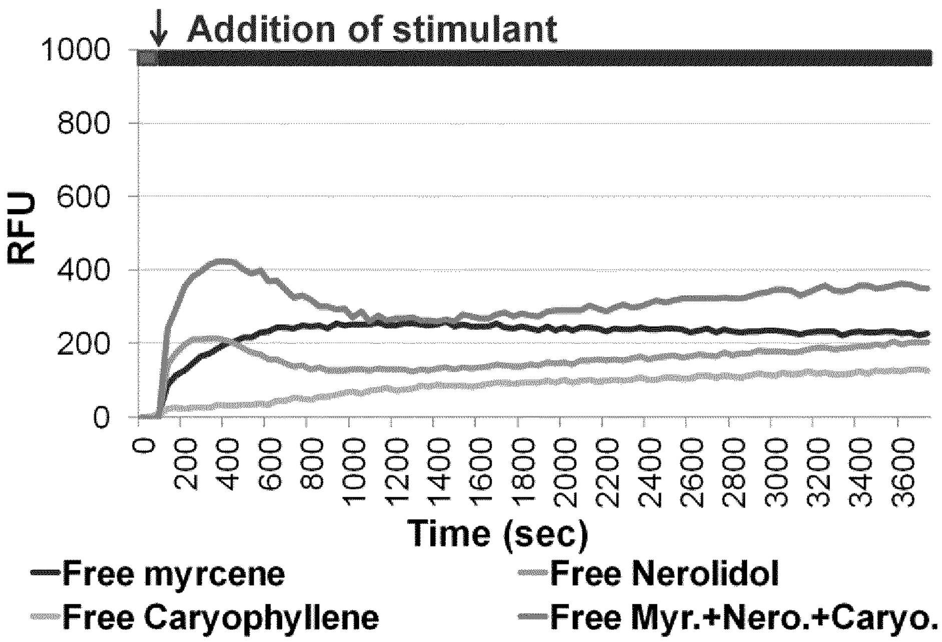


FIG. 19D

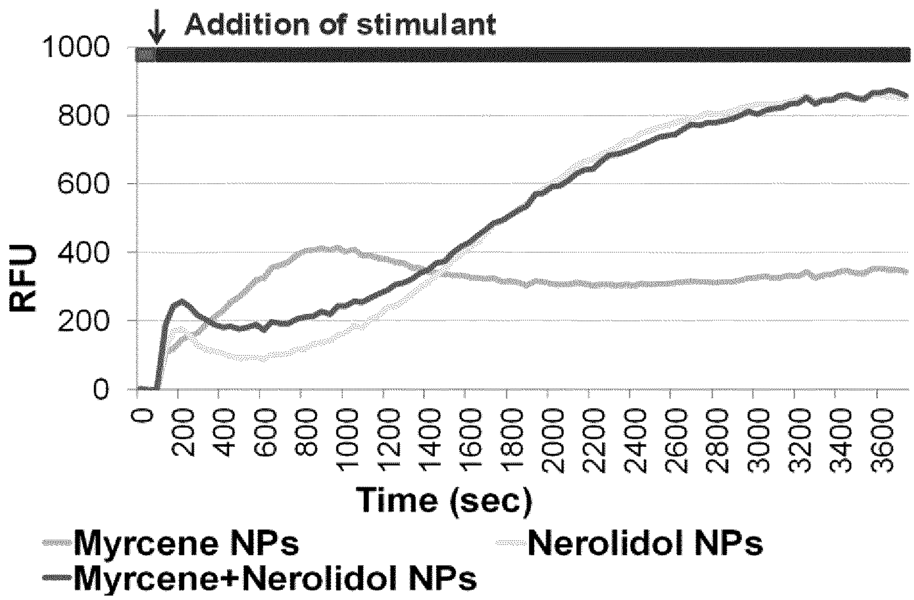


FIG. 20A

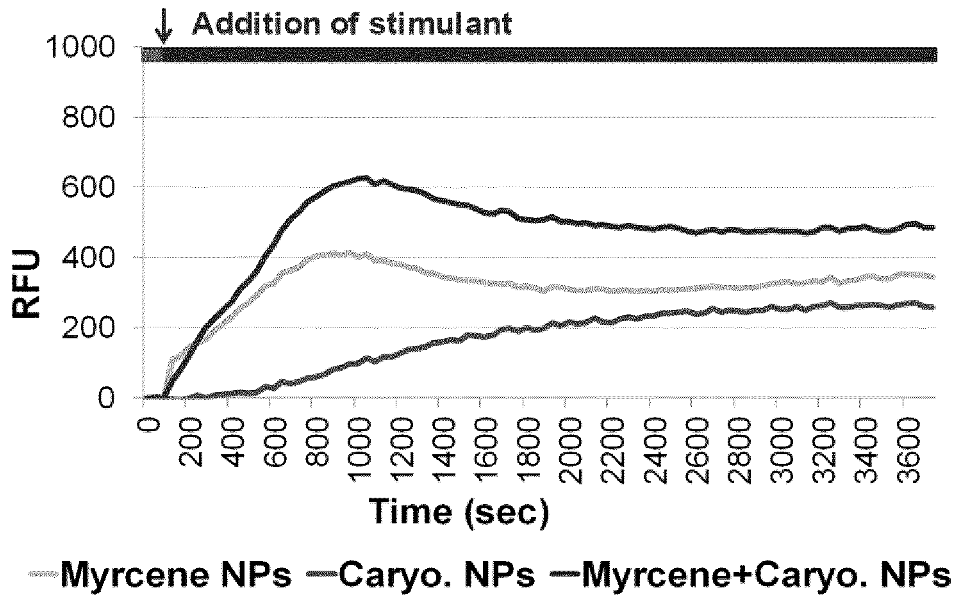


FIG. 20B

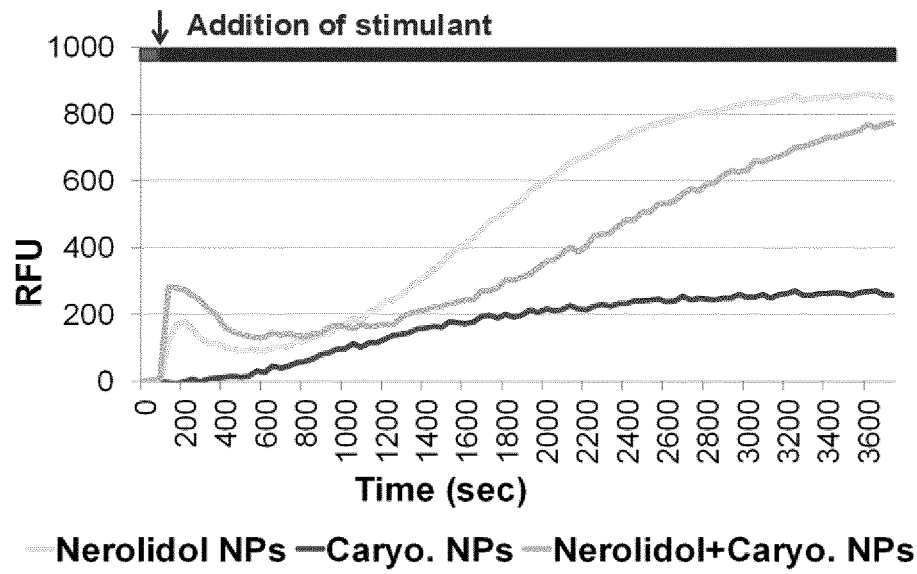


FIG. 20C

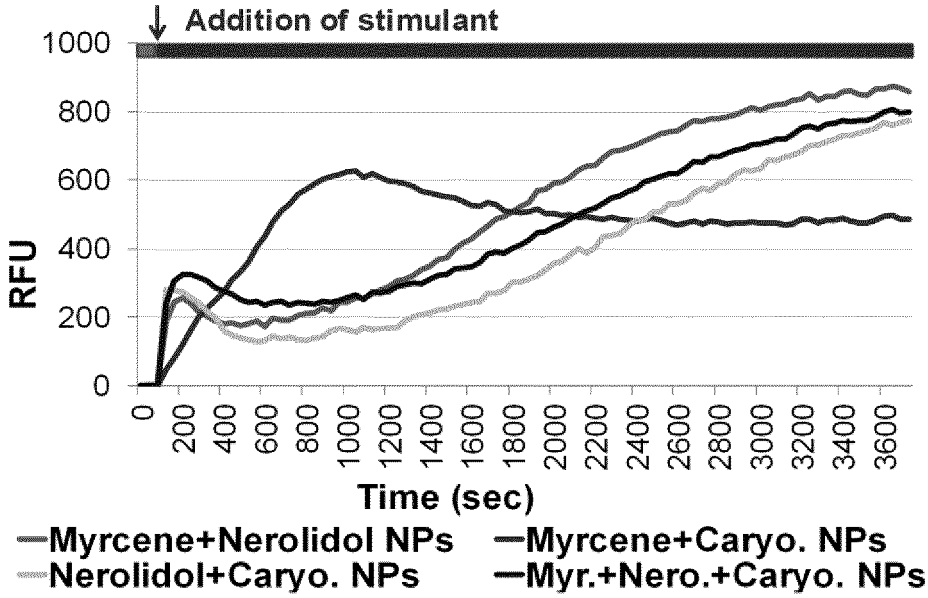


FIG. 20D

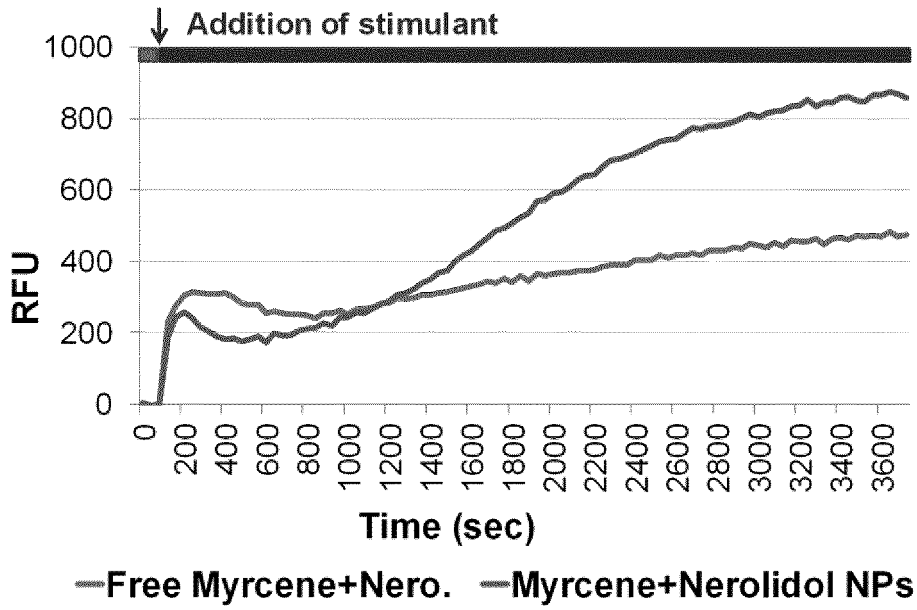


FIG. 21A

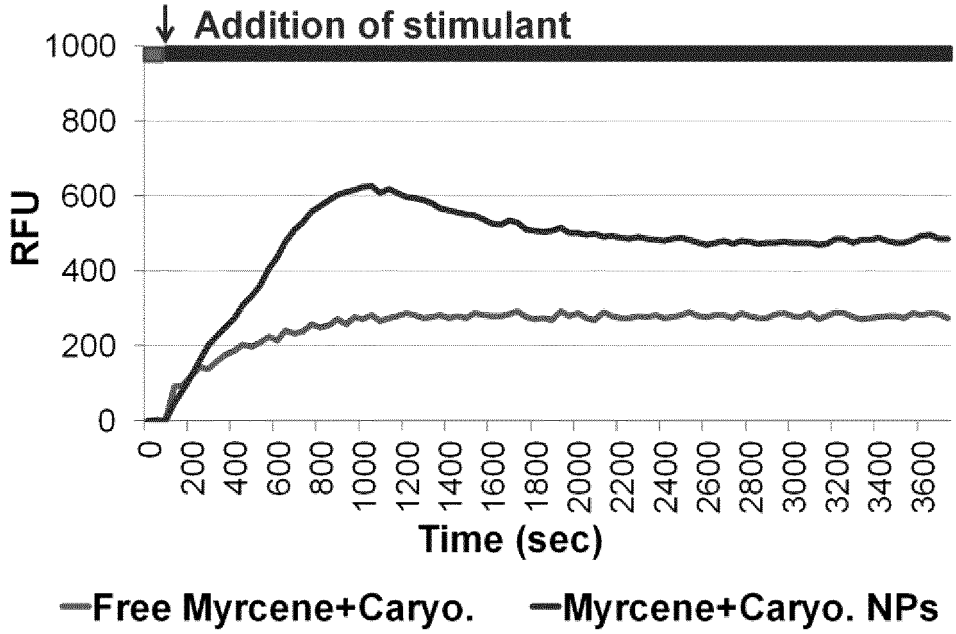


FIG. 21B

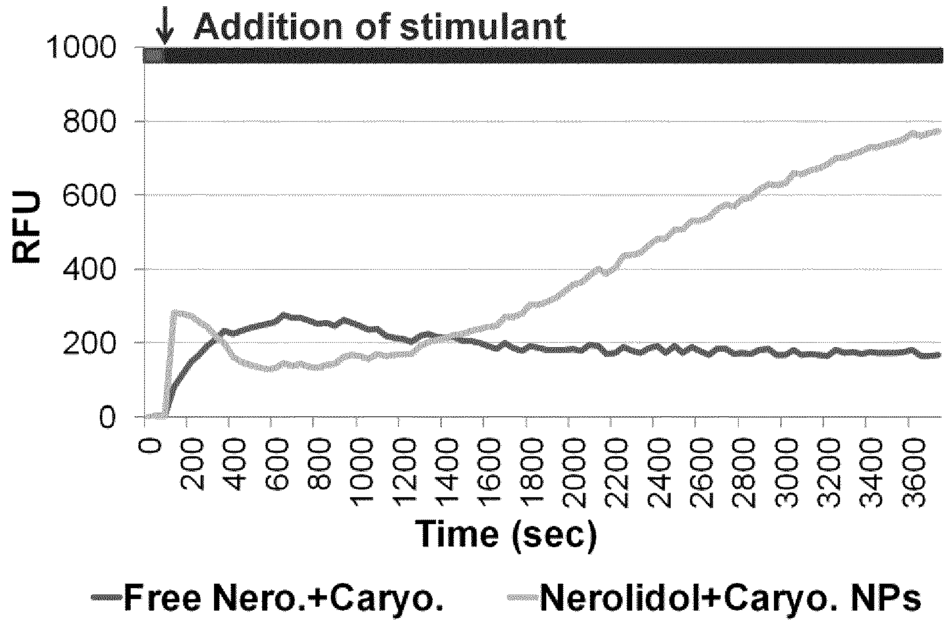


FIG. 21C

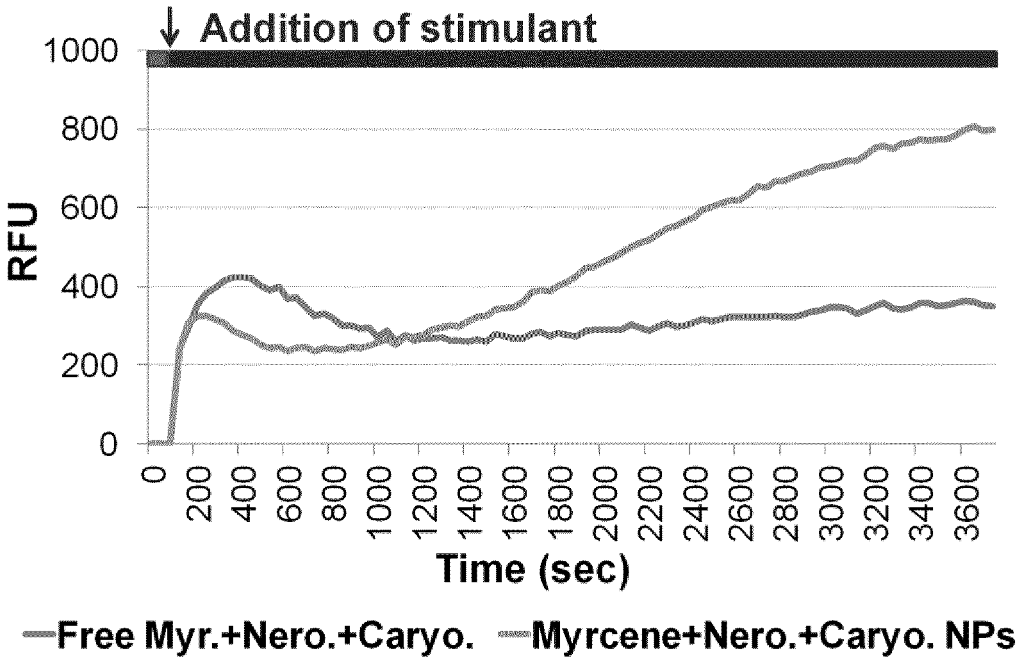
↓ Addition of stimulant
1000
800
600
400
200
0
RFU
0
200
400
600
800
1000
1200
1400
1600
1800
2000
2200
2400
2600
2800
3000
3200
3400
3600
Time (sec)
Free Myr.+Nero.+Caryo.
Myrcene+Nero.+Caryo. NPs

FIG. 21D

## INTERNATIONAL SEARCH REPORT

International application No
PCT/ES2019/070765

A. CLASSIFICATION OF SUBJECT MATTER
INV. A61K31/01 A61K31/015 A61K31/045 A61K31/05 A61K31/192 A61K31/352 A61P9/00 A61P11/14 A61P13/10 A61P25/02
ADD.

According to International Patent Classification (IPC) or to both national classification and IPC

B. FIELDS SEARCHED

Minimum documentation searched (classification system followed by classification symbols)
A61K A61P

Documentation searched other than minimum documentation to the extent that such documents are included in the fields searched

Electronic data base consulted during the international search (name of data base and, where practicable, search terms used)

EPO-Internal, WPI Data

C. DOCUMENTS CONSIDERED TO BE RELEVANT

| Category* | Citation of document, with indication, where appropriate, of the relevant passages | Relevant to claim No. |
|---|---|---|
| X<br><br>Y | WANG YUJIE ET AL: "Preparation and evaluation of paclitaxel-loaded nanoparticle incorporated with galactose-carrying polymer for hepatocyte targeted delivery.", DRUG DEVELOPMENT AND INDUSTRIAL PHARMACY SEP 2012, vol. 38, no. 9, September 2012 (2012-09), pages 1039-1046, XP002797666, ISSN: 1520-5762<br>the whole document<br>----- | 1-108<br><br>109-134 |

-/--

[X] Further documents are listed in the continuation of Box C.

[X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 13 February 2020 | 17/03/2020 |

| Name and mailing address of the ISA/ | Authorized officer |
|---|---|
| European Patent Office, P.B. 5818 Patentlaan 2<br>NL - 2280 HV Rijswijk<br>Tel. (+31-70) 340-2040,<br>Fax: (+31-70) 340-3016 | Giró, Annalisa |

1

Form PCT/ISA/210 (second sheet) (April 2005)

page 1 of 2

## INTERNATIONAL SEARCH REPORT

International application No
PCT/ES2019/070765

C(Continuation). DOCUMENTS CONSIDERED TO BE RELEVANT

| Category* | Citation of document, with indication, where appropriate, of the relevant passages | Relevant to claim No. |
|---|---|---|
| X | JULIANA PELISOLI HOLZ ET AL: "Menthol-loaded PLGA Micro and Nanospheres: Synthesis, Characterization and Degradation in Artificial Saliva", MATERIALS RESEARCH, vol. 21, no. 2, 15 February 2018 (2018-02-15), XP055667997, BR ISSN: 1516-1439, DOI: 10.1590/1980-5373-mr-2017-0488 | 1-108 |
| Y | the whole document | 109-134 |
| X | I Mu?oz-Rubio ET AL: "Malaga-Spain Design and production of cannabinoids-loaded PLGA nanoparticles for treatment of neuropathic pain", , 1 January 2010 (2010-01-01), XP055373939, Retrieved from the Internet: URL:http://www.nanospainconf.org/2010/Posters/NanoSpain2010_MunozRubio.pdf [retrieved on 2017-05-17] | 135, 163-257 |
| Y | the whole document | 258-271 |
| X | WO 2013/006729 A2 (WET INC [US]; BEVIER JONATHAN [US]) 10 January 2013 (2013-01-10) | 135, 163-257 |
| Y | the whole document | 258-271 |
| X | US 2018/042860 A1 (MARTÍN BANDERAS LUCÍA [ES] ET AL) 15 February 2018 (2018-02-15) | 135-181, 234-257 |
| Y | the whole document | 258-271 |
| Y | GOMES PAULA-FREIRE LYVIA IZAURA ET AL: "Ocimum gratissimum Essential Oil and Its Isolated Compounds (Eugenol and Myrcene) Reduce Neuropathic Pain in Mice", PLANTA MEDICA,, vol. 82, no. 3, 1 February 2016 (2016-02-01), pages 211-216, XP002793802, the whole document | 109-134 |
| Y | US 2018/049994 A1 (AUNG DIN RONALD [US]) 22 February 2018 (2018-02-22) the whole document | 109-134 |

1

Form PCT/ISA/210 (continuation of second sheet) (April 2005)

page 2 of 2

## INTERNATIONAL SEARCH REPORT

Information on patent family members

International application No
PCT/ES2019/070765

| Patent document cited in search report | Publication date | Patent family member(s) | Publication date |
|---|---|---|---|
| WO 2013006729 A2 | 10-01-2013 | CA 2840907 A1 | 10-01-2013 |
| | | CN 103796637 A | 14-05-2014 |
| | | DK 2729123 T3 | 11-01-2016 |
| | | EP 2729123 A2 | 14-05-2014 |
| | | EP 2995302 A1 | 16-03-2016 |
| | | JP 2014520817 A | 25-08-2014 |
| | | KR 20140065387 A | 29-05-2014 |
| | | MX 340962 B | 27-07-2016 |
| | | US 2013011484 A1 | 10-01-2013 |
| | | US 2014302121 A1 | 09-10-2014 |
| | | WO 2013006729 A2 | 10-01-2013 |
| US 2018042860 A1 | 15-02-2018 | CA 2976040 A1 | 18-08-2016 |
| | | EP 3257503 A1 | 20-12-2017 |
| | | ES 2582287 A2 | 12-09-2016 |
| | | US 2018042860 A1 | 15-02-2018 |
| | | WO 2016128591 A1 | 18-08-2016 |
| US 2018049994 A1 | 22-02-2018 | AU 2017313063 A1 | 07-03-2019 |
| | | BR 112019003007 A2 | 14-05-2019 |
| | | CA 3034250 A1 | 22-02-2018 |
| | | EP 3500235 A1 | 26-06-2019 |
| | | JP 2019528277 A | 10-10-2019 |
| | | US 2018049994 A1 | 22-02-2018 |
| | | US 2019274969 A1 | 12-09-2019 |
| | | WO 2018035212 A1 | 22-02-2018 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62757660
- US 62897235 B
- US 9084786 B
- US 2018033956 W

### Non-patent literature cited in the description

- **LLOYD.** The Art, Science and Technology of Pharmaceutical Compounding. 2012
- **IANNITELLI et al.** *Int. J. Mol. Sci.,* 2011, vol. 12, 5039-5051